# EUROPEAN PATENT APPLICATION

(11) **EP 4 793 367 A2**
(43) Date of publication of application: **19.08.2026**
(21) Application number: 26169988.8
(22) Date of filing: 24.02.2023
(51) Int. Cl.: C12Q 1/6869

(54) **CALIBRATION SEQUENCES FOR NUCELOTIDE SEQUENCING**

(30) Priority: 25.02.2022 US 202263268547 P
(62) Document number(s) of the earlier application(s) in accordance with Art. 76 EPC: 23715341.6 / 4 483 372
(71) Applicant: Illumina, Inc., San Diego, CA 92122 (US)
(72) Inventor: VIECELI, John S, San Diego, CA 92122 (US); LU, Bo, San Diego, CA 92122 (US); FISHER, Jeffrey S, San Diego, CA 92122 (US)
(74) Representative: Robinson, David Edward Ashdown

(57) **Abstract**

This disclosure describes methods, non-transitory computer readable media, and systems that can introduce short calibration sequences into a sequencing device and run calibration cycles to adjust or otherwise determine a sequencing parameter corresponding to the sequencing device. For instance, the disclosed systems can detect a flow cell (or other sample-nucleotide slide) with calibration sequences incorporated into samples' library fragments or into a surface of the sample-nucleotide slide. By running one or more calibration cycles to incorporate nucleobases on oligonucleotides corresponding to calibration sequences and capture corresponding images for calibration sequences-separate from genomic sequencing cycles for sample genomic sequences-the disclosed systems can determine a sequencing parameter corresponding to the sequencing device.

## Description

### CROSS-REFERENCE TO RELATED APPLICATIONS

The present application claims the benefit of, and priority to, U.S. Provisional Application No. 63/268,547, entitled "CALIBRATION SEQUENCES FOR NUCELOTIDE SEQUENCING," filed on February 25, 2022. The aforementioned application is hereby incorporated by reference in its entirety.

### BACKGROUND

In recent years, biotechnology firms and research institutions have improved hardware and software for both sequencing machines that determine nucleotide-fragment reads for a genomic sample (or other nucleic-acid polymer) and sequencing-data-analysis software that analyzes the nucleobase calls for such nucleotide-fragment reads. To generate and analyze nucleobase calls for genomic samples, some existing sequencing machines, on-device software, and sequencing-data-analysis software (together "existing sequencing systems") configure sequencing parameters that demarcate boundaries, set baseline signals or noise, or otherwise guide or figure into determining nucleobase calls. Existing sequencing systems can determine some such sequencing parameters offline for a specific sequencing machine or pipeline, such as equalizer coefficients or other predetermined sequencing parameters, or determine other sequencing parameters during a sequencing run on a sequencing machine, such as offset correction parameters. To calibrate certain sequencing parameters, existing sequencing systems often run genomic sequencing cycles of oligonucleotides extracted from a well-defined genome sample, such as Phi-X, and determine or adjust parameters based on the known sequence of the well-defined genome sample. But conventional calibration by existing sequencing systems can introduce sequencing failures and inaccurate nucleobase calls-as well as consume computing and other resources for calibration on genomic sequencing cycles.

As just suggested, existing sequencing parameters that predetermine sequencing parameters for a sequencing machine or sequencing pipeline can introduce errors. For instance, a sequencing parameter predetermined offline for one sequencing machine may not work well for the environment of another sequencing machine, flow cell, or set of reagents, notwithstanding similar hardware and similar consumables used for the different sequencing machine. Further, even if a predetermined sequencing parameter is well configured for a sequencing machine initially (e.g., at time of shipment), a sequencing machine's hardware or software may change over time and no longer reflect the state of the hardware or software used at the time of predetermining the sequencing parameter, such as an equalizer coefficient for extracting intensity values.

In addition to errors fomented by predetermined sequencing parameters, existing sequencing systems often exhibit random failures during some (or all) of a conventional sequencing run on a sequencing machine when using genomic sequencing cycles in a lab for calibration. For instance, existing sequencing systems frequently rely on metrics captured during an initial genomic sequencing cycle (or set of initial genomic sequencing cycles) to configure sequencing parameters. But random failures in the early genomic sequencing cycles can affect and introduce errors into the remaining genomic sequencing cycles. In an initial genomic sequencing cycle, for instance, a sequencing machine may incorporate nucleotides and capture images from clusters of oligonucleotides affected by a bubble on a nucleotide-sample slide, capture images of such clusters using an out-of-focus camera or a camera that has been auto-centered on a cluster incorrectly, or capture images at an initial temperature unrepresentative of temperatures for later cycles. Overtime, as the environment of the sequencing machine and nucleotide-sample slide changes, sequencing parameters configured from pre-genomic sequencing cycles can misrepresent conditions in later genomic sequencing cycles and skew sequencing parameters for nucleobase calls. In some cases, random failures in early pre-genomic sequencing cycles can result in a complete failure of an entire sequencing run, such as when an existing sequencing system mis-estimates distortion coefficients or phase offsets for super-resolution instruments in pre-genomic sequencing cycles.

Beyond random conditions of a sequencing machine or sample-nucleotide slide, in some cases, the distribution of nucleobase types in nucleotide-fragments reads for an unknown genomic sample or a well-defined genomic sample can skew or bias sequencing parameters. In some genomic sequencing cycles, for instance, the cluster of oligonucleotides may be incorporating nucleobases that disproportionately represent one nucleobase type over another. When learning sequencing parameters from early genomic sequencing cycles, for instance, existing sequencing systems can converge on an inaccurate sequencing parameter from a genomic sample with regions of low nucleobase-type diversity. In one recent study, researchers found genomic sequencing cycles in which the sequencing machine incorporated 100% of a single nucleobase type during an initial cycle for Phi-X. When sequencing machines configure sequencing parameters based on genomic sequencing cycles of disproportionately distributed nucleobase types, the sequencing machine's accuracy for nucleobase calls can suffer. Indeed, without a Phi-X spike-in, the sequencing parameters configured from a cycle of 100% of a single nucleobase resulted in a two-folder higher error rate for nucleobase calls.

Independent of accuracy problems, in some cases, existing sequencing systems use conventional calibration approaches that consume computing and sequencing machine resources that could otherwise be used to determine a genomic sequence of an unknown sample. For instance, existing sequencing systems commonly dedicate clusters of oligonucleotides to a well-defined genome samples to facilitate calibrating a sequencing parameter. By dedicating a well or other portion of a sample-nucleotide slide to a control sample, however, existing sequencing systems shrink the portion of the sample-nucleotide slide and genomic sequencing cycles that can be used to determine unknown genomic samples.

These, along with additional problems and issues exist in existing sequencing systems.

### SUMMARY

This disclosure describes one or more embodiments of systems, methods, and non-transitory computer readable storage media that solve one or more of the problems described above or provide other advantages over the art. In particular, the disclosed system can introduce short calibration sequences into a sequencing device and run calibration cycles to adjust or otherwise determine a sequencing parameter corresponding to the sequencing device. For instance, the disclosed systems can detect a flow cell (or other sample-nucleotide slide) with calibration sequences incorporated into samples' library fragments or into a surface of the sample-nucleotide slide. By running one or more calibration cycles to incorporate nucleobases on oligonucleotides corresponding to calibration sequences and capturing corresponding images for calibration sequences-separate from genomic sequencing cycles for sample genomic sequences-the disclosed systems can determine a sequencing parameter corresponding to the sequencing device. For instance, the disclosed system can directly estimate some sequencing parameters that are detected during or after a calibration cycle, determine a sequencing parameter from initial sequencing parameters detected during multiple calibration cycles, and/or adjust initial sequencing parameters based on base-call differences between (i) nucleobase calls for complimentary strands to calibration sequences and (ii) known complimentary nucleobases for calibration sequences.

Additional features and advantages of one or more embodiments of the present disclosure will be set forth in the description which follows, and in part will be obvious from the description, or may be learned by the practice of such example embodiments.

### BRIEF DESCRIPTION OF THE DRAWINGS

The detailed description refers to the drawings briefly described below.
FIG. 1 illustrates an environment in which a calibration-sequencing system can operate in accordance with one or more embodiments of the present disclosure.
FIG. 2A illustrates a schematic diagram of the calibration-sequencing system receiving nucleotide-sample slide comprising calibration sequences and determining one or more sequencing parameters corresponding to a sequencing device based on the calibration sequences in accordance with one or more embodiments of the present disclosure.
FIG. 2B illustrates a schematic diagram of the calibration-sequencing system performing different types of calibration cycles to determine one or more sequencing parameters corresponding to a sequencing device in accordance with one or more embodiments of the present disclosure.
FIG. 2C illustrates a schematic diagram of the calibration-sequencing system using sequencing parameters from calibration cycles during a genomic sequencing cycle or an indexing cycle in accordance with one or more embodiments of the present disclosure.
FIGS. 3A-3B illustrate a calibration sequence integrated within a sample library fragment in accordance with one or more embodiments of the present disclosure.
FIG. 4 illustrates example calibration cycles of the calibration-sequencing system in accordance with one or more embodiments of the present disclosure.
FIGS. 5A-5E illustrate calibration sequences integrated within sample library fragments and the sample library fragments distributed through clusters of oligonucleotides within a nucleotide-sample slide in accordance with one or more embodiments of the present disclosure.
FIG. 6 illustrates intensity-value distributions for different channels corresponding to different nucleobase types based on either randomly called nucleobases or known nucleobases from calibration sequences in accordance with one or more embodiments of the present disclosure.
FIGS. 7A-7B illustrate scatter plots for intensity values corresponding to different nucleobase types for different channels based on calibration sequences in accordance with one or more embodiments of the present disclosure.
FIGS. 8A-8B illustrate scatter plots for intensity values corresponding to cycles with un-calibrated sequencing parameters and calibrated sequencing parameters in accordance with one or more embodiments of the present disclosure.
FIG. 9 illustrate series of acts for receiving nucleotide-sample slide comprising calibration sequences and determining one or more sequencing parameters corresponding to a sequencing device based on the calibration sequences in accordance with one or more embodiments of the present disclosure.
FIG. 10 illustrate series of acts for receiving nucleotide-sample slide comprising calibration nucleobases and determining one or more sequencing parameters corresponding to a sequencing device based on the calibration nucleobases in accordance with one or more embodiments of the present disclosure.
FIG. 11 illustrates a block diagram of an example computing device in accordance with one or more embodiments of the present disclosure.

### DETAILED DESCRIPTION

This disclosure describes one or more embodiments of a calibration-sequencing system that can detect short calibration sequences associated with library fragments deposited on a sample-nucleotide slide and run calibration cycles to determine a sequencing parameter corresponding to a sequencing device based on the short calibration sequences. For instance, the calibration-sequencing system can detect a flow cell (or other sample-nucleotide slide) that includes calibration sequences incorporated into (i) library fragments for one or more genomic samples or (ii) a substrate of the sample-nucleotide slide. The calibration-sequencing system can subsequently run one or more calibration cycles to incorporate nucleobases into oligonucleotides corresponding to the calibration sequences and determine one or more sequencing parameters (e.g., intensity values, intensity-value boundaries, equalizer coefficients) based on the calibration sequences' nucleobases. Such calibration cycles can run before genomic sequencing cycles that later sequence sample genomic sequences or complimentary deoxyribonucleic acid (cDNA) from the library fragments.

As suggested above, the calibration-sequencing system can determine a sequencing parameter in a variety of ways using one or more calibration cycles. For instance, in some cases, the calibration-sequencing system runs one or more calibration cycles to directly detect a sequencing parameter that corresponds to certain known nucleobase types in the calibration sequences, such as by detecting intensity values for nucleobase signals, nucleobase centroids for intensity values, intensity-value boundaries for nucleobases, or other sequencing parameters. Additionally or alternatively, the calibration-sequencing system can determine a sequencing parameter from initial sequencing parameters detecting during or after multiple calibration cycles. Further, in certain implementations, the calibration-sequencing system runs calibration cycles to determine nucleobase calls for the calibration sequences and adjusts one or more sequencing parameters based on base-call differences (or other detected sequencing-metric differences) between the nucleobase calls for complimentary strands/oligonucleotides to the calibration sequences and known complimentary nucleobases for the calibration sequences.

By running calibration cycles with calibration sequences, the calibration-sequencing system can determine various types of sequencing parameters corresponding to a sequencing device or sequencing pipeline. For instance, in some cases, the calibration-sequencing system uses calibration sequences to determine one or more sequencing parameters that previously have been determined offline and provided as pre-configured parameters, such as equalizer coefficients. As a further example, in certain embodiments, the calibration-sequencing system uses calibration sequences to determine one or more sequencing parameters learned during a sequencing run on a sequencing device, such as non-linear optical distortion parameters, structured illumination microscopy (SIM) parameters, per-cluster normalization parameters, or offset correction parameters for a particular channel.

When running calibration cycles, the calibration-sequencing system can arrange or order the calibration cycles in a variety of ways. For instance, in certain implementations, the calibration-sequencing system performs calibration cycles before and/or without performing genomic sequencing cycles or indexing cycles. During a given calibration cycle, the calibration-sequencing system can incorporate nucleobases of one or more nucleobase types into a calibration-sequence position of growing oligonucleotides that mirror one or more calibration sequences-thereby reflecting that a set of calibration sequences may have a single nucleobase type or different nucleobase types at a given calibration-sequence position. In some such calibration cycles, the calibration-sequencing system incorporates a proportional distribution of two, three, or four nucleobase types into a given calibration-sequence position of growing oligonucleotides that mirror one or more calibration sequences-thereby reflecting that a set of calibration sequences may have an approximately equal distribution of two, three, or four nucleobase types at a given calibration-sequence position.

Indeed, a given set of calibration sequences may include a single sequence of nucleobases (e.g., ACGTAC) in a same order, a sequence of nucleobases in different orders (e.g., ACGTAC for one order and CGTACA for a different order, etc.), different sequences of nucleobases with a proportional distribution of nucleobase types at given calibration-sequence positions (e.g., a 2-plex of G/C at 50%/50% in a first position, a two-plex of G/T at 50%/50% in a second position, etc.), or various other sequence orders or arrangements set forth below.

Regardless of the arrangement or order of a calibration cycle, the calibration-sequencing system can receive or determine the contents of calibration sequences (i) placed in different locations within (or with respect to) sample library fragments prepared from genomic samples or (ii) attached to a surface of a sample-nucleotide slide. For instance, a calibration sequence may be part or all of a non-genomic sequence and/or non-transcriptomic sequence within a sample library fragment. In some such cases, the calibration sequence is located between a binding adapter sequence and an indexing sequence, between the indexing sequence and a read priming sequence, or between the read priming sequence and a sample genomic sequence. By contrast, a calibration sequence may be part or all of a non-random unique molecular identifier (UMI) sequence, a unique dual index (UDI) sequence, an indexing sequence, or a binding adapter sequence.

In addition to various different locations for a calibration sequence, the calibration-sequencing system can receive or place calibration sequences (or sample library fragments that include calibration sequences) in different locations of a nucleotide-sample slide, such as a flow cell. For instance, the calibration-sequencing system may receive or detect a nucleotide-sample slide with calibration sequences (i) in each well for seeding each cluster of oligonucleotides within each well or (ii) in a subset of wells for seeding each cluster within the subset of wells or seeding a subset of clusters of oligonucleotides within the subset of wells. Alternatively, in some embodiments, the calibration-sequencing system receives or detects a nucleotide-sample slide with (i) calibration sequences in a subset of wells or a subset of clusters of oligonucleotides within the subset of wells and (ii) no calibration sequences in another subset of wells or another subset of clusters of oligonucleotides. As described below, such different well placements for calibration sequences may be combined with different locations of a calibration sequence associated with sample library fragments.

As indicated above, the calibration-sequencing system provides several technical advantages relative to existing sequencing systems, such as by improving the accuracy, speed, and efficiency of sequencing parameters and nucleotide sequencing relative to existing sequencing systems. For instance, in some embodiments, the calibration-sequencing system improves the accuracy of one or more sequencing parameters for a specific sequencing device, image analysis, and corresponding nucleobase calls. As noted above, some existing sequencing systems skew sequencing parameters by estimating such parameters from unknown or well-known genomic samples (e.g., Phi-X) that disproportionately represent certain nucleobase types in a given genomic sequencing cycle or across genomic sequencing cycles. By contrast, the calibration-sequencing system receives or detects nucleotide-sample slides with calibration sequences of nucleobases that can even out a distribution of nucleobase types for a given calibration cycle or across calibration cycles-thereby exhibiting a more proportionate representation of nucleobase types. By performing calibration cycles to incorporate one or more nucleobases of a set of calibration sequences, the calibration-sequencing system can determine more accurate sequencing parameters corresponding to a sequencing device. Unlike existing systems that use sequencing parameters for pipelines of similar hardware and similar consumables, the calibration-sequencing system can run calibration cycles on a specific sequencing device to generate intensity values, intensity-value boundaries, equalizer coefficients, or other sequencing parameters specific to the sequencing device. The calibration-sequencing system can accordingly use calibration sequences and calibration cycles to determine sequencing parameters with an accuracy customized for a specific sequencing device. As a consequence of such improved sequencing parameters, the calibration-sequencing system likewise improves the accuracy with which a sequencing device captures images of clusters of oligonucleotides during sequencing cycles, analyzes those images, and determines nucleobase calls based on such analysis.

In addition to improved accuracy, in certain implementations, the calibration-sequencing system extemporaneously adjusts one or more sequencing parameters corresponding to a sequencing device to correct for abnormalities or errors in real time-introducing a type of extemporaneous flexibility not present in existing sequencing systems. As noted above, some existing sequencing systems skew or outdate sequencing parameters by using early genomic sequencing cycles (sometimes with errors) to determine sequencing parameters and subsequently fail to adjust such parameters as software or hardware may change over time. In contrast to existing systems, in some embodiments, the calibration-sequencing system receives nucleotide-sample slides that include calibration sequences and run calibration cycles using the calibration sequences to extemporaneously determine a sequencing parameter corresponding to a current state of a sequencing device. The calibration-sequencing system can accordingly determine (or adjust) one or more sequencing parameters with each nucleotide-sample slide comprising calibration sequences and/or across calibration cycles. As a camera for a sequencing device changes focus or centering, as temperatures change in a sequencing device, or as other hardware or software components change, the calibration-sequencing system can determine one or more sequencing parameters that reflect a current or recent state of a sequencing device or corresponding software.

Beyond improved parameter accuracy and timely adjustment, in some embodiments, the calibration-sequencing system improves the computing efficiency of nucleotide sequencing by preserving space and computing resources of a sequencing device and corresponding sequencing cycles. As noted above, some existing sequencing systems consume genomic sequencing cycles on unknown or well-known genomic samples (e.g., Phi-X) with dedicated clusters for calibration of sequencing parameters. Indeed, some existing sequencing systems dedicate entire sequencing runs or specific clusters of a nucleotide-sample slide to genomic sequences for calibration. In contrast to existing systems, in certain implementations, the calibration-sequencing system uses relatively short calibration sequences that can be embedded within (or attached to) sample library fragments and calibration cycles that can be performed during a same sequencing run as genomic sequencing cycles for determining sample genomic sequences within the sample library fragments. By running calibration cycles with relatively short calibration sequences, the calibration-sequencing system can determine (or adjust) sequencing parameters corresponding to a sequencing device during a sequencing run that also performs genomic sequencing cycles-thereby preserving computing resources and space on a nucleotide-sample slide for genomic sequencing of an unknown sample that would have otherwise been dedicated to calibration.

As illustrated by the foregoing discussion, the present disclosure utilizes a variety of terms to describe features and advantages of the calibration-sequencing system. As used herein, for example, the term "calibration sequence" refers to a sequence of two or more nucleobases representing nucleobase types that facilitate calibrating one or more sequencing parameters of a sequencing device or corresponding software. For instance, a calibration sequence includes an artificial, non-genomic and/or non-transcriptomic sequence of nucleobases having a targeted distribution of nucleobase types used to determine or adjust sequencing parameters. Such a non-genomic and/or non-transcriptomic sequence is accordingly not part of a gDNA fragment, a cDNA fragment, or an RNA fragment (e.g., mRNA fragment) from a sample. Such a targeted distribution of nucleobase types may include a proportional distribution of different nucleobase types across positions of a set of calibration sequences, a proportional distribution of a combination of different nucleobase types at a given calibration-sequence position, a representative distribution of nucleobase types (e.g., average or otherwise representative distribution of a taxonomic phylum, class, order, family, genus, or species) across positions of a set of calibration sequences, or another predetermined distribution of nucleobase types. A given set of calibration sequences, for instance, may include a single sequence of nucleobases having a targeted distribution of nucleobase types in a same order (e.g., ACTGCGC), a sequence of nucleobases having a targeted distribution of nucleobase types in different orders (e.g., ACGT for one order and CGTA for a different order, etc.), or different sequences of nucleobases with a targeted distribution of nucleobase types at given calibration-sequence positions (e.g., a four-plex of A/C/G/T at 25%/25%/25%/25% in a first position, another four-plex of A/C/G/T at 25%/25%/25%/25% in a second position, etc.). In some cases, a calibration sequence is relatively short, such as 4-8 nucleobases in length. A calibration sequence may be ligated to (or part of) one or more nucleotide sequences of a sample library fragment or ligated to a surface of the nucleotide-sample slide.

Relatedly, the term "calibration nucleobase" refers to a nucleobase that facilitates calibrating one or more sequencing parameters of a sequencing device or corresponding software. For instance, a group of calibration nucleobases includes an artificial, non-genomic and/or non-transcriptomic set of nucleobases having a targeted distribution of nucleobase types used to determine or adjust sequencing parameters. Such a non-genomic and/or non-transcriptomic set of nucleobases is accordingly not part of or extracted from a gDNA fragment or cDNA fragment. Such a targeted distribution of nucleobase types may include a proportional distribution of different nucleobase types across different sets of sample library fragments, a representative distribution of nucleobase types (e.g., average or otherwise representative distribution of a taxonomic phylum, class, order, family, genus, or species) across different sets of sample library fragments, or another predetermined distribution of nucleobase types. Similar to a calibration sequence, a calibration nucleobase may be ligated to (or part of) one or more nucleotide sequences of a sample library fragment or ligated to a surface of the nucleotide-sample slide.

As further used herein, the term "sample library fragment" refers to a sample genomic sequence (or cDNA sequence) that is ligated to include one or more adapter sequences or primer sequences that facilitate detection or isolation of the sample genomic sequence or cDNA sequence. For instance, a sample library fragment can include, but is not limited to, a sample genomic sequence (or cDNA sequence) that is extracted from a sample and ligated to bond directly or indirectly with one or more of a binding adapter sequence, an indexing sequence, or a read priming sequence.

Relatedly, the term "sample genomic sequence" refers to a nucleotide sequence extracted or copied from a sample's chromosome. For example, a sample genomic sequence includes a nucleotide sequence that has been separated or copied from chromosomal DNA of a sample. Accordingly, a sample genomic sequence includes genomic DNA (gDNA) for a particular unknown sample. As described herein, in some embodiments, the calibration-sequencing system can use a sample complimentary sequence comprising cDNA rather than a sample genomic sequence comprising gDNA in a sample library fragment or wherever suitable cDNA may replace gDNA as understood by a skilled artisan.

By contrast, an "indexing sequence" refers to a unique and artificial nucleotide sequence that identifies nucleotide-fragment reads for a sample and that is ligated to a sample's nucleotide sequence (e.g., a gDNA fragment or cDNA fragment) or to another sequence within a sample library fragment. As indicated above, an indexing sequence can be part of a sample library fragment. Similarly, an indexing sequence can be used to sort nucleotide-fragment reads by sample or into different files, among other things. In some cases, a sample library fragment includes an indexing primer sequence that differs from a read priming sequence and that indicates a starting point or starting nucleobase for determining nucleobases of an indexing sequence.

As further used herein, the term "binding adapter sequence" refers to a nucleotide sequence that binds to oligonucleotides on a surface (e.g., a well) of a nucleotide-sample slide and that is ligated to a sample's nucleotide sequence (e.g., a gDNA fragment or cDNA fragment) or to another sequence within a sample library fragment. In some cases, a binding adapter sequence includes a nucleotide sequence that is ligated to an end (e.g., 5' end or 3' end) of a sample library fragment and that binds to an oligonucleotide from an oligonucleotide lawn of a sample-nucleotide slide.

As also used herein, the term "read priming sequence" refers to a nucleotide sequence that indicates a starting point or starting nucleobase for determining nucleobases of a sample's nucleotide sequence (e.g., a gDNA fragment or cDNA fragment) and that is ligated to the sample's nucleotide sequence. During sequencing, in some cases, a sequencing primer binds or anneals the read priming sequence from a sample library fragment, and a polymerase enzyme or other enzyme incorporates nucleobases that add to the sequencing primer and are complimentary to a sample genomic sequence (and/or other nucleotide sequences from a sample library fragment) to sequence a nucleotide-fragment read. As indicated above, a read priming sequence can be part of a sample library fragment.

Relatedly, as used herein, the term "sequencing primer" refers to a nucleotide fragment that binds to a read priming sequence (or other primer site) as a beginning portion of a nucleotide-fragment read. In particular, a sequencing primer includes a nucleotide fragment that binds to a read priming sequence (or other primer site) during a sequencing cycle (e.g., genomic sequencing cycle, calibration cycle) and that forms a nascent sequence for a nucleotide-fragment read. As suggested above, during a sequencing cycle, an enzyme adds or incorporates nucleobase by nucleobase to the sequencing primer to grow or sequence the nucleotide-fragment read.

As further noted above, the calibration-sequencing system can perform or run one or more calibration cycles. The term "calibration cycle" refers to an iteration of adding or incorporating one or more nucleobases to one or more oligonucleotides representing or corresponding to one or more calibration sequences for a cluster of oligonucleotides or a set of clusters of oligonucleotides. In particular, a calibration cycle can include an iteration of capturing and analyzing one or more images of clusters of oligonucleotides indicating one or more nucleobases added or incorporated into an oligonucleotide (or to oligonucleotides in parallel) representing or corresponding to one or more calibration sequences. Because some clusters of oligonucleotides or corresponding wells may include a calibration sequence-while other clusters of oligonucleotides or corresponding wells may not include a calibration sequence-a calibration cycle, in some cases, is specific to a cluster of oligonucleotides or a set of clusters of oligonucleotides. A calibration cycle differs from an indexing cycle or a genomic sequencing cycle in that a calibration cycle includes sequencing of at least a nucleobase (or a majority of nucleobases) from one or more calibration sequences. Due to potential differences among clusters in distributing calibration sequences, in some embodiments, the calibration-sequencing system may concurrently (i) perform a calibration cycle for one cluster of oligonucleotides (or one subset of clusters of oligonucleotides) by incorporating one or more nucleobases corresponding to one or more calibration sequences and (ii) perform a genomic sequencing cycle or an indexing cycle for another cluster of oligonucleotides (or another subset of clusters of oligonucleotides) by incorporating one or more nucleobases corresponding to either one or more sample genomic sequences or one or more indexing sequences, respectively.

By contrast, the term "indexing cycle" refers to an iteration of adding or incorporating a nucleobase to an oligonucleotide representing or corresponding to an indexing sequence or an iteration of adding or incorporating nucleobases to oligonucleotides representing or corresponding to indexing sequences in parallel. In particular, an indexing cycle can include an iteration of capturing and analyzing one or more images of clusters of oligonucleotides indicating one or more nucleobases added or incorporated into an oligonucleotide or to oligonucleotides (in parallel) representing or corresponding to one or more indexing sequences. An indexing cycle differs from a calibration cycle or a genomic sequencing cycle in that an indexing cycle includes sequencing of at least a nucleobase (or a majority of nucleobases) from one or more indexing sequences that identify or encode one or more sample library fragments. Because calibration cycles may be specific to a cluster or clusters of oligonucleotides, an indexing cycle for one cluster of oligonucleotides may be performed at a same time as a calibration cycle for another cluster of oligonucleotides.

As further used herein, the term "genomic sequencing cycle" refers to an iteration of adding or incorporating a nucleobase to an oligonucleotide representing or corresponding to a sample genomic sequence (or cDNA sequence) or an iteration of adding or incorporating nucleobases to oligonucleotides representing or corresponding to sample genomic sequences (or cDNA sequences) in parallel. In particular, a genomic sequencing cycle can include an iteration of capturing and analyzing one or more images with data indicating individual nucleobases added or incorporated into an oligonucleotide or to oligonucleotides (in parallel) representing or corresponding to one or more sample genomic sequences. For example, in one or more embodiments, each genomic sequencing cycle involves capturing and analyzing images to determine either single reads of DNA (or RNA) strands representing part of a genomic sample (or transcribed sequence from a genomic sample). As suggested above, however, a genomic sequencing cycle, in some cases, is specific to a cluster of oligonucleotides or a set of clusters of oligonucleotides. Because of potential differences among clusters in distributing calibration sequences, in some embodiments, the calibration-sequencing system may concurrently (i) perform a calibration cycle for one cluster of oligonucleotides (or one subset of clusters of oligonucleotides) and (ii) perform a genomic sequencing cycle or an indexing cycle for another cluster of oligonucleotides (or another subset of clusters of oligonucleotides).

In some cases, each of a calibration cycle, an indexing cycle, and a genomic sequencing cycle involve a camera capturing an image of a nucleotide-sample slide or images of multiple sections (e.g., tiles) of the nucleotide-sample slide to generate image data of particular nucleobases added or incorporated into particular oligonucleotides, which are often grouped in clusters. Following the image capture stage, the calibration-sequencing system can remove certain fluorescent labels from incorporated nucleobases and perform another cycle until a calibration sequence, an indexing sequence, or a sample genomic sequence has been completely sequenced.

As further used herein, the term "sequencing run" refers to an iterative process on a sequencing device to determine a primary structure of nucleotide sequences from a sample (e.g., genomic sample). In particular, a sequencing run includes cycles of sequencing chemistry and imaging performed by a sequencing device that incorporate nucleobases into growing oligonucleotides to determine nucleotide-fragment reads from nucleotide sequences extracted from a sample (or other sequences within a library fragment) and seeded throughout a nucleotide-sample slide. In some cases, a sequencing run includes replicating nucleotide sequences from one or more genome samples seeded in clusters throughout a nucleotide-sample slide (e.g., a flow cell). Upon completing a sequencing run, a sequencing device can generate base-call data in a file.

As just suggested, the term "base-call data" refers to data representing nucleobase calls for nucleotide-fragment reads and/or corresponding sequencing metrics. For instance, base-call data includes textual data representing nucleobase calls for nucleotide-fragment reads as text (e.g., A, C, G, T) along with corresponding base-call-quality metrics, depth metrics, and/or other sequencing metrics. In some cases, base-call data is formatted in a text file, such as a binary base call (BCL) sequence file or as a fast-all quality (FASTQ) file.

As further used here, the term "sequencing parameter" refers to a standardized or scaled factor, metric, or value that quantifies or represents (i) a setting, boundary, or environment in which a nucleobase of a particular nucleobase type can be accurately detected, quantified, or analyzed using a sequencing device or (ii) a particular signal of (or noise or chemical related to) a nucleobase type can be accurately detected, quantified, or analyzed using the sequencing device. For instance, a sequencing parameter includes, but is not limited to, one or more of equalizer coefficients, convolutional kernel coefficients, nucleobase centroids for intensity values, nucleobase-specific-background intensity values, intensity normalization coefficients, gaussian covariance matrices, non-linear optical distortion parameters, structured illumination microscopy (SIM) parameters, per-cluster normalization parameters for a particular channel, channel-specific normalization parameters (e.g., offset correction parameters for a particular channel, per-cluster-intensity parameters for a particular channel, per-cluster-brightness parameters for the particular channel), cluster-specific-signal normalization parameters (e.g., a sequencing-device-cluster-amplification-efficiency parameter, a sequencing-device-cluster-hybridization-efficiency parameter), color-channel-to-color-channel crosstalk parameters, or polycolonality parameters.

As further used herein, the term "nucleotide-sample slide" refers to a plate or slide comprising oligonucleotides for sequencing nucleotide sequences from genomic samples or other sample nucleic-acid polymers. In particular, a nucleotide-sample slide can refer to a slide containing fluidic channels through which reagents and buffers can travel as part of sequencing. For example, in one or more embodiments, a nucleotide-sample slide includes a flow cell (e.g., a patterned flow cell or non-patterned flow cell) comprising small fluidic channels and short oligonucleotides complementary to binding adapter sequences. As indicated above, a nucleotide-sample slide can include wells (e.g., nanowells) comprising clusters of oligonucleotides.

As suggested above, a flow cell or other nucleotide-sample slide can (i) include a device having a lid extending over a reaction structure to form a flow channel therebetween that is in communication with a plurality of reaction sites of the reaction structure and (ii) include a detection device that is configured to detect designated reactions that occur at or proximate to the reaction sites. A flow cell or other nucleotide-sample slide may include a solid-state light detection or imaging device, such as a Charge-Coupled Device (CCD) or Complementary Metal-Oxide Semiconductor (CMOS) (light) detection device. As one specific example, a flow cell may be configured to fluidically and electrically couple to a cartridge (having an integrated pump), which may be configured to fluidically and/or electrically couple to a bioassay system. A cartridge and/or bioassay system may deliver a reaction solution to reaction sites of a flow cell according to a predetermined protocol (e.g., sequencing-by-synthesis), and perform a plurality of imaging events. For example, a cartridge and/or bioassay system may direct one or more reaction solutions through the flow channel of the flow cell, and thereby along the reaction sites. At least one of the reaction solutions may include four types of nucleotides having the same or different fluorescent labels. The nucleotides may bind to the reaction sites of the flow cell, such as to corresponding oligonucleotides at the reaction sites. The cartridge and/or bioassay system may then illuminate the reaction sites using an excitation light source (e.g., solid-state light sources, such as light-emitting diodes (LEDS)). The excitation light may provide emission signals (e.g., light of a wavelength or wavelengths that differ from the excitation light and, potentially, each other) that may be detected by the light sensors of the flow cell.

As further used herein, the term "nucleotide-fragment read" (or simply "read") refers to an inferred sequence of one or more nucleobases (or nucleobase pairs) from all or part of a sample nucleotide sequence (e.g., a sample genomic sequence, cDNA). In particular, a nucleotide-fragment read includes a determined or predicted sequence of nucleobase calls for a nucleotide sequence (or group of monoclonal nucleotide sequences) from a sample library fragment corresponding to a genome sample. For example, in some cases, a sequencing device determines a nucleotide-fragment read by generating nucleobase calls for nucleobases passed through a nanopore of a nucleotide-sample slide, determined via fluorescent tagging, or determined from a cluster in a flow cell.

As further used herein, the term "nucleobase call" (or simply "base call") refers to a determination or prediction of a particular nucleobase (or nucleobase pair) for an oligonucleotide (e.g., read) during a sequencing cycle or for a genomic coordinate of a sample genome. In particular, a nucleobase call can indicate (i) a determination or prediction of the type of nucleobase that has been incorporated within an oligonucleotide on a nucleotide-sample slide (e.g., read-based nucleobase calls) or (ii) a determination or prediction of the type of nucleobase that is present at a genomic coordinate or region within a genome, including a variant call or a non-variant call in a digital output file. In some cases, for a nucleotide-fragment read, a nucleobase call includes a determination or a prediction of a nucleobase based on intensity values resulting from fluorescent-tagged nucleotides added to an oligonucleotide of a nucleotide-sample slide (e.g., in a cluster of a flow cell). Alternatively, a nucleobase call includes a determination or a prediction of a nucleobase from chromatogram peaks or electrical current changes resulting from nucleotides passing through a nanopore of a nucleotide-sample slide. By contrast, a nucleobase call can also include a final prediction of a nucleobase at a genomic coordinate of a sample genome for a variant call file (VCF) or other base-call-output file-based on nucleotide-fragment reads corresponding to the genomic coordinate. Accordingly, a nucleobase call can include a base call corresponding to a genomic coordinate and a reference genome, such as an indication of a variant or a non-variant at a particular location corresponding to the reference genome. Indeed, a nucleobase call can refer to a variant call, including but not limited to, a single nucleotide variant (SNV), an insertion or a deletion (indel), or base call that is part of a structural variant. As suggested above, a single nucleobase call can be an adenine (A) call, a cytosine (C) call, a guanine (G) call, or a thymine (T) call.

Relatedly, the term "nucleobase type" refers to a particular type or kind of nitrogenous base. For instance, a genome or nucleotide sequence may include five different nucleobase types, including adenine (A), cytosine (C), guanine (G), or thymine (T), or uracil (U).

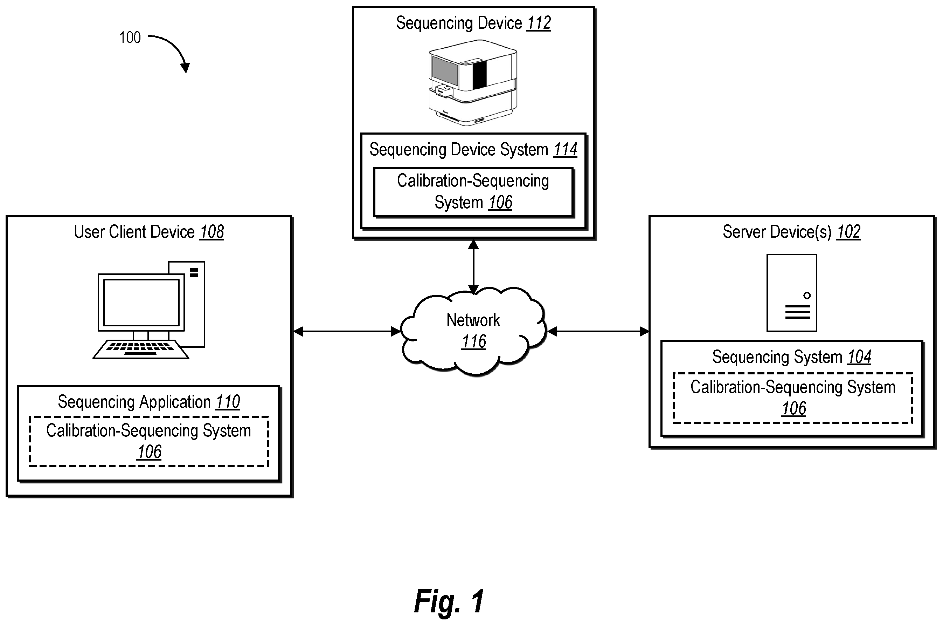

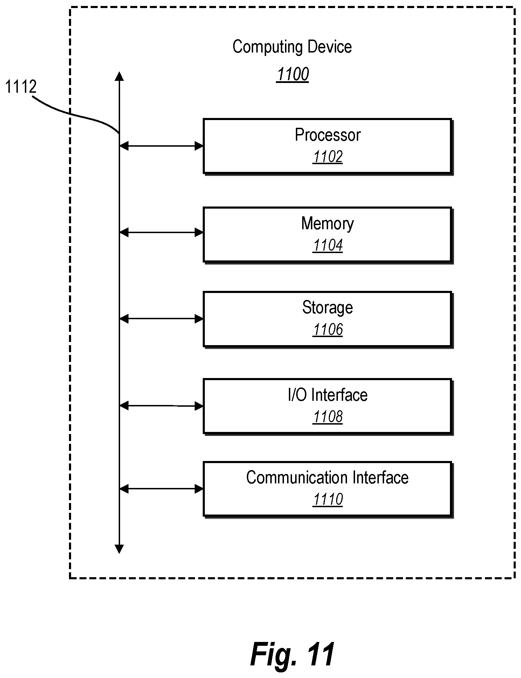

The following paragraphs describe the calibration-sequencing system with respect to illustrative figures that portray example embodiments and implementations. For example, FIG. 1 illustrates a schematic diagram of a system environment (or "environment") 100 in which a calibration-sequencing system 106 operates in accordance with one or more embodiments. As illustrated, the environment 100 includes server device(s) 102, a sequencing device 112, and a user client device 108 via a network 116. While FIG. 1 shows an embodiment of the calibration-sequencing system 106, this disclosure describes alternative embodiments and configurations below. As shown in FIG. 1, the sequencing device 112, the server device(s) 102, and the user client device 108 can communicate with each other via the network 116. The network 116 comprises any suitable network over which computing devices can communicate. Example networks are discussed in additional detail below with respect to FIG. 11.

As indicated by FIG. 1, the sequencing device 112 comprises a computing device, a sequencing device system 114, and the calibration-sequencing system 106 for sequencing a genomic sample or other nucleic-acid polymer and running calibration cycles. In some embodiments, by executing the sequencing device system 114 or the calibration-sequencing system 106, the sequencing device 112 analyzes nucleic-acid segments or oligonucleotides extracted from genomic samples to generate nucleotide-fragment reads or other data utilizing computer implemented methods and systems (described herein) either directly or indirectly on the sequencing device 112. More particularly, the sequencing device 112 receives nucleotide-sample slides (e.g., flow cells) comprising nucleotide sequences extracted from samples and then copies and determines the nucleobase sequence of such extracted nucleotide sequences.

As suggested above, by executing the sequencing device system 114 or the calibration-sequencing system 106, the sequencing device 112 can run one or more calibration cycles, indexing cycles, or genomic sequencing cycles as part of a sequencing run. By executing the calibration-sequencing system 106, for instance, the sequencing device 112 can detect calibration sequences associated with library fragments deposited on a sample-nucleotide slide and run calibration cycles to determine a sequencing parameter corresponding to the sequencing device 112 based on the calibration sequences. In one or more embodiments, the sequencing device 112 utilizes Sequencing by Synthesis (SBS) to sequence nucleic-acid polymers into nucleotide-fragment reads. In addition or in the alternative to communicating across the network 116, in some embodiments, the sequencing device 112 bypasses the network 116 and communicates directly with the server device(s) 102 or the user client device 108.

In some cases, the server device(s) 102 is located at or near a same physical location of the sequencing device 112 or remotely from the sequencing device 112. Indeed, in some embodiments, the server device(s) 102 and the sequencing device 112 are integrated into a same computing device. The server device(s) 102 may run a sequencing system 104 or the calibration-sequencing system 106 to generate, receive, analyze, store, and transmit digital data, such as by receiving base-call data or determining variant calls based on analyzing such base-call data. As suggested by FIG. 1, the sequencing device 112 may send (and the server device(s) 102 may receive) base-call data generated during a sequencing run of the sequencing device 112. By executing software in the form of the sequencing system 104 or the calibration-sequencing system 106, the server device(s) 102 may align nucleotide-fragment reads with a reference genome and determine genetic variants based on the aligned nucleotide-fragment reads. The server device(s) 102 may also communicate with the user client device 108. In particular, the server device(s) 102 can send data to the user client device 108, including a variant call file (VCF), or other information indicating nucleobase calls, sequencing metrics, error data, or other metrics.

In some embodiments, the server device(s) 102 comprise a distributed collection of servers where the server device(s) 102 include a number of server devices distributed across the network 116 and located in the same or different physical locations. Further, the server device(s) 102 can comprise a content server, an application server, a communication server, a web-hosting server, or another type of server.

As further illustrated and indicated in FIG. 1, the user client device 108 can generate, store, receive, and send digital data. In particular, the user client device 108 can receive variant calls and corresponding sequencing metrics from the server device(s) 102 or receive base-call data (e.g., BCL or FASTQ) and corresponding sequencing metrics from the sequencing device 112. Furthermore, the user client device 108 may communicate with the server device(s) 102 or the server device(s) 102 to receive a VCF comprising nucleobase calls and/or other metrics, such as a base-call-quality metrics or pass-filter metrics. The user client device 108 can accordingly present or display information pertaining to variant calls or other nucleobase calls within a graphical user interface to a user associated with the user client device 108.

Although FIG. 1 depicts the user client device 108 as a desktop or laptop computer, the user client device 108 may comprise various types of client devices. For example, in some embodiments, the user client device 108 includes non-mobile devices, such as desktop computers or servers, or other types of client devices. In yet other embodiments, the user client device 108 includes mobile devices, such as laptops, tablets, mobile telephones, or smartphones. Additional details regarding the user client device 108 are discussed below with respect to FIG. 11.

As further illustrated in FIG. 1, the user client device 108 includes a sequencing application 110. The sequencing application 110 may be a web application or a native application stored and executed on the user client device 108 (e.g., a mobile application, desktop application). The sequencing application 110 can include instructions that (when executed) cause the user client device 108 to receive data from the calibration-sequencing system 106 and present, for display at the user client device 108, base-call data (e.g., from a BCL) or data from a VCF.

As further illustrated in FIG. 1, a version of the calibration-sequencing system 106 may be located on the user client device 108 as part of the sequencing application 110 or on the server device(s) 102. Accordingly, in some embodiments, the calibration-sequencing system 106 is implemented by (e.g., located entirely or in part) on the user client device 108. In yet other embodiments, the calibration-sequencing system 106 is implemented by one or more other components of the environment 100, such as the server device(s) 102. In particular, the calibration-sequencing system 106 can be implemented in a variety of different ways across the sequencing device 112, the user client device 108, and the server device(s) 102. For example, the calibration-sequencing system 106 can be downloaded from the server device(s) 102 to the sequencing device 112 and/or the user client device 108 where all or part of the functionality of the calibration-sequencing system 106 is performed at each respective device within the environment 100.

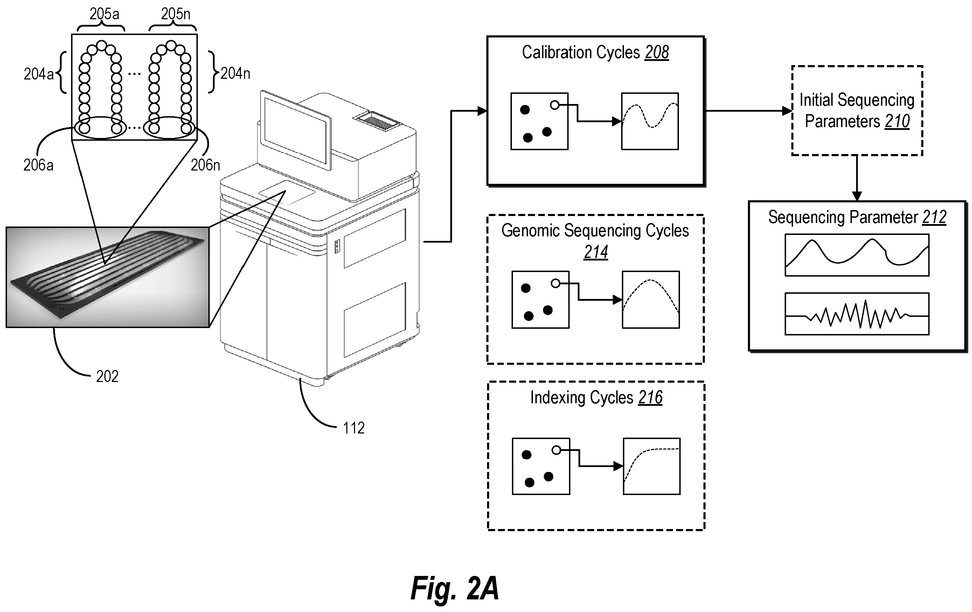

As indicated above, the calibration-sequencing system 106 can detect a nucleotide-sample slide comprising calibration sequences and run calibration cycles to adjust or otherwise determine a sequencing parameter corresponding to a sequencing device. In accordance with one or more embodiments, FIG. 2A illustrates an example of the calibration-sequencing system 106 (i) receiving a nucleotide-sample slide comprising calibration sequences and sample library fragments associated with the sample library fragments, (ii) running calibration cycles to incorporate nucleobases into oligonucleotides corresponding to the calibration sequences, and determining a sequencing parameter corresponding to a sequencing device based on the calibration cycles and the calibration sequences.

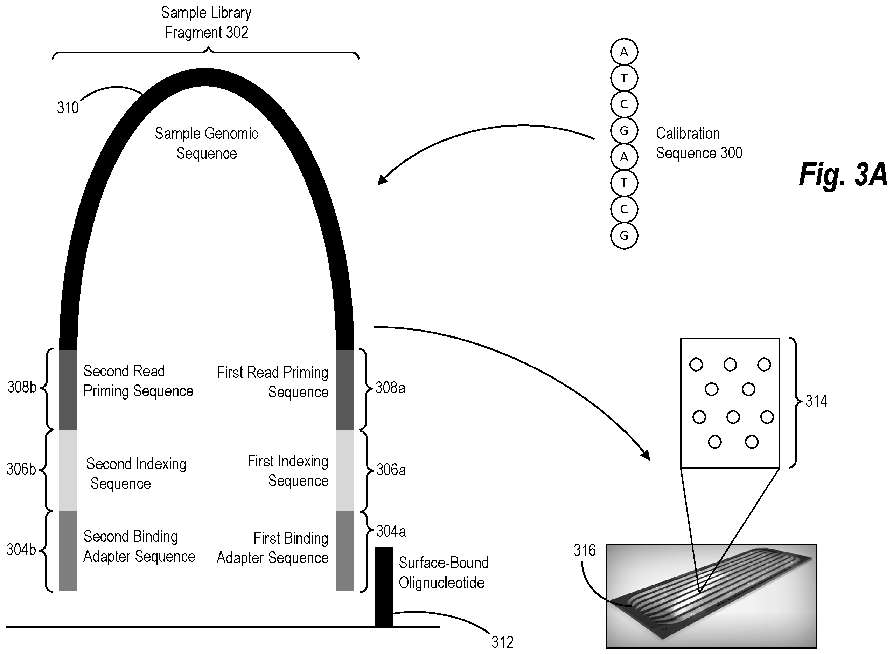

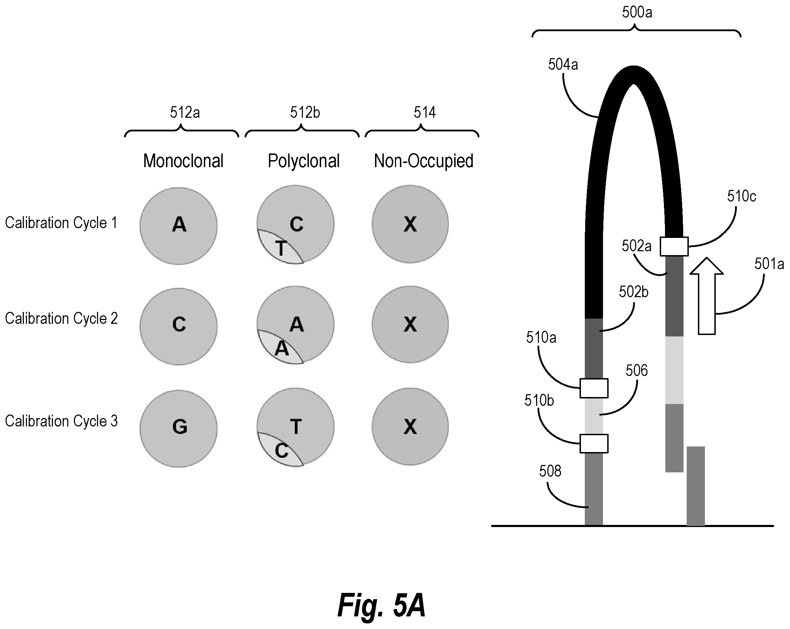

As shown in FIG. 2A, for instance, the calibration-sequencing system 106 receives or detects a nucleotide-sample slide 202 comprising calibration sequences 204a - 204n associated with sample library fragments 205a - 205n. As depicted, in some cases, the calibration sequences 204a - 204n are relatively short, such as 4-8 nucleobases in length. The nucleotide-sample slide 202 includes the sample library fragments 205a - 205n within wells 206a - 206n (e.g., nanowells), and the calibration sequences 204a - 204n are part of the sample library fragments 205a - 205n. Accordingly, the calibration sequences 204a - 204n have been deposited within the wells 206a - 206a (or, in some cases, on a surface of an un-patterned nucleotide-sample slide that lacks wells) as part of seeding clusters of oligonucleotides with the sample library fragments 205a - 205n. Further embodiments of calibration sequences are described below with reference to FIGS. 3A-3B and 5A-5E.

As further shown in FIG. 2A, the calibration-sequencing system 106 performs one or more calibration cycles 208 using the sequencing device 112. During a calibration cycle, for instance, the calibration-sequencing system 106 uses the sequencing device 112 to incorporate nucleobases of one or more nucleobase types into growing oligonucleotides corresponding to the calibration sequences 204a - 204n, such as by adding nucleobases of nucleobase types that complement the nucleobases of the calibration sequences 204a - 204n (e.g., incorporating an A complimenting a T). By adding nucleobases to growing oligonucleotides that compliment or follow the calibration sequences 204a - 204n within a single nucleotide-fragment read or a paired-end nucleotide-fragment read, in some cases, the calibration-sequencing system 106 generates clusters of oligonucleotides that mirror a targeted distribution of nucleobase types within the calibration sequences 204a - 204n.

As suggested above, the calibration-sequencing system 106 either determines or does not determine nucleobase calls for the calibration sequences 204a - 204n during the calibration cycles 208. Accordingly, during a given calibration cycle, the calibration-sequencing system 106 can capture and analyze images of cluster of oligonucleotides that have incorporated nucleobases with tags (e.g., fluorescent tags) complimenting or mirroring the calibration sequences 204a - 204n. But the calibration-sequencing system 106 does not necessarily determine nucleobase calls for the incorporated nucleobases with tags during the given calibration cycle.

During or after the calibration cycles 208, in some embodiments, the calibration-sequencing system 106 detects or otherwise determines initial sequencing parameters 210. For instance, the calibration-sequencing system 106 can determine one or more of the initial sequencing parameters 210 by determining one or more of intensity values for a signal corresponding to a nucleobase, intensity-value boundaries, nucleobase centroids for intensity values, a noise metric, or other various metrics detected or output during a calibration cycle. As explained further below, the initial sequencing parameters 210 can directly or indirectly form a basis for one or more sequencing parameters.

With or without the initial sequencing parameters 210, the calibration-sequencing system 106 determines a sequencing parameter 212 corresponding to the sequencing device 112. To do so, in some cases, the calibration-sequencing system 106 estimates or detects a sequencing parameter anew and directly from the sequencing device 112, adjusts a default or preconfigured sequencing parameter (e.g., equalizer coefficient) corresponding to the sequencing device 112, or estimates a sequencing parameter based on initial sequencing parameters from multiple calibration cycles (e.g., as an average of initial sequencing parameters). Regardless of the approach, in some cases, the calibration-sequencing system 106 can either determine the sequencing parameter 212 directly or indirectly from the sequencing device 112 (or its environment) or adjust an initial sequencing parameter to generate the sequencing parameter 212 based on nucleobase calls corresponding to the calibration sequences 204a - 204n.

As suggested above, the calibration-sequencing system 106 can determine the sequencing parameter 212 by (i) determining a sequencing parameter that had conventionally been determined offline and provided as pre-configured parameters, such as equalizer coefficients or (ii) determining a sequencing parameter learned during a sequencing run on a sequencing device, such as non-linear optical distortion parameters, structured illumination microscopy (SIM) parameters, per-cluster normalization parameters, or offset correction parameters for a particular channel.

As further shown in FIG. 2A, in addition to the calibration cycles 208, the calibration-sequencing system 106 optionally performs genomic sequencing cycles 214 and indexing cycles 216. After performing the calibration cycles 208 for the calibration sequences 204a - 204n and determining the sequencing parameter 212, for instance, the calibration-sequencing system 106 optionally (i) performs the genomic sequencing cycles 214 by incorporating nucleobases that compliment or follow sample genomic sequences within the sample library fragments 205a - 205n and determines nucleobase calls for such incorporated nucleobases and (ii) performs the indexing cycles 216 by incorporating nucleobases that compliment or follow indexing sequences within the sample library fragments 205a - 205n and determines nucleobase calls for such incorporated nucleobases. In some cases, the calibration-sequencing system 106 runs the indexing cycles 216 before or after the genomic sequencing cycles 214. Indeed, contrary to some existing sequencing systems, the calibration-sequencing system 106 sometimes executes the indexing cycles 216 before the genomic sequencing cycles 214 depending on locations of the calibration sequences 204a - 204n within the sample library fragments 205a - 205n, respectively. As noted above, in some embodiments, an indexing cycle for one cluster of oligonucleotides may be performed at a same time as a calibration cycle for another cluster of oligonucleotides-because calibration cycles may be specific to a cluster or clusters of oligonucleotides as outlined by various calibration schemes below.

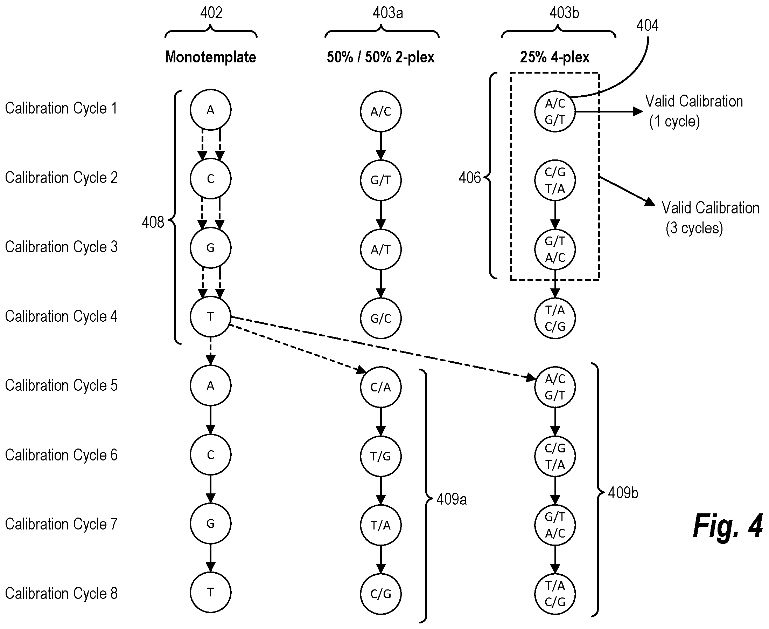

In addition or in the alternative to the calibration sequences 204a - 204n, in some embodiments, the calibration-sequencing system 106 receives or detects a nucleotide-sample slide (e.g., the nucleotide-sample slide 202) comprising calibration nucleobases associated with sample library fragments. For instance, each sample library fragment within a set of sample library fragments may include a single calibration nucleobase. Across the set of sample library fragments, however, a set of calibration nucleobases can exhibit a targeted distribution of different nucleobase types, such as a proportional distribution of four different nucleobase types (e.g., A, T, C, G) across calibration nucleobases. This disclosure provides further examples of such calibration nucleobases below with respect to FIG. 4.

Similar to the calibration sequences 204a - 204n, in certain implementations, the calibration-sequencing system 106 may use calibration nucleobases to determine a sequencing parameter corresponding to the sequencing device 112. For instance, in some embodiments, the calibration-sequencing system 106 performs a calibration cycle using the sequencing device 112 to incorporate complimentary nucleobases into oligonucleotides corresponding to the calibration nucleobases and determine a sequencing parameter based on the calibration cycles and the calibration nucleobases.

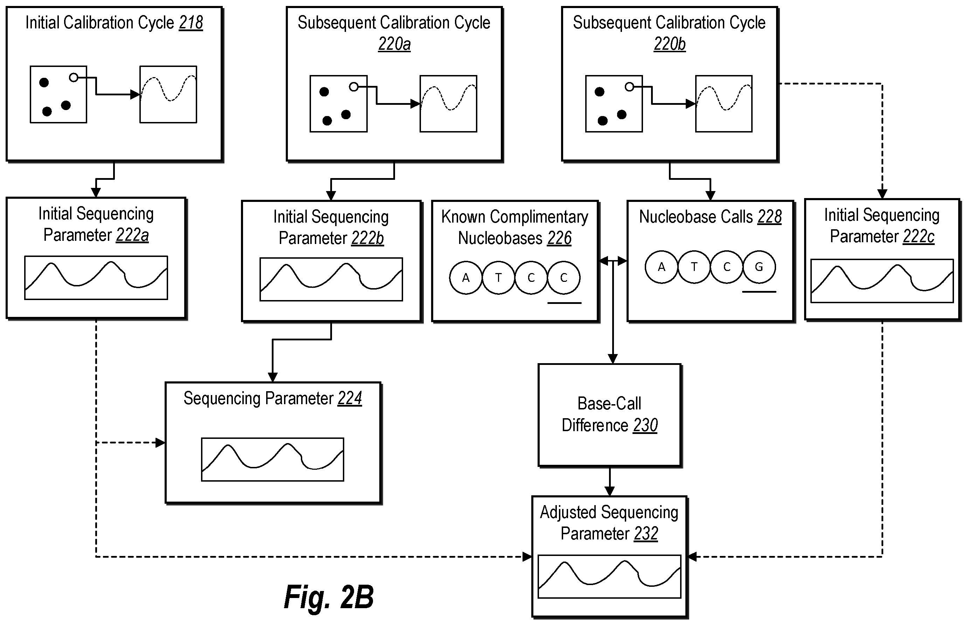

As noted above, the calibration-sequencing system 106 can determine a sequencing parameter corresponding to a sequencing device in a variety of ways. In accordance with one or more embodiments, FIG. 2B depicts the calibration-sequencing system 106 running calibration cycles to (i) directly determine a sequencing parameter detected during or after a calibration cycle from the sequencing device, (ii) determine a sequencing parameter based on initial sequencing parameters detected during multiple calibration cycles, and/or (iii) adjust initial sequencing parameters based on a base-call difference between nucleobase calls for complimentary strands to calibration sequences and known complimentary nucleobases for calibration sequences.

As noted above, the calibration-sequencing system 106 can detect a sequencing parameter directly from a sequencing device during or after one or more calibration cycles. As shown in FIG. 2B, for instance, the calibration-sequencing system 106 performs an initial calibration cycle 218 to incorporate a nucleobase complimenting or matching a known nucleobase of one or more calibration sequences into oligonucleotides within a cluster. During or after the initial calibration cycle 218, the calibration-sequencing system 106 detects an initial sequencing parameter 222a from a camera, sensor, or other component of a sequencing device. In some cases, the calibration-sequencing system 106 does not adjust the initial sequencing parameter 222a, but uses the initial sequencing parameter 222a as a sequencing parameter to facilitate determining nucleobase calls during subsequent genomic sequencing cycles or indexing cycles.

As an example of direct detection during or after the initial calibration cycle 218, in some cases, the calibration-sequencing system 106 detects or measures a brightness parameter for a particular channel (e.g., an image for a particular fluorescent label) corresponding to a particular cluster of oligonucleotides within a nucleotide-sample slide. Additionally or alternatively, the calibration-sequencing system 106 detects or measures noise or background intensity values of a specific nucleobase that is not incorporated into an oligonucleotide during the initial calibration cycle 218. As a further example, the calibration-sequencing system 106 detects or measures a color-channel-to-color-channel crosstalk parameter indicating spectral overlap between one or more fluorescently labeled nucleobases during the initial calibration cycle 218.

As further noted above, the calibration-sequencing system 106 can determine a sequencing parameter based on initial sequencing parameters detected during multiple calibration cycles. As shown in FIG. 2B, in certain implementations, the calibration-sequencing system 106 performs both the initial calibration cycle 218 and a subsequent calibration cycle 220a. The subsequent calibration cycle 220a can occur either immediately after the initial calibration cycle 218 or after one or more additional calibration cycles following the initial calibration cycle 218.

In the initial calibration cycle 218 and the subsequent calibration cycle 220a, the calibration-sequencing system 106 determines the initial sequencing parameter 222a and an initial sequencing parameter 222b. Depending on the type of sequencing parameter, in certain implementations, the initial sequencing parameters 222a and 222b represent a same type of sequencing parameter, such as by determining initial per-cluster-brightness parameter for a particular channel for both the initial sequencing parameters 222a and 222b or determining initial color-channel-to-color-channel crosstalk parameters for both the initial sequencing parameters 222a and 222b. Depending on the initial sequencing parameters 222a and 222b, in some cases, the calibration-sequencing system 106 incorporates nucleobases of a same nucleobase type during the initial calibration cycle 218 and the subsequent calibration cycle 220a to facilitate determining an accurate sequencing parameter.

As further shown in FIG. 2B, in some cases, the calibration-sequencing system 106 determines a sequencing parameter 224 based on the initial sequencing parameters 222a and 222b. For instance, in certain embodiments, the calibration-sequencing system 106 determines an average or a weighted average of the initial sequencing parameters 222a and 222b to determine the sequencing parameter 224. In some such cases, the calibration-sequencing system 106 weights an earlier or later calibration cycle more heavily. In contrast to averaging, in some cases, the calibration-sequencing system 106 incrementally adjusts the initial sequencing parameter 222a (e.g., by increasing or decreasing a value up to or within a threshold-value change) based on the initial sequencing parameter 222b to determine the sequencing parameter 224.

In addition or in the alternative to determining a sequencing parameter from a combination of initial sequencing parameters from multiple calibration cycles (e.g., the initial calibration cycle 218 and the subsequent calibration cycle 220a), the calibration-sequencing system 106 can adjust one or more initial sequencing parameters based on a base-call difference between nucleobase calls for (or complimentary to) calibration sequences and known nucleobases for the calibration sequences.

As shown in FIG. 2B, for instance, the calibration-sequencing system 106 performs a subsequent calibration cycle 220b. During the subsequent calibration cycle 220b, the calibration-sequencing system 106 determines an initial sequencing parameter 222c and determines nucleobase calls 228 for nucleobases incorporated into a set of growing oligonucleotides corresponding to one or more calibration sequences. Depending on the corresponding calibration sequences, the incorporated nucleobases may be of a same nucleobase type or have a targeted distribution of different nucleobase types.

Based on a comparison of known complimentary nucleobases 226 for the relevant calibration sequences, the calibration-sequencing system 106 determines a base-call difference 230. As shown in FIG. 2B, for instance, the base-call difference 230 may be a nucleobase call of one nucleobase type (e.g., G) when a known nucleobase (or complimentary nucleobase) for the relevant calibration sequences is a different nucleobase type (e.g., A). The calibration-sequencing system 106 further adjusts either or both of the initial sequencing parameters 222a and 222c based on the base-call difference 230 to generate an adjusted sequencing parameter 232. To generate the adjusted sequencing parameter 232, for instance, the calibration-sequencing system 106 may adjust a nucleobase centroid for intensity values or intensity-value boundaries for the nucleobase to correct for the base-call difference 230 and reflect the known nucleobase (or complimentary nucleobase) that should have been called based on the relevant calibration sequences.

The calibration-sequencing system 106 may rely on a base-call difference to adjust a sequencing parameter when, for instance, nucleobase calls corresponding to a set of calibration sequences differ from a targeted distribution exhibited by the set of calibration sequences. For instance, in some cases, a set of calibration sequences may have a targeted distribution of nucleobase types at a given calibration-sequence position (e.g., a four-plex of A/C/G/T at 25%/25%/25%/25% in a given position). When the calibration-sequencing system 106 determines nucleobase calls that do not compliment the targeted distribution of nucleobase types (e.g., T/G/C/A at 23%/27%/25%/25% in a given position), the calibration-sequencing system 106 can adjust a sequencing parameter (e.g., a nucleobase centroid, intensity-value boundaries, color-channel-to-color-channel crosstalk parameter) to a degree or value that would have produced the targeted distribution (or complimentary targeted distribution) of nucleobase types exhibited by the known calibration sequences.

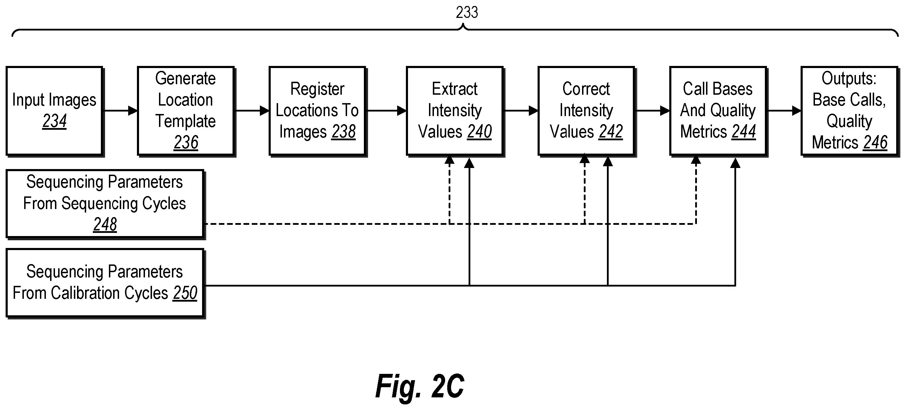

After determining one or more sequencing parameters, the calibration-sequencing system 106 can apply such sequencing parameters at various stages during an indexing cycle or a genomic sequencing cycle. In accordance with one or more embodiments, FIG. 2C illustrates the calibration-sequencing system 106 or the sequencing device system 114 applying one or more sequencing parameters determined during one or more calibration cycles to (i) extract intensity values from an image of a cluster of oligonucleotides, (ii) correct the extracted intensity values, or (iii) determine nucleobase calls for incorporated nucleobases during either a genomic sequencing cycle or an indexing cycle.

As shown in FIG. 2C, the calibration-sequencing system 106 or the sequencing device system 114 performs a sequencing cycle 233, such as by performing a genomic sequencing cycle or an indexing cycle. FIG. 2C depicts selected stages of the sequencing cycle 233 executed after one or more calibration cycles. As part of or the sequencing cycle 233, for instance, the calibration-sequencing system 106 or the sequencing device system 114 captures and inputs images 234 of various sections (e.g., tiles) of the nucleotide-sample slide 202 into on-device software, such as the sequencing device system 114. In some cases, the images depict fluorescent light emitted by fluorescently tagged nucleobases incorporated into clusters of oligonucleotides during the sequencing cycle 233.

After inputting the images 234, the calibration-sequencing system 106 or the sequencing device system 114 generates a location template 236 by identifying locations of the clusters of oligonucleotides captured by the images 234. The calibration-sequencing system 106 or the sequencing device system 114 further registers locations to images 238 by registering or mapping the identified locations of the clusters of oligonucleotides to the images 234 using a coordinate system (e.g., X and Y positions). As indicated by FIG. 2C, the calibration-sequencing system 106 or the sequencing device system 114 optionally determines one or more sequencing parameters 248 from the sequencing cycle 233 using existing methods. Accordingly, the one or more sequencing parameters 248 differ significantly from sequencing parameters 250 from calibration cycles and are subject to one or more of the technical problems described above.

As further shown in FIG. 2C, after registering cluster locations, the calibration-sequencing system 106 or the sequencing device system 114 extracts intensity values 240 from the images 234. For instance, the calibration-sequencing system 106 or the sequencing device system 114 converts the fluorescent light corresponding to a cluster to the intensity values 240 in numeric format. When extracting the intensity values 240, the calibration-sequencing system 106 or the sequencing device system 114 can apply one or more of the sequencing parameters 250 from calibration cycles. For instance, the calibration-sequencing system 106 or the sequencing device system 114 can apply equalizer coefficients, which were determined during previous calibration cycles, to pixels as part of extracting the intensity values 240 from the images 234. In some such cases, the calibration-sequencing system 106 or the sequencing device system 114 applies equalizer coefficients as described by Equalization-Based image Processing and Spatial Crosstalk Attenuator, U.S. Patent No. 11,188,778 (issued Nov. 30, 2021), which is hereby incorporated by reference in its entirety.

After extracting intensity values from images, the calibration-sequencing system 106 or the sequencing device system 114 corrects the intensity values 242 using one or more of the sequencing parameters 250 from calibration cycles. For instance, in some cases, the calibration-sequencing system 106 or the sequencing device system 114 applies or factors in a normalization parameter, a noise level, or a signal-to-noise-ratio metric to one or more of the intensity values 240 as part of a correction (e.g., signal correction function). As a further example, in certain embodiments, the calibration-sequencing system 106 or the sequencing device system 114 applies a phasing coefficient to one or more of the intensity values 240 as part of a correction (e.g., signal correction function). In some such cases, the calibration-sequencing system 106 or the sequencing device system 114 applies phasing coefficients as described by Generating Cluster-Specific-Signal Corrections for Determining Nucleotide-Base Calls, U.S. Application No. 63/285,187 (filed Dec. 2, 2021), which is hereby incorporated by reference in its entirety.

As further shown in FIG. 2C, after correcting intensity values, the calibration-sequencing system 106 or the sequencing device system 114 calls bases and quality metrics 244. For instance, in some cases, the calibration-sequencing system 106 or the sequencing device system 114 determines nucleobase calls for one or more nucleobases incorporated into the imaged clusters of oligonucleotides during the sequencing cycle 233 based on corrected intensity values. As part of determining nucleobase calls and quality metrics 244, the calibration-sequencing system 106 or the sequencing device system 114 can apply or factor in one or more of the sequencing parameters 250 from calibration cycles. For instance, the calibration-sequencing system 106 or the sequencing device system 114 can determine nucleobase calls for incorporated nucleobases based on corrected intensity values according to nucleobase centroids for intensity values and/or intensity-value boundaries for particular nucleobases. As a further example, in some cases, the calibration-sequencing system 106 or the sequencing device system 114 can determine quality metrics of nucleobase calls, such as base-call-quality metrics, according to an algorithm or function that factors in per-cluster-intensity parameters for a particular channel or per-cluster-brightness parameters for the particular channel.

After calling bases and quality metrics 244, as further shown in FIG. 2C, the calibration-sequencing system 106 or the sequencing device system 114 can generate outputs 246, such as base calls and quality metrics. In some cases, for instance, the calibration-sequencing system 106 or the sequencing device system 114 generates base-call data for the sequencing cycle 233 and other sequencing cycles in a sequencing run in the form of a BCL file.

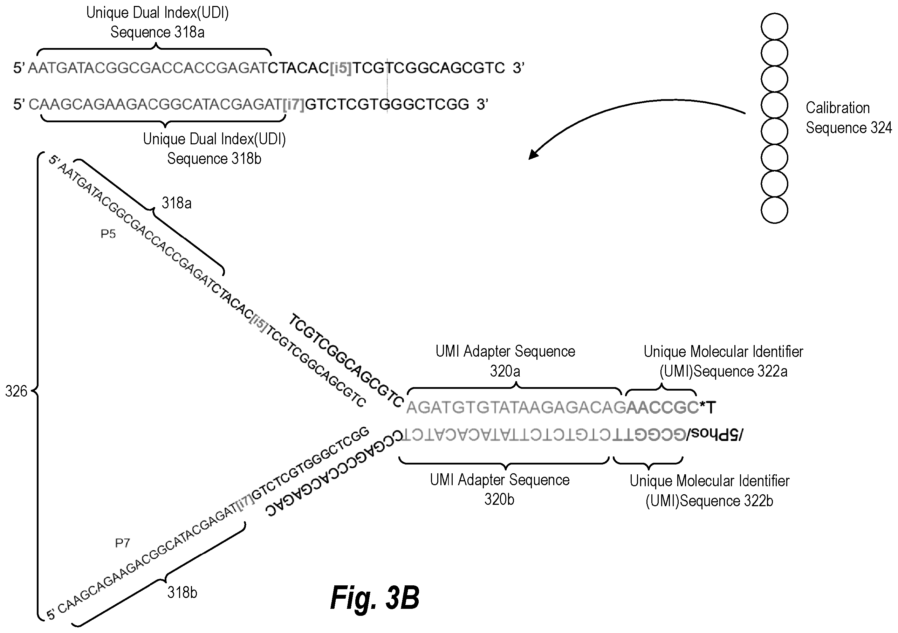

As indicated above, a calibration sequence may be part or all of a non-genomic sequence and/or non-transcriptomic sequence within a sample library fragment or an associated y-adapter. In accordance with one or more embodiments, FIGS. 3A-3B depict the calibration-sequencing system 106 inserting or ligating a calibration sequence within (or as part of) a non-genomic and non-transcriptomic sequence that is either within or otherwise associated with a sample library fragment. In particular, FIG. 3A depicts the calibration-sequencing system 106 adding (or detecting a nucleotide-sample slide comprising) a calibration sequence in between one or more of a binding adapter sequence, an indexing sequence, a read priming sequence, or a sample genomic sequence. FIG. 3B depicts the calibration-sequencing system 106 adding (or detecting a nucleotide-sample slide comprising) a calibration sequence as part or all of a unique molecular identifier (UMI) sequence or a unique dual index (UDI) sequence from a y-adapter for a sample library fragment.

As shown in FIG. 3A, for instance, a calibration sequence 300 is inserted or ligated into a sample library fragment 302. As depicted, the calibration sequence 300 is relatively short (e.g., 4-8 nucleobases long). But the calibration-sequencing system 106 may use a calibration sequence of any suitable length. As shown, the sample library fragment 302 includes a sample genomic sequence 310, such as a gDNA fragment or, alternatively, includes a sample transcriptomic sequence, such as a cDNA fragment. But the calibration sequence 300 is not added or inserted into the sample genomic sequence 310 or, alternatively, a sample transcriptomic sequence.

As suggested above, the calibration-sequencing system 106 may detect or receive a nucleotide-sample slide 316 comprising the calibration sequence 300 in between various non-genomic and non-transcriptomic sequences. As suggested by FIG. 3A, in some cases, the calibration sequence 300 is (i) between a first binding adapter sequence 304a and a first indexing sequence 306a, (ii) between a second binding adapter sequence 304b and a second indexing sequence 306b, (iii) between the first indexing sequence 306a and a first read priming sequence 308a, (iv) between the second indexing sequence 306b and a second read priming sequence 308b, (v) between the first read priming sequence 308a and the sample genomic sequence 310 (or sample transcriptomic sequence), or (vi) between the second read priming sequence 308b and the sample genomic sequence 310 (or sample transcriptomic sequence).

In some cases, the various adapter, primer, and indexing sequences between which a calibration sequence is inserted can take the form of known sequences that are commercially available. For instance, in certain embodiments, the first binding adapter sequence 304a constitutes a P7 binding adapter sequence, the first indexing sequence 306a constitutes an i7 indexing sequence, the first read priming sequence 308a constitutes a first read priming sequence (e.g., sbs12, B15ME), the second read priming sequence 308b constitutes a second read priming sequence (e.g., sbs3', A14ME), the second indexing sequence 306b constitutes an i5 indexing sequence, and the second binding adapter sequence 304b constitutes a P5 binding adapter sequence. As described below, FIG. 3B depicts additional specific examples of an adapter binding sequence (e.g., a UDI sequence).

As further suggested above, the calibration-sequencing system 106 may detect or receive the nucleotide-sample slide 316 comprising the calibration sequence 300 as part or all of certain non-genomic and non-transcriptomic sequences. As suggested by FIG. 3A, for instance, the calibration sequence 300 may be part or all of a nucleotide sequence forming the first binding adapter sequence 304a, the first indexing sequence 306a, the second binding adapter sequence 304b, the second indexing sequence 306b, or a surface-bound oligonucleotide 312. While the surface-bound oligonucleotide 312 is not part of the sample library fragment 302, the surface-bound oligonucleotide 312 is attached to a surface (e.g., well) of the nucleotide-sample slide 316 and anneals with either the first binding adapter sequence 304a or the second binding adapter sequence 304b.

As further shown in FIG. 3A, the sample library fragment 302 is deposited within one of wells 314 within the nucleotide-sample slide 316. As suggested above, additional sample library fragments comprising or otherwise associated with calibration sequences are likewise deposited within the wells 314 of the nucleotide-sample slide 316. The nucleotide-sample slide 316 can subsequently be inserted in and detected by a sequencing device (e.g., the sequencing device 112).

Turning now to FIG. 3B, in this figure, a calibration sequence 324 is inserted or integrated into a y-adapter 326 of a sample library fragment. As part of or after using the y-adapter 326 to prepare the sample library fragment, for instance, the calibration-sequencing system 106 can insert or integrate the calibration sequence 324 into part or all of a unique molecular identifier (UMI) sequence 322a or a UMI sequence 322b, which are shown near UMI adapter sequences 320a and 320b, respectively. In some cases, the UMI sequences 322a and/or 322b may be non-random UMI sequences. Alternatively, in some embodiments, the calibration-sequencing system 106 inserts or integrates the calibration sequence 324 into part or all of a unique dual index (UDI) sequence 318a or a UDI sequence 318b.

As indicated above, the calibration-sequencing system 106 can perform calibration cycles in conjunction with genomic sequencing cycles and indexing cycles-where the indexing cycle and corresponding indexing sequence occurs before or after the genomic sequencing cycle and corresponding sample genomic sequence. When the calibration sequence 324 is part or all of the UDI sequence 318a or the UDI sequence 318b, in some embodiments, the calibration-sequencing system 106 performs indexing cycles for an indexing sequence before genomic sequencing cycles for a sample genomic sequence from a sample library fragment. By contrast, when the calibration sequence 324 is part or all of the UMI sequence 322a or the UMI sequence 322b, in certain implementations, the calibration-sequencing system 106 performs genomic sequencing cycles for a sample genomic sequence before indexing cycles for an indexing sequence from a sample library fragment.

With respect to either FIG. 3A or FIG. 3B, when inserting or integrating a calibration sequence into a binding adapter sequence, such as a UDI sequence, the calibration-sequencing system 106 can either use nucleobases already present in the binding adapter sequence (e.g., upstream of gDNA) as the calibration sequence or add synthetic nucleobases as a calibration sequence to the binding adapter sequence, which is attached on the end of sample genomic sequence during library preparation process. To use nucleobases already present in the binding adapter sequence, the calibration-sequencing system 106 can shorten a read priming sequence by a number of nucleobases. Consequently, the initially incorporated bases of cycles (e.g., calibration cycles) correspond to the binder adapter sequence. To add synthetic nucleobase as a calibration sequence to the binding adapter sequence, the calibration-sequencing system 106 can add (or embed) synthetic nucleobases into the binding adapter sequence (e.g., by lengthening the binding adapter sequence with common nucleobases).

Whether using existing nucleobases or adding synthetic nucleobases to a binding adapter sequence for a calibration sequence, the calibration-sequencing system 106 can incorporate the same calibration sequence at the beginning of a nucleotide-fragment read for every cluster of oligonucleotides in a nucleotide-sample slide-without needing to use up permutations or combinations of indexing sequences. By using the binding adapter sequence for a calibration sequence, the calibration-sequencing system 106 may provide a more accurate calibration because the already present binding adapter sequence would not create a variation source (template to primer hybridization efficiency) that could change by using each different primer annealing sequencing and template hybridization.

Regardless of a calibration sequence's location with respect to a sample library fragment, the calibration-sequencing system 106 can arrange or order the nucleobases making up calibration sequences and nucleobases being incorporated into growing oligonucleotides during calibration sequences in a variety of ways. In accordance with one or more embodiments, FIG. 4 depicts examples of different calibration sequences or calibration nucleobases having different targeted distributions of nucleobase types. As noted above, by using targeted distributions of nucleobase types, the calibration-sequencing system 106 can improve an accuracy of sequencing parameters and corresponding nucleobase calls by eliminating (and evening out a distribution) a skewed distribution of irregularly distributed genomic samples.

As shown in FIG. 4, for instance, the calibration-sequencing system 106 can use a calibration sequence 402 of nucleobases having a proportional distribution of four nucleobase types across calibration cycles. In particular, the calibration sequence 402 includes a proportional distribution of A, C, G, and T across calibration cycles. In some cases, the calibration sequence 402 spans four nucleobases with a different nucleobase type at four calibration-sequence positions; in other cases, the calibration sequence 402 spans eight nucleobases with one of four different nucleobase types at two of eight calibration-sequence positions (and corresponding calibration cycles) shown in FIG. 4. The calibration sequence 402 can represent a monotemplate that the calibration-sequencing system 106 can use as a calibration sequence in each cluster of oligonucleotides or a subset of clusters in a nucleotide-sample slide (e.g., as part of sample library fragments). In some cases, for instance, the calibration-sequencing system 106 uses a set of calibration sequences that each comprise a same or a single sequence of nucleobases that follow the calibration sequence 402 or a similar monotemplate sequence. Whether in a single cluster or in multiple clusters of oligonucleotides, as shown in FIG. 4, the calibration-sequencing system 106 can perform calibrations cycles 1-8 to determine one or more sequencing parameters based on the distribution of nucleobase types in the calibration sequence 402.

As further shown in FIG. 4, in certain embodiments, the calibration-sequencing system 106 uses a set of calibration sequences 403a having a proportional distribution of two nucleobase types (or a combination of two nucleobase types) at different calibration-sequence positions and corresponding calibration cycles. The set of calibration sequences 403a represents a 2-plex-template-calibration-sequence set with approximately 50% of one nucleobase type at a calibration-sequence position and approximately 50% of another nucleobase type at the same calibration-sequence position. Accordingly, a given calibration sequence within the set of calibration sequences 403a has an approximately 50% probability of comprising one of two different nucleobase types at a calibration-sequence position. Calibration sequences from the set of calibration sequences 403a can likewise be distributed to each cluster of oligonucleotides or to a subset of clusters in a nucleotide-sample slide.

As illustrated by FIG. 4, the set of calibration sequences 403a has a proportional distribution of two nucleobase types (e.g., A and C) at a first calibration-sequence position corresponding to a first calibration cycle and a proportional distribution of two different nucleobase types (e.g., G and T) at a second calibration-sequence position corresponding to a second calibration cycle. As the calibration-sequence positions and calibration cycles continue, the set of calibration sequences 403a has a proportional distribution of a different combination of two nucleobase types (e.g., A and T) at a third calibration-sequence position corresponding to a third calibration cycle and a proportional distribution of another different combination of two nucleobase types (e.g., G and C) at a fourth calibration-sequence position corresponding to a fourth calibration cycle. In some cases, the set of calibration sequences 403a continues with a calibration sequence set 409a exhibiting a proportional distribution of combinations of two nucleobase types at calibration-sequence positions 5-8 and corresponding calibration cycles 5-8.

As further shown in FIG. 4, in one or more embodiments, the calibration-sequencing system 106 uses a set of calibration sequences 403b having a proportional distribution of four nucleobase types (or a combination of four nucleobase types) at different calibration-sequence positions and corresponding calibration cycles. The set of calibration sequences 403b represents a 4-plex-template-calibration-sequence set with approximately 25% of four different nucleobase types (e.g., A, C, G, T) at a given calibration-sequence position. Accordingly, a given calibration sequence within the set of calibration sequences 403b has an approximately 25% probability of comprising one of four different nucleobase types at a calibration-sequence position. Calibration sequences from the set of calibration sequences 403b can likewise be distributed to each cluster of oligonucleotides or to a subset of clusters in a nucleotide-sample slide.

As further illustrated by FIG. 4, the set of calibration sequences 403b has a proportional distribution of four nucleobase types (e.g., A, C, G, T) at each calibration-sequence position and each corresponding calibration cycle. The set of calibration sequences 403b can likewise span various calibration-sequence positions and calibration cycles, such as 3-8 calibration-sequence positions and 3-8 corresponding calibration cycles. As shown by calibration-sequence set 406, in some embodiments, the calibration-sequencing system 106 can run three calibration cycles with the set of calibration sequences 403b spanning three calibration-sequence positions (e.g., each calibration sequence spanning three nucleobases) and thereby validly calibrate a sequencing device and determine one or more sequencing parameters.

As suggested above, in some embodiments, the calibration-sequencing system 106 uses calibration nucleobases to calibrate a sequencing device and determine one or more sequencing parameters. As shown in FIG. 4, for instance, the set of calibration nucleobases 404 have a proportional distribution of four different nucleobase types (e.g., A, C, G, T) at a single calibration-sequence position and a single calibration cycle. Indeed, in some embodiments, the calibration-sequencing system 106 can run a single calibration cycle with the set of calibration nucleobases 404 at a single calibration-sequence position (e.g., one calibration nucleobase within each sample library fragment) and thereby validly calibrate a sequencing device and determine one or more sequencing parameters.

As further suggested by FIG. 4, in some embodiments, the calibration-sequencing system 106 uses different combinations of the calibration sequence 402, the set of calibration sequences 403a, and the set of calibration sequences 403b for a sequencing run. As one example path for calibration cycles 1-8, in certain implementations, the calibration-sequencing system 106 (i) uses a set of calibration sequences that follow a monotemplate nucleotide sequence 408 from the calibration sequence 402 to perform calibration cycles 1-4 and (ii) subsequently uses the calibration sequence set 409a with a 2-plex-template-calibration-sequence set to perform calibration cycles 4-8. As another example path for calibration cycles 1-8, in certain implementations, the calibration-sequencing system 106 (i) uses a set of calibration sequences that follow a monotemplate nucleotide sequence 408 from the calibration sequence 402 to perform calibration cycles 1-4 and (ii) subsequently uses the calibration sequence set 409b with a 4-plex-template-calibration-sequence set to perform calibration cycles 4-8. The calibration-sequencing system 106 can likewise combine various other calibration sequences described herein during calibration cycles.

As noted above, the calibration-sequencing system 106 can detect or receive a nucleotide-sample slide comprising calibration sequences placed in a variety of locations with respect to sample library fragments and deposited in a variety of clusters. In accordance with one or more embodiments, FIGS. 5A-5E depict different calibration schemes of integrating calibration sequences within (or nearby) sample library fragments and distributing such sample library fragments in different clusters of oligonucleotides in a nucleotide-sample slide. As further described below, in some cases, the calibration-sequencing system 106 combines different calibration schemes depicted in FIGS. 5A-5E.

As indicated by FIG. 5A, under a first calibration scheme, the calibration-sequencing system 106 receives a nucleotide-sample slide comprising a set of calibration sequences having a well-diversified set of nucleobase types both within a given calibration cycle and across calibration cycles. In some cases, for instance, the calibration-sequencing system 106 uses a set of calibration sequences comprising (i) a first calibration sequence including different nucleobase types according to a first order of nucleobases (e.g., ordered A, C, G, T in a sequence), (ii) a second calibration sequence including the different nucleobase types according to a second order of nucleobases (e.g., ordered C, G, T, A in a sequence), (iii) a third calibration sequence including the different nucleobase types according to a third order of nucleobases (e.g., ordered G, T, A, C in a sequence), and (iv) a fourth calibration sequence including the different nucleobase types according to a fourth order of nucleobases (e.g., ordered T, A, C, G in a sequence). Each of the first, second, third, and fourth calibration sequences may likewise be part of first, second, third, and fourth subsets of calibration sequences comprising different nucleobase types according to the different orders just described.

As noted above, some existing sequencing systems bias or skew sequencing parameters by calibrating a sequencing device using genomic samples of relatively low diversity, such as repeat mononucleotides, repeat dinucleotides, high G/C repeats, or other motifs. Indeed, some existing calibration methods function on an erroneous assumption that an existing sequencing system calibrates on a well-diversified genomic sample. By running calibration cycles using a set of well-diversified calibration sequences with known nucleobase types, however, the calibration-sequencing system 106 can determine sequencing parameters, such as gaussian covariance matrices or intensity-value boundaries for particular nucleobases, that are consistently known a priori and are not subject to the bias of relying on genomic samples of relatively low nucleobase-type diversity.

As shown in Table 1 below, for instance, the calibration-sequencing system 106 can perform calibration cycles using a set of well-diversified calibration sequences comprising four different calibration sequences that provide a uniform composition of nucleobase types at each of four calibration cycles. In some embodiments, as shown in Table 1 below, such a set of well-diversified calibration sequences can take the form of a 4-plex-template-calibration-sequence set with approximately 25% of four different nucleobase types (e.g., A, C, G, T) at a given calibration-sequence position.

**Table 1**

| | Calibr. Cycle 1 | Calibr. Cycle 2 | Calibr. Cycle 3 | Calibr. Cycle 4 |
|---|---|---|---|---|
| Calibr. Sequence 1 | A | C | G | T |
| Calibr. Sequence 2 | C | G | T | A |
| Calibr. Sequence 3 | G | T | A | C |
| Calibr. Sequence 4 | T | A | C | G |

As depicted in FIG. 5A, in some cases, the calibration-sequencing system 106 detects or receives a nucleotide-sample slide comprising a given calibration sequence, from such a set of well-diversified calibration sequences, that has been integrated within a sample library fragment 500a at locations 510a, 510b, or 510c. When integrated at location 510a, for instance, a calibration sequence is located between a second read priming sequence 502b and an indexing sequence 506 or, alternatively, integrated as part or all of the second read priming sequence 502b or the indexing sequence 506.

In some cases, indexing sequences are double stranded (i.e., arranged as nucleobase pairs) and span 8, 10, or 20 nucleobases long. By using such indexing sequences, the calibration-sequencing system 106 theoretically has approximately 4¹⁶, 4²⁰, or 4⁴⁰ unique combinations for indexing respectively. After factoring Hamming edit distance requirements, the number of permutations available for biological sample labeling in an indexing sequence will decrease, but should still be sufficient to allow incorporation of calibration cycles. As a consequence of the number of unique combinations, the calibration-sequencing system 106 can leverage a few indexing cycles to become calibration cycles. As suggested above, to perform calibration cycles using indexing sequences, the calibration-sequencing system 106 can perform calibration cycles followed by indexing cycles and subsequently genomic sequencing cycles.

When integrated at location 510b, a calibration sequence is located between the indexing sequence 506 and a binding adapter sequence 508 or, alternatively, integrated as part or all of the indexing sequence 506 or the binding adapter sequence 508. For instance, the calibration sequence may be part or all of a non-random UMI sequence, as depicted in FIG. 3B. In some such cases, non-random UMI sequences are ligated to ends of a sample genomic sequence followed by a Polymerase Chain Reaction (PCR) to add or finish off binding adapter sequences (e.g., P7 and P5) and the index sequences.

When integrated at location 510c, a calibration sequence is located between a first read priming sequence 502a and a sample genomic sequence 504a or, alternatively, integrated as part or all of the first read priming sequence 502a. As indicated by FIG. 5A, in some embodiments, a sequencing primer 501a binds to the first read priming sequence 502a. During a sequencing run, an enzyme adds or incorporates nucleobase by nucleobase to the sequencing primer 501a to generate a nucleotide-fragment read that is complimentary to a corresponding nucleotide sequence from the sample library fragment 500a. Accordingly, in some cases, the calibration-sequencing system 106 uses an enzyme (e.g., polymerase enzyme) to incorporate nucleobases that add to the sequencing primer 501a and that are complimentary to a calibration sequence at location 510c. While the ordering of sequences and location of a calibration sequence may differ, the calibration-sequencing system 106 can likewise use an enzyme and a sequencing primer to incorporate nucleobases complimentary to a calibration sequence at other locations described or depicted by this disclosure.

As further shown in FIG. 5A, a given calibration sequence from a set of well-diversified calibration sequences according to the first calibration scheme can be (i) integrated within a sample library fragment deposited in a monoclonal cluster of oligonucleotides 512a (e.g., within a well of a nucleotide-sample slide) or (ii) integrated within different sample library fragments deposited in a polyclonal cluster of oligonucleotides 512b (e.g., within a well of a nucleotide-sample slide). For comparison, FIG. 5A also depicts a non-occupied well 514 that lacks a calibration sequence or a sample library fragment and can be used to determine certain sequencing parameters, such as in-well background intensity values, by comparing intensity values from non-occupied wells with intensity values from wells comprising sample library fragments and/or calibration sequences. Consistent with the description above, the calibration-sequencing system 106 incorporates nucleobases of a first nucleobase type (e.g., A) into the monoclonal cluster of oligonucleotides 512a during a first calibration cycle; incorporates nucleobases of a second nucleobase type (e.g., C) into the monoclonal cluster of oligonucleotides 512a during a second calibration cycle; and incorporates nucleobases of a third nucleobase type (e.g., G) into the monoclonal cluster of oligonucleotides 512a during a third calibration cycle.

When a given calibration sequence from a set of well-diversified calibration sequences according to the first calibration scheme is integrated within the polyclonal cluster of oligonucleotides 512b, for example, the given calibration sequence can be part of select known locations within a well. When running calibration cycles that cover the polyclonal cluster of oligonucleotides 512b, for instance, the calibration-sequencing system 106 incorporates nucleobases of the second and fourth nucleobase types (e.g., C and T) into the polyclonal cluster of oligonucleotides 512b during the first calibration cycle; incorporates nucleobases of the first nucleobase type (e.g., A) into the polyclonal cluster of oligonucleotides 512b during the second calibration cycle; and incorporates nucleobases of the fourth and second nucleobase types (e.g., T and C) into the polyclonal cluster of oligonucleotides 512b during the third calibration cycle.

Because a given calibration sequence from a set of well-diversified calibration sequences would have a diversity of nucleobase types across calibration cycles, it would be unlikely to find two of the same calibration sequence often together in a polyclonal cluster. In some cases, the set of well-diversified calibration sequences can have different calibration sequences integrated into (and specific for) each sample library fragment. Accordingly, with a set of well-diversified calibration sequences according to the first calibration scheme, monoclonal clusters each comprise incorporated nucleobases that emit a unique and pure light signal during a calibration cycle. But polyclonal clusters comprise incorporated nucleobases that together likely emit a diverse signal due to specific calibration sequences being integrated into different sample library fragments that seeded the polyclonal cluster.

As depicted in Table 1 above or otherwise described, by running calibration cycles using a set of well-diversified calibration sequences according to the first calibration scheme, the calibration-sequencing system 106 can more accurately determine sequencing parameters that are often skewed during a learning process for genomic sequencing cycles. For instance, the calibration-sequencing system 106 can determine more accurate non-linear optical distortion parameters during calibration cycles rather than using early genomic sequencing cycles to execute a non-linear mapping function that converts theoretical locations of wells into actual locations observed in images of nucleotide-sample slide sections.

As further examples, the calibration-sequencing system 106 can determine more accurate structured illumination microscopy (SIM) parameters, per-cluster normalization parameters, or offset correction parameters for a particular channel by running calibration cycles using a set of well-diversified calibration sequences rather than learning such parameters during early and unpredictable genomic sequencing cycles. As yet another example, the calibration-sequencing system 106 can determine more accurate equalizer coefficients by running calibration cycles according to the first calibration scheme rather than estimated such equalizer coefficients out of context and offline for each sequencing device using genomic sequencing data. Rather than learning such sequencing parameters by consuming genomic sequencing cycles subject to optical defects of a camera, low-nucleobase-type diversity of a genomic sample, difficult-to-sequence genomic regions, or fluidic defects, the calibration-sequencing system 106 can avoid these technical obstacles to determine the same sequencing parameters during calibration cycles with a set of well-diversified calibration sequences according to the first calibration scheme.

In addition to improving sequencing parameters, by running calibration cycles using a set of well-diversified calibration sequences according to the first calibration scheme, the calibration-sequencing system 106 can also improve processes for determining which wells of a nucleotide-sample slide are not occupied with a cluster of oligonucleotides, such as the non-occupied well 514, or determining that nucleobase calls for a nucleotide-fragment read corresponding to a cluster passes a quality filter. By improving the sequencing parameters that provide standards for nucleobase calls, such as gaussian covariance matrices or intensity-value boundaries for particular nucleobases, the calibration-sequencing system 106 improves an accuracy and reliability of base-call-quality metrics and, consequently, determining which nucleobase calls pass quality filters.

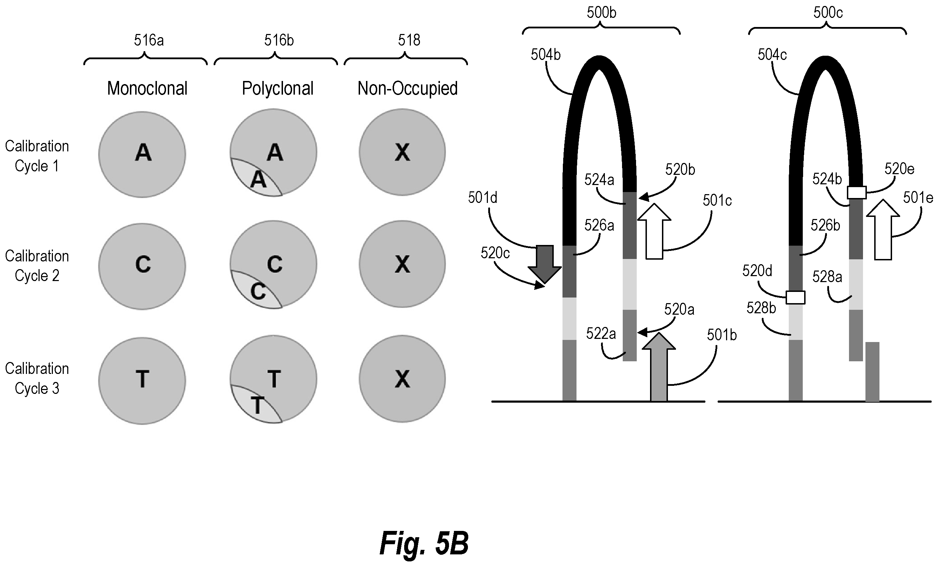

Turning now to FIG. 5B, under a second calibration scheme, the calibration-sequencing system 106 receives a nucleotide-sample slide comprising a set of cross-cycle-diversified calibration sequences having different nucleobase types across calibration cycles. In some cases, the calibration-sequencing system 106 uses a set of cross-cycle-diversified calibration sequences in which each calibration sequence follows a monotemplate calibration sequence. As an example of a monotemplate calibration sequence, in some case, each calibration sequence within such a set comprises a single sequence of nucleobases following a same order and having four nucleobase types within the single sequence of nucleobases. Such a monotemplate calibration sequence may be relatively short, such as spanning two, four, six, or eight nucleobases.

To use a set of cross-cycle-diversified calibration sequences, in certain cases, the calibration-sequencing system 106 uses a set of calibration sequences that (i) collectively include, at a first calibration-sequence position, a proportional distribution of a first nucleobase type and a second nucleobase type (e.g., G and C), (ii) collectively include, at a second calibration-sequence position, a proportional distribution of the first nucleobase type and a third nucleobase type (e.g., G and T), and (iii) collectively include, at a third calibration-sequence position, a proportional distribution of the first nucleobase type and a fourth nucleobase type (e.g., G and A).

As shown in Table 2 below, for instance, the calibration-sequencing system 106 can perform calibration cycles using a set of cross-cycle-diversified calibration sequences by following a monotemplate calibration sequence:

**Table 2**

| | Calibr. Cycle 1 | Calibr. Cycle 2 | Calibr. Cycle 3 | Calibr. Cycle 4 |
|---|---|---|---|---|
| Monotemplate Calibr. Sequence | T | G | A | C |

Additionally, or alternatively, as shown in Table 3 below, the calibration-sequencing system 106 can perform calibration cycles using a set of cross-cycle-diversified calibration sequences having a proportional distribution of a combination of different nucleobase types at different calibration-sequence positions corresponding to different calibration cycles. For instance, the set of cross-cycle-diversified calibration sequences can follow a 2-plex-template-calibration-sequence set as show in Table 3:

**Table 3**

| | Calibr. Cycle 1 | Calibr. Cycle 2 | Calibr. Cycle 3 |
|---|---|---|---|
| 2-Plex-Template-Calibr.-Sequence Set | G/C (Approx. 50%/50%) | G/T (Approx. 50%/50%) | G/A (Approx. 50%/50%) |

As depicted in FIG. 5B, in some cases, the calibration-sequencing system 106 detects or receives a nucleotide-sample slide comprising a given calibration sequence, from a set of cross-cycle-diversified calibration sequences, that has been integrated within a sample library fragment 500b at locations 520a, 520b, or 520c or within a sample library fragment 500c at locations 520d or 520e. When integrated at location 520a of the sample library fragment 500b, for instance, a calibration sequence is integrated as part or all of a binding adapter sequence 522a. When integrated at location 520b of the sample library fragment 500b, a calibration sequence is integrated as part or all of a first read priming sequence 524a. By contrast, when integrated at location 520c of the sample library fragment 500b, a calibration sequence is integrated as part or all of a second read priming sequence 526a. As suggested above, a sample genomic sequence 504b does not include a calibration sequence.

At locations 520a, 520b, or 520c for a calibration sequence, the calibration-sequencing system 106 can include calibration sequences as part of binding adapter sequences, indexing sequences, or read priming sequences in a sample library fragment from a nucleotide-sample-slide-preparation kit. In some such cases, the calibration sequences are common to all seeded sample library fragments because commonality already exists in the sequences of such sample library fragments (e.g., SBS sequences). When integrated at one of locations 520a, 520b, or 520c, in some embodiments, the calibration-sequencing system 106 shortens a corresponding sequencing primer (e.g., by shortening a sequencing primer of approximately 30 base pairs by one or more nucleobases) such that the nucleobases incorporated by the calibration-sequencing system 106 after the corresponding shortened sequencing primer correspond to the calibration sequence. In some such cases, the shortened sequencing primer exposes the calibration sequence for sequencing by an enzyme. By integrating calibration sequences as part of binding adapter sequences, indexing sequences, or read priming sequences-and shortening a corresponding sequencing primer-the calibration-sequencing system 106 can make calibration sequences compatible with components of a sample-library-fragment-preparation kit while changing sequencing primers from a nucleotide-sample-slide-preparation kit.

As depicted in FIG. 5B, for instance, the calibration-sequencing system 106 can use a shortened sequencing primer to facilitate sequencing a calibration sequence integrated at locations 520a, 520b, or 520c. As an example, in some cases, a calibration sequence is integrated within a downstream portion of the binding adapter sequence 522a (at location 520a) such that nucleobases that the calibration-sequencing system 106 incorporates after the sequencing primer 501b compliment the calibration sequence. As a further example, in some embodiments, a calibration sequence is integrated within a downstream portion of the first read priming sequence 524a (at location 520b) such that nucleobases that the calibration-sequencing system 106 incorporates after the sequencing primer 501c compliment the calibration sequence. As yet another example, in some implementations, a calibration sequence is integrated within a downstream portion of the second read priming sequence 526a (at location 520c) such that nucleobases that the calibration-sequencing system 106 incorporates after the sequencing primer 501d compliment the calibration sequence.

By integrating a monotemplate calibration sequence or a calibration sequence from a 2-plex-template-calibration-sequence set as part of a read priming sequence or a binding adapter sequence, the calibration-sequencing system 106 can use the same calibration sequences at the beginning of a nucleotide-fragment read for every cluster-without needing to consume nucleobase permutations or combinations of indexing sequences. By so using part of a read priming sequence or a binding adapter sequence as a calibration sequence, the calibration-sequencing system 106 may more accurately determine sequencing parameters-because such a primer-or-adapter approach removes the variation source (sample library fragment to read priming sequence hybridization efficiency) that could change with each different hybridization of a different sample library fragment and different read priming sequence.

In contrast to locations within the sample library fragment 500b, as further shown in FIG. 5B, when integrated at location 520d of the sample library fragment 500c, a calibration sequence is located between a second read priming sequence 526b and an indexing sequence 528b. Alternatively, a calibration sequence could be integrated as part or all of an indexing sequence 528a or the indexing sequence 528b. When integrated at location 520e of the sample library fragment 500c, a calibration sequence is located between the first read priming sequence 524b and a sample genomic sequence 504c. As indicated by FIG. 5B, in some embodiments, a sequencing primer 501e binds to the first read priming sequence 524b positioned upstream from the calibration sequence at location 520e. During a sequencing run, an enzyme adds or incorporates nucleobase by nucleobase to the sequencing primer 501e to generate a nucleotide-fragment read that is complimentary to a corresponding nucleotide sequence from the sample library fragment 500c-including nucleobases complimentary to the calibration sequence at location 520e.

When a calibration sequence is integrated within the sample library fragment 500c at locations 520d or 520e, in some cases, the calibration-sequencing system 106 changes a sample-library-fragment-preparation kit to include calibration sequences for placement between a read priming sequence and an indexing sequence or between a read priming sequence and a sample genomic sequence. With calibration sequences placed in such locations and part of a modified sample-library-fragment-preparation kit, however, the calibration-sequencing system 106 does not need to change sequencing primers from a nucleotide-sample-slide-preparation kit.

When a monotemplate calibration sequence or a calibration sequence from a 2-plex-template-calibration-sequence set is integrated as part or all of the indexing sequence 528a or the indexing sequence 528b, in some embodiments, the calibration-sequencing system 106 can incur approximately a same cost in reducing available nucleobases for the sample genomic sequence 504c for labeling as typically consumed when using indexing sequences. Because some existing double-stranded indexing sequences that span 8, 10, or 20 nucleobases have approximately 4¹⁶, 4²⁰, or 4⁴⁰ unique combinations, the calibration-sequencing system 106 has sufficient permutation space in nucleobases to use part of indexing sequences as calibration sequences. Because indexing primer sequence and a read priming sequence usually use separate biochemical hybridization events, however, a calibration cycle of the calibration-sequencing system 106 that uses part of an indexing sequence as a calibration sequence may detect additional (but likely negligible) noise when determining sequencing parameters based on such calibration sequences in clusters of oligonucleotides.

As further shown in FIG. 5B, a given calibration sequence from a set of cross-cycle-diversified calibration sequences having different nucleobase types can be (i) integrated within a sample library fragment deposited in a monoclonal cluster of oligonucleotides 516a (e.g., within a well of a nucleotide-sample slide) or (ii) integrated within different sample library fragments deposited in a polyclonal cluster of oligonucleotides 516b (e.g., within a well of a nucleotide-sample slide). For comparison, FIG. 5B also depicts a non-occupied well 518 that lacks a calibration sequence or a sample library fragment and can be used to determine certain sequencing parameters, such as in-well background intensity values. Consistent with the description above of a monotemplate calibration sequence, the calibration-sequencing system 106 incorporates nucleobases of a first nucleobase type (e.g., A) into the monoclonal cluster of oligonucleotides 516a during a first calibration cycle; incorporates nucleobases of a second nucleobase type (e.g., C) into the monoclonal cluster of oligonucleotides 516a during a second calibration cycle; and incorporates nucleobases of a third nucleobase type (e.g., T) into the monoclonal cluster of oligonucleotides 512a during a third calibration cycle. The incorporated nucleobases from the example in Table 3 would have more potential nucleobase-type diversity.

When a given calibration sequence from a set of cross-cycle-diversified calibration sequences having different nucleobase types is integrated within the polyclonal cluster of oligonucleotides 516b, as above, the given calibration sequence can be part of select known locations within a well. Across calibration cycles that cover the polyclonal cluster of oligonucleotides 516b, the calibration-sequencing system 106 incorporates nucleobases of the first nucleobase type (e.g., A) into the polyclonal cluster of oligonucleotides 516b during the first calibration cycle; incorporates nucleobases of the second nucleobase type (e.g., C) into the polyclonal cluster of oligonucleotides 516b during the second calibration cycle; and incorporates nucleobases of the third nucleobase types (e.g., T) into the polyclonal cluster of oligonucleotides 516b during the third calibration cycle.

When using a monotemplate calibration sequence, the common calibration sequence could be read by using an existing nucleotide sequence in a binding adapter sequence (as endogenous code) or by introducing a new nucleotide sequence into the binding adapter sequence (as exogenous code). To use endogenous code from the binding adapter sequence, the calibration-sequencing system 106 can shorten read priming sequences by several nucleobases (e.g., locations 520a, 520b, and 520c in FIG. 5B) and read nucleobases within the native binding adapter sequence. Such a native binding adapter sequence would be fully back compatible with all sample-library-fragment-preparation kits, but require customize read priming sequences. Alternatively, the calibration-sequencing system 106 can introduce a calibration sequence as exogenous code upstream (e.g., location 520d in FIG. 5B), downstream within an indexing sequence, or just before the genomic sample sequence (e.g., location 520e in FIG. 5B). In any such location, the calibration-sequencing system 106 can exclude the calibration cycles from a sequencing run either by changing the read priming sequences or by using dark calibration cycles that do not capture an image of incorporated nucleobases.

By using a monotemplate calibration sequence according to the second calibration scheme, the calibration-sequencing system can exploit an a priori known nucleotide sequence to determine sequencing parameters that compliment one or more sequencing parameters determined using a set of well-diversified calibration sequences with examples above with respect to FIG. 5A. For example, by using a monotemplate calibration sequence, the calibration-sequencing system 106 can determine more accurate nucleobase-specific-background intensity values. Because an incorporated nucleobase for each calibration cycle is known, the calibration-sequencing system 106 estimates background intensity values for each nucleobase type corresponding to the monotemplate calibration sequence. The calibration-sequencing system 106 can use such nucleobase-specific-background intensity values to provide feedback to improve or optimize surface chemistry or develop fully functional nucleotide (ffN) nucleotide location parameters to improve chemistry and signal-to-noise-ratio metrics.

As a further example, by using a set of cross-cycle-diversified calibration sequences according to the second calibration scheme, such as a monotemplate calibration sequence, the calibration-sequencing system 106 can determine more accurate intensity normalization coefficients. To determine such intensity normalization coefficients, the calibration-sequencing system 106 can estimate a maximum intensity and a minimum intensity of each nucleobase type in calibration cycles and determine intensity normalization coefficients based on the maximum and minimum intensities.

As yet another example, by using a set of cross-cycle-diversified calibration sequences according to the second calibration scheme, such as a monotemplate calibration sequence, the calibration-sequencing system 106 can determine more accurate gaussian covariance matrices and gaussian mean parameters for a Gaussian Mixture Model (GMM). By incorporating only a single nucleobase type for a monotemplate calibration sequence, the calibration-sequencing system 106 simplifies the estimation of gaussian covariance matrices and gaussian mean parameters without needing to fit a GMM or perform Expectation Maximization (EM). The calibration-sequencing system 106 can use such a simplified calculation as seeds for low-nucleobase-type-diversity sequencing cycles or as initial estimates for the actual GMM parameter in later genomic sequencing cycles to increase robustness and accuracy of adjusting sequencing parameters or determining nucleobase calls.

Further, by using a set of cross-cycle-diversified calibration sequences according to the second calibration scheme, such as a monotemplate calibration sequence, the calibration-sequencing system 106 can also determine more accurate per-cluster intensity normalization parameters for a particular channel. Because the calibration-sequencing system 106 can detect parameters for each cluster in each base position exactly once, the calibration-sequencing system 106 can perform the same by-cluster calibration as the first calibration scheme with small modifications of an estimation algorithm for certain sequencing parameters.

In addition to the improved sequencing parameters above, in some cases, by using a set of cross-cycle-diversified calibration sequences according to the second calibration scheme, such as a monotemplate calibration sequence, the calibration-sequencing system 106 can also estimate sequencing parameters that facilitate sequencing device chemistry and assays. For instance, the calibration-sequencing system 106 can estimate a sequencing-device-cluster-amplification-efficiency parameter and a sequencing-device-cluster-hybridization-efficiency parameter by determining a tightness or value difference of a single-color distribution (e.g., for a channel). As a further example, the calibration-sequencing system 106 can determine a color-channel-to-color-channel crosstalk parameter that could help facilitate improving sensor or optics design for a sequencing device.

As yet another example, by using a set of cross-cycle-diversified calibration sequences according to the second calibration scheme, the calibration-sequencing system 106 can determine a polycolonality of each cluster of oligonucleotides when the cluster is seeded by multiple sample library fragments. Because the calibration cycles for a monotemplate calibration sequence incorporate the same nucleobases across different sample library fragments, in some embodiments, the calibration-sequencing system 106 can identify a set of monoclonal inputs from the calibration cycles and subsequently identify polyclonal inputs from genomic sequencing cycles-thereby identifying input signals that, when compared, result in a measure of polycolonality on a per-cluster basis.

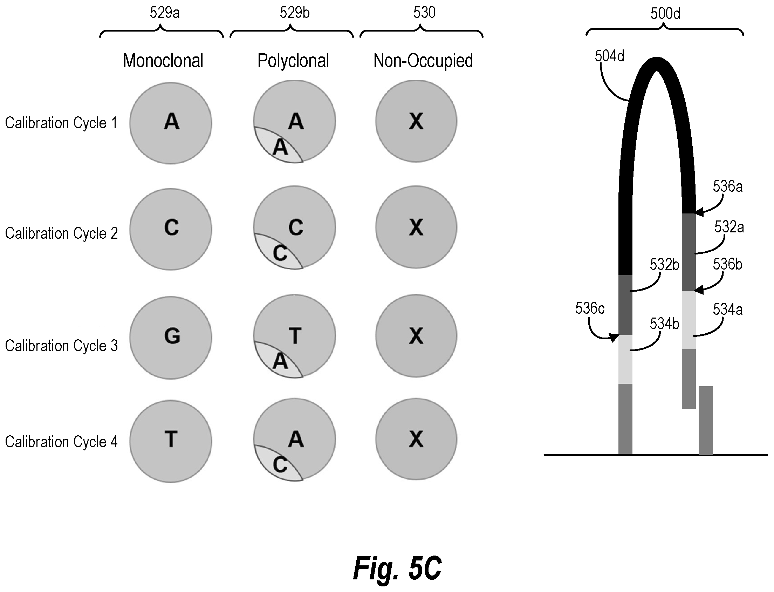

Turning now to FIG. 5C, under a third calibration scheme, the calibration-sequencing system 106 that hybridizes or combines the first calibration scheme and the second calibration scheme. To hybridize or combine such calibration schemes, the calibration-sequencing system 106 performs calibration cycles using a set of calibration sequences having a hybrid-diversified set of nucleobase types across calibration-sequence positions and across calibration cycles. By hybridizing the first and second calibration schemes, the calibration-sequencing system 106 can perform calibration cycles using calibration sequences (or calibration subsequences) following a monotemplate calibration sequence or a 2-plex-template-calibration-sequence set to determine a first set of sequencing parameters and other calibration sequences (or calibration subsequences) following a 4-plex-template-calibration-sequence set to determine a second set of sequencing parameters. Such a set of hybrid-diversified calibration sequences can accordingly determine sequencing parameters (e.g., non-linear optical distortion parameters, SIM parameters) better calibrated using the first calibration scheme and other sequencing parameters (e.g., nucleobase-specific-background intensity values, intensity normalization coefficients) better calibrated using the second calibration scheme in a set of calibration cycles.

For example, a set of hybrid-diversified calibration sequences may include (i) a first calibration sequence (or subsequence) following a monotemplate calibration sequence and (ii) a second calibration sequence (or subsequence) following a 4-plex-template-calibration-sequence set (e.g., approximately 25% of four different nucleobase types at a given calibration-sequence position. As described above and depicted in FIG. 4, for instance, the calibration-sequencing system 106 performs calibration cycles using a set of hybrid-diversified calibration sequences by (i) using a set of calibration sequences that follow the monotemplate nucleotide sequence 408 from the calibration sequence 402 to perform calibration cycles 1-4 and (ii) using the calibration sequence set 409b with a 4-plex-template-calibration-sequence set to perform calibration cycles 4-8.

As further depicted in FIG. 5C, in some cases, the calibration-sequencing system 106 detects or receives a nucleotide-sample slide comprising a given calibration sequence, from a set of hybrid-diversified calibration sequences, that has been integrated as part or all of a non-genomic sequence and/or non-transcriptomic sequence within a sample library fragment 500d. When integrated within the sample library fragment 500d according to the third calibration scheme, in some cases, a calibration sequence from a set of hybrid-diversified calibration sequences is integrated as part or all of a read priming sequence 532a (e.g., a first read priming sequence) or a read priming sequence 532b (e.g., a second read priming sequence). In certain cases, when integrated as part of the read priming sequences 532a or 532b, the calibration-sequencing system 106 shortens the relevant read priming sequence by a number of nucleobases, retains existing or common nucleobases providing a site for an enzyme (e.g., DNA polymerase), and adds nucleobases consistent with the first calibration scheme (e.g., a 4-plex-template-calibration-sequence set).

As further indicated by FIG. 5C for the third calibration scheme, in the alternative to the locations above, some or all of a calibration sequence from a set of hybrid-diversified calibration sequences can be integrated at location 536a in between the read priming sequence 532a and a sample genomic sequence 504d, at location 536b in between the read priming sequence 532a and an indexing sequence 534a, or at location 536c in between the read priming sequence 532b and an indexing sequence 534b.

When performing calibration cycles using a set of hybrid-diversified calibration sequences, in some cases, a nucleotide-sample slide comprises monoclonal clusters of oligonucleotides that incorporate nucleobases complimentary to a subset of hybrid-diversified calibration sequences (e.g., a subset of monotemplate calibration sequences) and polyclonal clusters of oligonucleotides that incorporate nucleobases complimentary to another subset of hybrid-diversified calibration sequences (e.g., a subset of 2- or 4-plex-template-calibration-sequence sets).

As indicated by FIG. 5C, for instance, a monoclonal cluster of oligonucleotides 529a includes a subset of monotemplate calibration sequences. In calibration cycles 1-4 for the monoclonal cluster of oligonucleotides 529a, the calibration-sequencing system 106 incorporates nucleobases of a first nucleobase type (e.g., A) into the monoclonal cluster of oligonucleotides 529a during a first calibration cycle; incorporates nucleobases of a second nucleobase type (e.g., C) into the monoclonal cluster of oligonucleotides 529a during a second calibration cycle; incorporates nucleobases of a third nucleobase type (e.g., G) into the monoclonal cluster of oligonucleotides 529a during a third calibration cycle; and incorporates nucleobases of a fourth nucleobase type (e.g., T) into the monoclonal cluster of oligonucleotides 529a during a fourth calibration cycle. For comparison, FIG. 5C also depicts a non-occupied well 530 that lacks a calibration sequence or a sample library fragment and can be used to determine certain sequencing parameters, such as in-well background intensity values.

As further indicated by FIG. 5C, a polyclonal cluster of oligonucleotides 529b includes a set of calibration sequences with a subset of 2-plex or 4-plex-template-calibration-sequence sets. In calibration cycles 1-4 for the polyclonal cluster of oligonucleotides 529b, the calibration-sequencing system 106 incorporates nucleobases of the first nucleobase type (e.g., A) into the polyclonal cluster of oligonucleotides 529b during the first calibration cycle; incorporates nucleobases of the second nucleobase type (e.g., C) into the polyclonal cluster of oligonucleotides 529b during the second calibration cycle; incorporates nucleobases of the fourth and first nucleobase types (e.g., T and A) into the polyclonal cluster of oligonucleotides 529b during the third calibration cycle; and incorporates nucleobases of the first and third nucleobase types (e.g., A and c) into the polyclonal cluster of oligonucleotides 529b during the fourth calibration cycle.

By performing calibration cycles with a set of hybrid-diversified calibration sequences under the third calibration scheme-which hybridizes the first and second calibration schemes-the calibration-sequencing system 106 lowers the memory that would have been consumed for running the second calibration scheme exclusively. In particular, under the third and hybridized calibration scheme, the calibration-sequencing system 106 no longer requires multiple calibration cycles to evaluate intensity values before estimating related sequencing parameters, such as nucleobase centroids for intensity values or gaussian covariance matrices. Such a set of hybrid-diversified calibration sequences under the third calibration scheme that combines a monotemplate calibration sequence with a well-diversified calibration sequence can be particularly efficient on a high-throughput sequence device. By performing calibration cycles exhibiting both well-diversified calibration sequences and cross-cycle-diversified calibration sequences, the calibration-sequencing system 106 likewise increases an accuracy and robustness of determining sequencing parameters.

In addition or in the alternative to a third and hybridized calibration scheme, in some embodiments, the calibration-sequencing system 106 operates under a fourth calibration scheme using a selected subset of clusters or wells within a nucleotide-sample slide. In this fourth calibration scheme, the calibration-sequencing system 106 implements one or more of the first calibration scheme, the second calibration scheme, or the third calibration scheme in selected clusters of oligonucleotides (or corresponding selected wells) within a nucleotide-sample slide. Indeed, in some cases, the calibration-sequencing system receives or detects a nucleotide-sample slide comprising calibration sequences following one or more of the first, second, or third calibration schemes (i) in each well of the nucleotide-sample slide for seeding each cluster of oligonucleotides within each well, (ii) in a subset of wells of the nucleotide-sample slide for seeding each cluster of oligonucleotides within the subset of wells, or (iii) in the subset of wells of the nucleotide-sample slide for seeding a subset of clusters of oligonucleotides within the subset of wells. Similarly, for un-patterned nucleotide-sample slides, the calibration-sequencing system receives or detects a nucleotide-sample slide comprising calibration sequences following one or more of the first, second, or third calibration schemes in each cluster of oligonucleotides or in a subset of clusters of oligonucleotides.

By selecting a subset of wells or a subset of clusters to integrate different types of calibration sequences within different sets of sample library fragments, the calibration-sequencing system 106 can determine different sequencing parameters corresponding to different types of calibration sequences. Additionally, by selecting a first subset of wells or a first subset of clusters to integrate calibration sequences-and omitting calibration sequences from a second subset of wells or a second subset of clusters-the calibration-sequencing system 106 can optionally perform calibration cycles for the first subset of clusters/wells while performing other sequencing cycles (e.g., indexing cycles) for the second subset of clusters-wells.

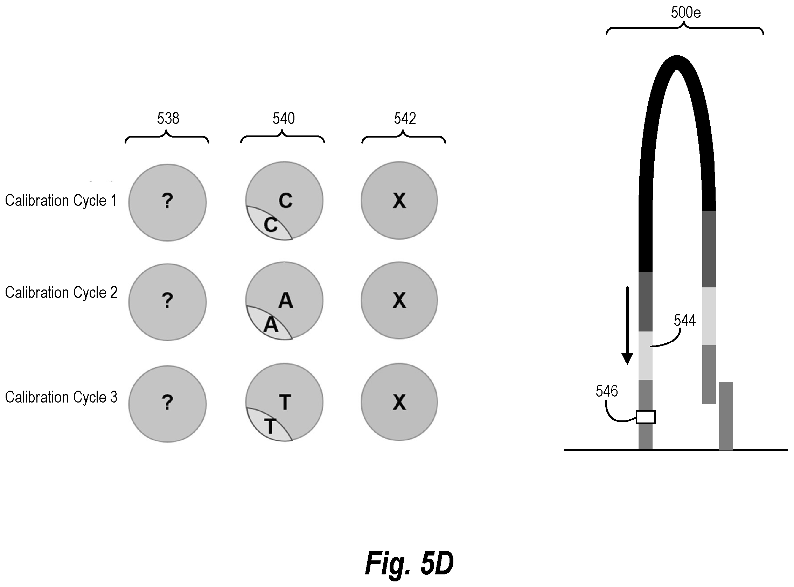

In accordance with one or more embodiments, FIG. 5D depicts an example of the calibration-sequencing system 106 running calibration cycles for one subset of wells or subset of clusters while running other sequencing cycles for another subset of wells or subset of clusters. As shown in FIG. 5D, for instance, the calibration-sequencing system 106 performs sequencing cycles, such as indexing cycles, for a first subset of wells 538 within a nucleotide-sample slide comprising sample library fragments that lack calibration sequences. In some cases, the first subset of wells 538 constitutes 85% or more of the wells within the nucleotide-sample slide. As further shown in FIG. 5D, the calibration-sequencing system 106 performs calibration cycles for a second subset of wells 540 within the nucleotide-sample slide comprising calibration sequences within sample library fragments. In some cases, the second subset of wells 540 constitutes 5% or less of the wells within the nucleotide-sample slide. As further shown by FIG. 5D, the nucleotide-sample slide includes a third subset of wells 542 without sample library fragments or calibration sequences to determine certain sequencing parameters, such as in-well background intensity values.

Consistent with the first, second, or third calibration schemes above, the calibration sequences in the second subset of wells 540 may be in different locations within sample library fragments. Under the fourth calibration scheme, FIG. 5D illustrates but one example of a location for a calibration sequence integrated within a sample library fragment within one well of the second subset of wells 540. When integrated within a sample library fragment 500e, for instance, a calibration sequence 546 is integrated as part or all of a binding adapter sequence 544. In some cases, when integrated as part of the binding adapter sequence 544, the calibration-sequencing system 106 uses some or all of a UDI sequence as the calibration sequence 546.

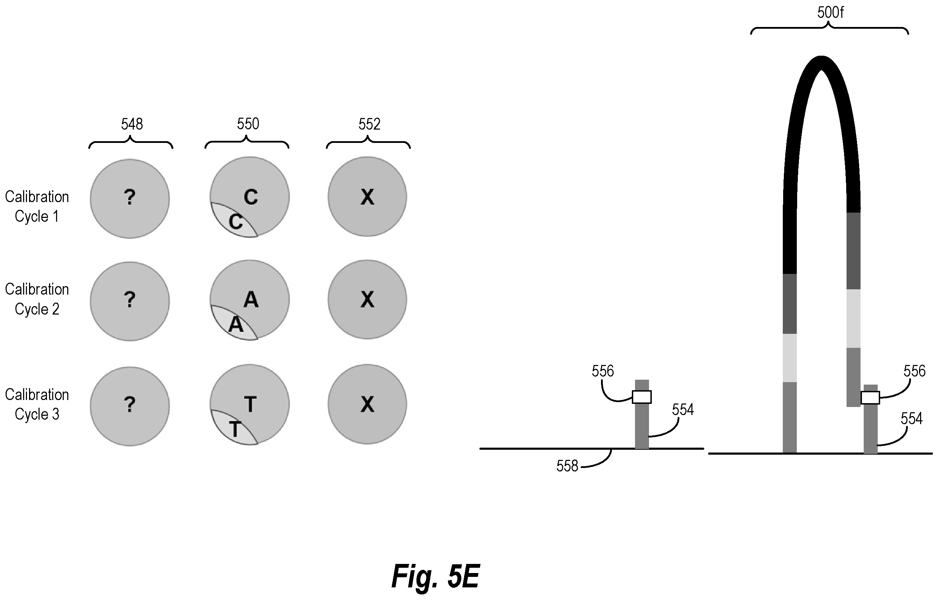

As suggested above, in some cases, the calibration-sequencing system 106 uses calibration sequences associated with sample library fragments comprising a first read priming sequence and a second read priming sequence that respectively initiate polymerases sequencing a first nucleotide-fragment read followed by a second nucleotide-fragment read. In accordance with one or more embodiments, FIG. 5E depicts calibration cycles and a location for a calibration sequence for concurrent sequencing of a first nucleotide-fragment read and a second nucleotide-fragment read. By adding or integrating a calibration sequence as part of one or more surface-bound oligonucleotides that are attached to a surface of the nucleotide-sample slide, in some embodiments, the calibration-sequencing system 106 can perform calibration cycles that avoid cross-talk signals from concurrently sequenced nucleotide-fragment reads from a sample library fragment.

As shown in FIG. 5E, for instance, a calibration sequence 556 is integrated as part or all of a surface-bound oligonucleotide 554 attached to a surface 558 of the nucleotide-sample slide. For instance, in some cases, the calibration sequence 556 is part or all of the surface-bound oligonucleotide 554 attached to a polymer coating for a nucleotide-sample slide's lawn, such as poly(N-(5-azidoacetamidylpentyl)acrylamide-co-acrylamide (PAZAM). By integrating the calibration sequence 556 as part of an oligonucleotide attached to PAZAM for a nucleotide-sample slide or attached to another surface-and performing a calibration cycle before a sample library fragment 500f anneals or bonds with the surface-bound oligonucleotide 554-the calibration-sequencing system 106 can perform calibration cycles that avoid cross-talk signals from concurrently sequenced nucleotide-fragment reads, such as SPEAR basecalling from Illumina, Inc., or before cleaving of a read priming sequence during concurrent sequencing. In some cases, such concurrent sequencing of nucleotide-fragment reads or cleaving of read priming sequences is described by Flow Cells, U.S. Patent Application No. 16/626,452 (filed Jun. 7, 2019) or by Flow Cells, U.S. Patent Application No. 17/126,548 (filed Dec. 18, 2020), which are hereby incorporated by reference in their entirety.

In the alternative to the surface-bound oligonucleotide 554, the calibration sequence 556 can be integrated into a location of the sample library fragment 500f. Although not shown in FIG. 5E, in some embodiments, the calibration sequence 556 is located in between a first read priming sequence and a sample genomic sequence of the sample library fragment 500f or in between a second read priming sequence and the sample genomic sequence of the sample library fragment 500f. In yet other embodiments, the calibration sequence 556 is integrated as part or all of a first read priming sequence or a second read priming sequence of the sample library fragment 500f.

When performing calibration cycles with calibration sequences in a sequencing run that concurrently sequences first and second nucleotide-fragment reads, in some embodiments, the calibration-sequencing system 106 uses the second calibration scheme from FIG. 5B, such as a monotemplate calibration sequence, with each well comprising a same type of calibration sequence in a given calibration cycle. Alternatively, in some embodiments, the calibration-sequencing system 106 uses the first calibration scheme from FIG. 5A, such as a 4-plex-template-calibration-sequence set, with wells comprising different types of calibration sequences in a given calibration cycle-in anticipation of advancements in lawn-chemistry patterning (SPEAR-like patterning).

Further, when performing calibration cycles with calibration sequences in a sequencing run that concurrently sequences first and second nucleotide-fragment reads, in some embodiments, the calibration-sequencing system 106 detects or receives a nucleotide-sample slide comprising calibration sequences in each well of the nucleotide-sample slide or in a subset of wells of the nucleotide-sample slide. To determine sequencing parameters, such as background intensity values, the nucleotide-sample slide may include empty wells (e.g., not seedable) during calibration cycles of the calibration-sequencing system 106.

As further shown in FIG. 5E, the calibration-sequencing system 106 performs sequencing cycles, such as indexing cycles, for a first subset of wells 548 within a nucleotide-sample slide that may or may not comprise sample library fragments. As noted above, when a calibration sequence is integrated as part of a surface-bound oligonucleotide, the calibration-sequencing system 106 can perform calibration cycles before sample library fragments anneal or bond with surface-bound oligonucleotides or a nucleotide-sample slide's surface. As further shown in FIG. 5E, the calibration-sequencing system 106 performs calibration cycles for a second subset of wells 550 within the nucleotide-sample slide comprising calibration sequences integrated as part of surface-bound oligonucleotides that are attached to a surface of the nucleotide-sample slide. As further shown by FIG. 5E, the nucleotide-sample slide includes a third subset of wells 552 without sample library fragments or calibration sequences to determine certain sequencing parameters, such as in-well background intensity values.

As described above, FIGS. 5A-5E depict various calibration schemes. In addition to or in conjunction with the embodiments described above with respect to FIGS. 5A-5E, Table 4 below sets forth a few specific examples for each calibration scheme with example numbers of calibration cycles and example target sequencing parameters determined under calibration schemes reference in Table 4. While some of the specific examples of calibration schemes include approximate numbers of calibration cycles, any suitable number of calibration cycles may be implemented for a given calibration scheme.

**Table 4**

| **Calibration Scheme** | **Calibration Cycles** | **Example Target Sequencing Parameters** | **Reagent / Library Prep Compatibility** |
|---|---|---|---|
| First Calibration Scheme (FIG. 5A): using part or all of non-random UMI sequence as calibration sequence | Approximately 4-8 calibration cycles of well-diversified calibration sequence (e.g., 4-plex) | Non-linear optical distortion parameters; | Sample-library-fragment-preparation kits that support UMI sequences |
| | | Extraction parameters; | |
| | | Per-cluster normalization parameters, brightness parameters, or intensity parameters; | |
| | | Gaussian seed parameters; | |
| | | ffN specific nucleotide location parameters | |
| First Calibration Scheme (FIG. 5A): using part or all of | Approximately 4-6 calibration cycles of | Non-linear optical distortion parameters; | Indexing-first workflow / primer |
| | | Extraction parameters; | |
| indexing sequence as calibration sequence | well-diversified calibration sequence (e.g., 4-plex) | Per-cluster normalization parameters, brightness parameters, or intensity parameters; | |
| | | Gaussian seed parameters; | |
| | | ffN specific nucleotide location parameters | |
| Second Calibration Scheme (FIG. 5B): using part or all of indexing sequence as calibration sequence with (i) first indexing primer or (ii) post-process via indexing cycles first | Calibration cycles for length of mono-template calibration sequence or 2-plex | Intensity normalization coefficients; | Indexing-first workflow |
| | | Per-cluster normalization parameters, brightness parameters, or intensity parameters; | |
| | On indexing sequence read | Color-channel-to-color-channel cross-talk parameters; | |
| | | Polycolonality | |
| | | SNR parameter without Polycolonality | |
| | | Occupancy | |
| Second Calibration Scheme (FIG. 5B): using part or all of y-adapter as calibration sequence | Calibration cycles for length of mono-template calibration sequence or 2-plex | Intensity normalization coefficients; | Generally compatible with all sample-library-fragment-preparation kits, although could be complicated if a technician mixes genomic samples from different preps (e.g., some SBS3 and some MeA14 libraries). |
| | | Per-cluster normalization parameters, brightness parameters, or intensity parameters; | |
| | On genomic read prior to gDNA nucleobases | Color-channel-to-color-channel cross-talk parameters; | |
| | | Polycolonality | |
| | | SNR parameter without Polycolonality | |
| | | Occupancy | |
| Third Calibration Scheme (FIG. 5C) | A few (e.g., 4) calibration cycles of well-diversified | Sequencing parameters from first and second calibration schemes combined | Index-first workflows or UMI workflows |
| | calibration sequences and a few calibration cycles of mono-template calibration sequence | | |
| Fourth Calibration Scheme (FIG. 5D) | Either on indexing read (picking barcodes) | Sequencing parameters from the first calibration scheme on select clusters/wells | Generally compatible with all sample-library-fragment-preparation kits |
| | Or on genomic read (shortened SBS3 read priming sequence + UMI sequence) Selected subset of clusters/wells | | |
| | First calibration scheme without polycolonality | | |
| | Each selected cluster/well will have 25% nucleobase call distribution | | |

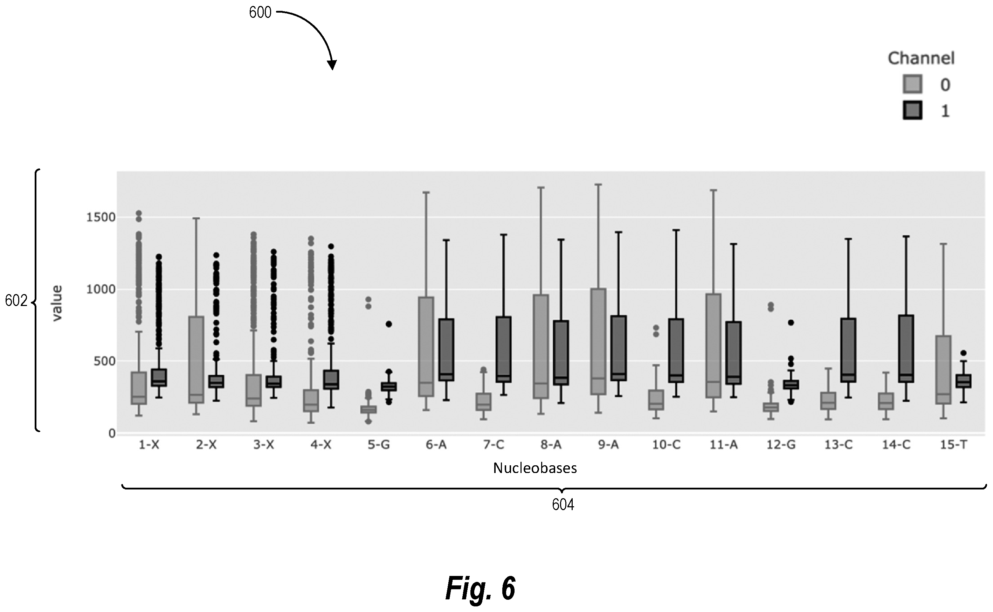

As noted above, in some embodiments, the calibration-sequencing system 106 improves the accuracy of one or more sequencing parameters and corresponding nucleobase calls by using calibration sequences to calibrate a sequencing device. In accordance with one or more embodiments, FIG. 6 illustrates a box-plot diagram 600 of intensity-value distributions for different channels corresponding to different nucleobase types based on either randomly called nucleobases or known nucleobases from calibration sequences. As indicated by the box-plot diagram 600, the calibration-sequencing system 106 can determine intensity parameters (e.g., per-cluster intensity parameters) for particular channels based on known nucleobases from calibration sequences better than unknown or randomly called nucleobases typical of conventional calibration.

As shown in FIG. 6, the box-plot diagram 600 shows intensity values for channel 0 and channel 1 along an intensity-value axis 602 and nucleobases called for cycles 1-15 along a nucleobase axis 604. To determine the intensity values, the calibration-sequencing system 106 performed sequencing cycles 1-4 to incorporate and determine intensity values for random nucleobases from non-calibration sequences, where an "X" indicates random nucleobases of different nucleobase types. The calibration-sequencing system 106 further performed calibration cycles 5-15 to incorporate and determine intensity values for known nucleobases from a set of calibration sequences following a monotemplate calibration sequence, where the letter next to the cycle number indicates the known nucleobase of a particular nucleobase type (e.g., guanine for "5-G" and adenine for "6-A"). Channel 0 represents light emitted by clusters for a particular fluorescent dye and an image capturing the light during a cycle. Channel 1 represents light emitted by clusters for a different fluorescent dye and a different image capturing the light during a cycle. The calibration-sequencing system 106 performed the cycles depicted in the box-plot diagram using an iSeqKepler with AZM dye and 65°C imaging.

As the box-plot diagram 600 illustrates, the calibration-sequencing system 106 determines clear and distinct ranges and means of intensity values for particular nucleobase types in different channels using a monotemplate calibration sequence. By contrast, the calibration-sequencing system 106 determines unclear and less helpful ranges or means of intensity values in different channels using random or unknown nucleobases.

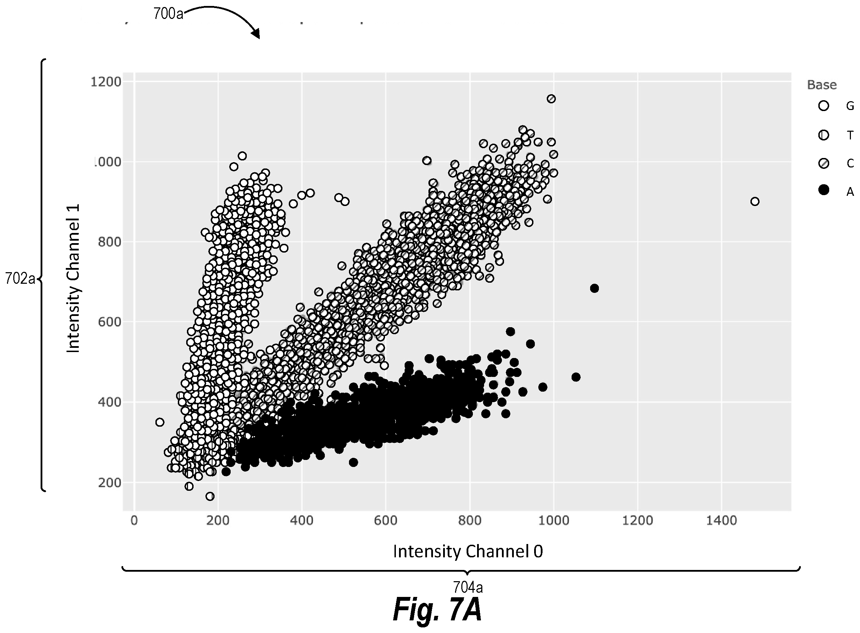

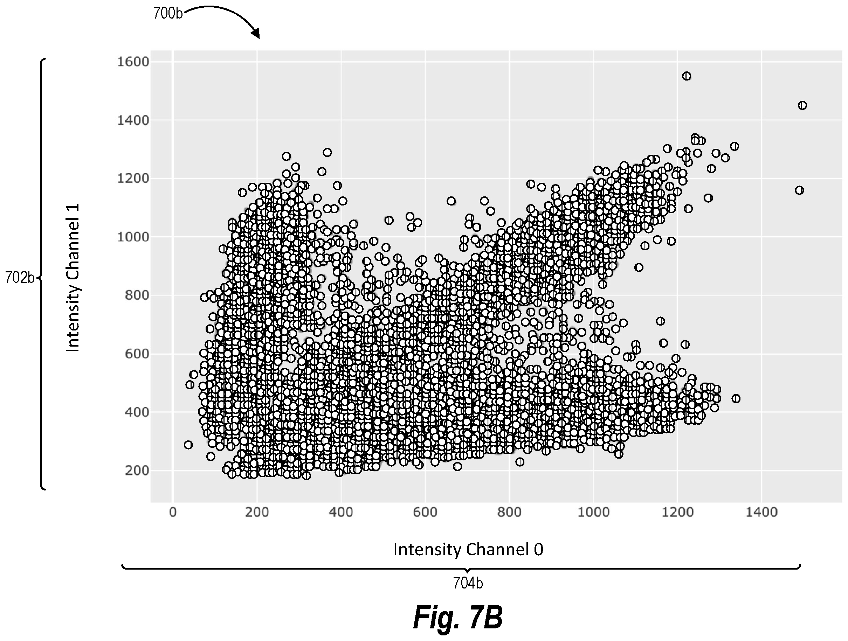

In addition to improving per-cluster intensity parameters, the calibration-sequencing system 106 can improve the accuracy of other sequencing parameters, such as intensity-value boundaries for particular nucleobase types, by using calibration sequences to calibrate a sequencing device. In accordance with one or more embodiments, FIGS. 7A-7B illustrate scatter-plot diagrams 700a and 700b of intensity values corresponding to different nucleobase types for different channels based on different types of calibration sequences. In particular, FIG. 7A illustrates a scatter-plot diagram 700a of intensity values corresponding to nucleobases of four different nucleobase types from a set of calibration sequences following a monotemplate calibration sequence under the second calibration scheme. FIG. 7B illustrates a scatter-plot diagram 700b of intensity values corresponding to nucleobases of four different nucleobase types from a set of calibration sequences following a 4-plex-template-calibration-sequence set under the first calibration scheme.

As shown in FIG. 7A, for instance, the scatter-plot diagram 700a shows intensity values for channel 1 along an intensity-value axis 702a and intensity values for channel 0 along an intensity-value axis 704a. To capture the intensity values shown in FIG. 7A, the calibration-sequencing system 106 performed 30 calibration cycles for a set of calibration sequences following a monotemplate calibration sequence. As noted above, such a monotemplate calibration sequence includes known nucleobases that facilitate determining sequencing parameters. Accordingly, the scatter-plot diagram 700a depicts intensity values for multiple clusters of oligonucleotides that passed a quality filter across 30 calibration cycles.

As shown in FIG. 7B, the scatter-plot diagram 700b shows intensity values for channel 1 along an intensity-value axis 702b and intensity values for channel 0 along an intensity-value axis 704b. To capture the intensity values shown in FIG. 7B, the calibration-sequencing system 106 performed a single calibration cycle for a set of calibration sequences following a 4-plex-template-calibration-sequence set. Accordingly, the scatter-plot diagram 700b depicts intensity values for multiple clusters of oligonucleotides that passed a quality filter for a single calibration cycle.

As shown by a comparison of FIG. 7A and FIG. 7B, the intensity-value clouds corresponding to four different nucleobase types (e.g., G, A, C, T) are consistent and correlate with each other in the scatter-plot diagrams 700a and 700b. In some embodiments, therefore, the calibration-sequencing system 106 can accurately determine intensity-value boundaries for particular nucleobase types using either a monotemplate calibration sequence or a 4-plex-template-calibration-sequence set. In some cases, the calibration-sequencing system 106 can also determine one or more sequencing parameters from calibration cycles using a monotemplate calibration sequence and then adjust (or use as starting points) the one or more sequencing parameters during calibration cycles using a 4-plex-template-calibration-sequence set.

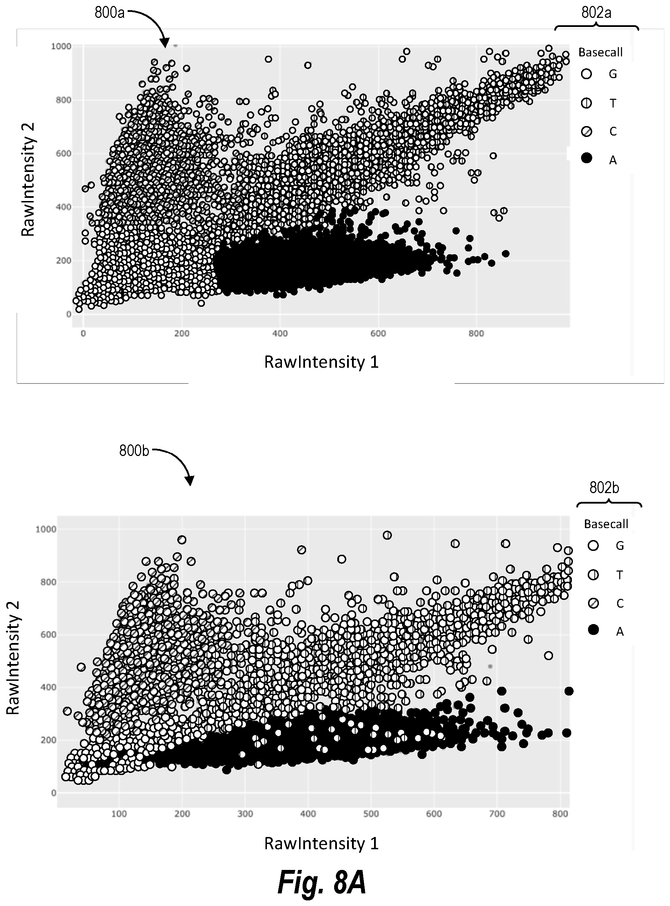

As further indicated above, in some embodiments, the calibration-sequencing system 106 improves nucleobase calls with improved sequencing parameters. In accordance with one or more embodiments, FIGS. 8A-8B illustrate scatter-plot diagrams of intensity values for nucleobases of different nucleobase types with corresponding nucleobase calls using non-calibrated sequencing parameters and calibrated sequencing parameters.

As shown in FIG. 8A, for instance, a scatter-plot diagram 800a depicts intensity values extracted from a set of images of light emitted by incorporated nucleobases of different nucleobase types (e.g., G, T, C, A) during a sequencing cycle (e.g., indexing cycle) using non-calibrated sequencing parameters and corresponding nucleobase calls for the incorporated nucleobases, as indicated by a color-coded key 802a for the nucleobase calls. In particular, for the scatter-plot diagram 800a, the calibration-sequencing system 106 determines intensity values and nucleobase calls using a normalization parameter set at 1 and offset correction parameter set at 0. By contrast, a scatter-plot diagram 800b depicts intensity values extracted from the same set of images using calibrated sequencing parameters, including calibrated per-cluster intensity normalization parameters, and corresponding nucleobase calls for the incorporated nucleobases, as indicated by a color-coded key 802b for the nucleobase calls. Such calibrated per-cluster intensity normalization parameters may include, for instance, a calibrated scaling factor, a calibrated offset correction parameter, a log normalization factor, or a sigmoidal transform. As shown by a comparison of the scatter-plot diagram 800a and the scatter-plot diagram 800b, the calibration-sequencing system 106 determines more accurate nucleobase calls using the calibrated sequencing parameters.

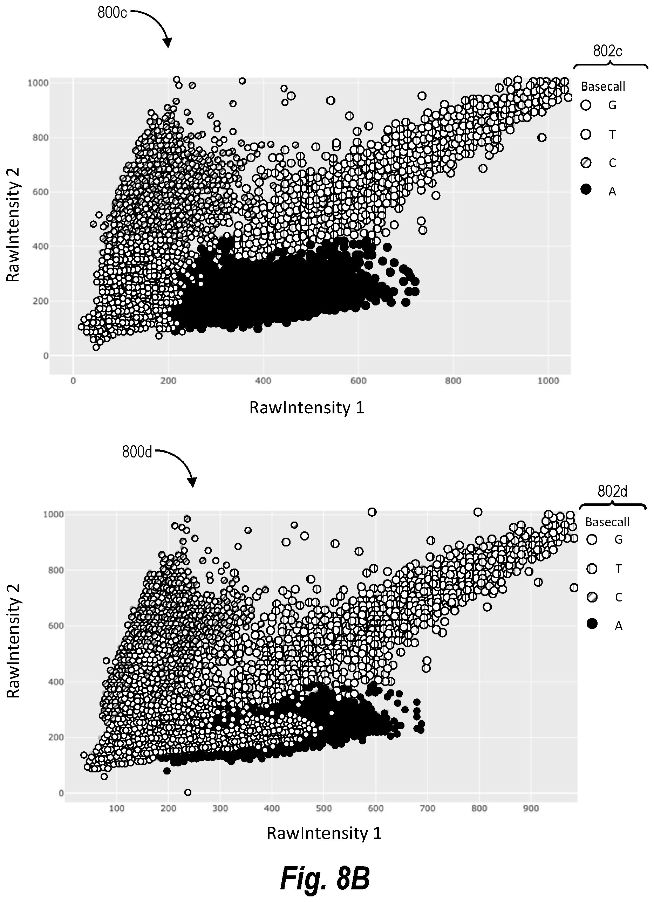

As shown in FIG. 8B, a scatter-plot diagram 800c depicts intensity values extracted from a set of images of light emitted by incorporated nucleobases of different nucleobase types (e.g., G, T, C, A) during a different sequencing cycle (e.g., indexing cycle) than that depicted in FIG. 8A using non-calibrated sequencing parameters and corresponding nucleobase calls for the incorporated nucleobases, as indicated by a color-coded key 802c for the nucleobase calls. In particular, for the scatter-plot diagram 800c of FIG. 8B, the calibration-sequencing system 106 determines intensity values and nucleobase calls using a scaling factor set at 1 and offset correction parameter set at 0. By contrast, a scatter-plot diagram 800d depicts intensity values extracted from the same set of images using calibrated sequencing parameters, including a calibrated scaling factor and a calibrated offset correction parameter, and corresponding nucleobase calls for the incorporated nucleobases, as indicated by a color-coded key 802d for the nucleobase calls. As shown by a comparison of the scatter-plot diagram 800c and the scatter-plot diagram 800d, the calibration-sequencing system 106 determines more accurate nucleobase calls using the calibrated sequencing parameters.

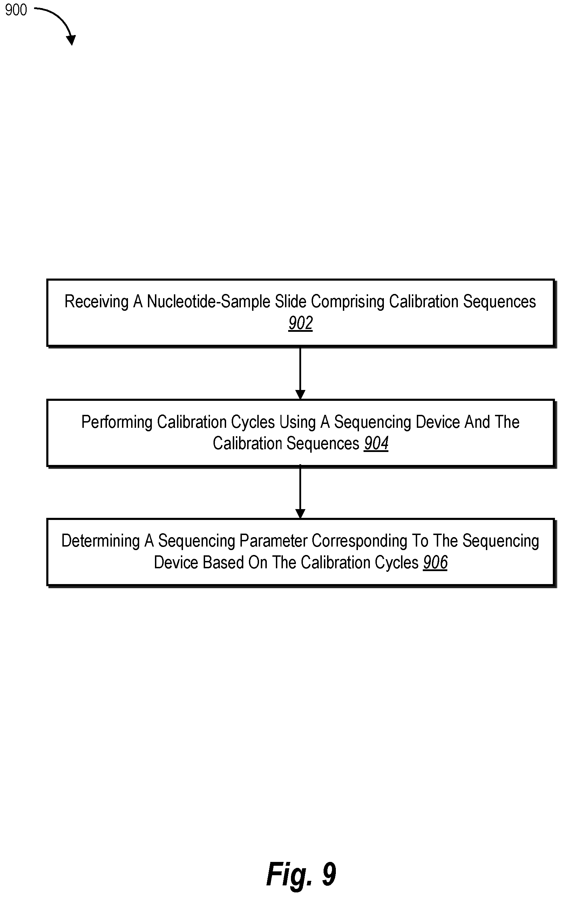

Turning now to FIG. 9, this figure illustrates a flowchart of a series of acts 900 of receiving nucleotide-sample slide comprising calibration sequences and determining one or more sequencing parameters corresponding to a sequencing device based on the calibration sequences in accordance with one or more embodiments of the present disclosure. While FIG. 9 illustrates acts according to one embodiment, alternative embodiments may omit, add to, reorder, and/or modify any of the acts shown in FIG. 9. The acts of FIG. 9 can be performed as part of a method. Alternatively, a non-transitory computer readable storage medium can comprise instructions that, when executed by one or more processors, cause a computing device or a system to perform the acts depicted in FIG. 9. In still further embodiments, a system comprising at least one processor and a non-transitory computer readable medium comprising instructions that, when executed by one or more processors, cause the system to perform the acts of FIG. 9.

As shown in FIG. 9, the acts 900 include an act 902 of receiving a nucleotide-sample slide comprising calibration sequences. In particular, in some embodiments, the act 902 includes receiving a nucleotide-sample slide comprising calibration sequences of nucleobases. Additionally, or alternatively, the act 902 includes receiving a nucleotide-sample slide comprising calibration sequences of nucleobases and sample library fragments associated with the calibration sequences. In some embodiments, the calibration sequences are part of sample library fragments deposited on the nucleotide-sample slide or part of surface-bound oligonucleotides that are attached to a surface of the nucleotide-sample slide.

In certain embodiments, the calibration sequences comprise different sequences of nucleobases having a proportional distribution of four nucleobase types. Relatedly, in certain implementations, the different sequences of nucleobases comprise a first calibration sequence including four nucleobase types according to a first order of nucleobases and a second calibration sequence including the four nucleobase types according to a second order of nucleobases.

Further, in some cases, the calibration sequences each comprise a single sequence of nucleobases following a same order of nucleobases and having four nucleobase types within the single sequence of nucleobases. Additionally, or alternatively, the calibration sequences each comprise a single sequence of nucleobases having a proportional distribution of four nucleobase types. In certain cases, the calibration sequences comprise different sequences of nucleobases that collectively include, at a given calibration-sequence position, a distribution of a first nucleobase type and a second nucleobase type and collectively include, at a subsequent calibration-sequence position, a distribution of the first nucleobase type and a third nucleobase type or a distribution of the third nucleobase type and a fourth nucleobase type.

As noted above, in some cases, receiving the nucleotide-sample slide comprises receiving the nucleotide-sample slide comprising: the calibration sequences in each well of the nucleotide-sample slide for seeding each cluster of oligonucleotides within each well; the calibration sequences in a subset of wells of the nucleotide-sample slide for seeding each cluster of oligonucleotides within the subset of wells; or the calibration sequences in the subset of wells of the nucleotide-sample slide for seeding a subset of clusters of oligonucleotides within the subset of wells.

As further indicated above, in some embodiments, receiving the nucleotide-sample slide comprises receiving the nucleotide-sample slide comprising: a first set of sample library fragments including a first set of calibration sequences for calibrating a first sequencing parameter; and a second set of sample library fragments including a second set of calibration sequences for calibrating a second sequencing parameter. Relatedly, in certain embodiments, receiving the nucleotide-sample slide comprises receiving the nucleotide-sample slide comprising a sample library fragment including a calibration sequence as part or all of a non-genomic and/or non-transcriptomic sequence within the sample library fragment.

Additionally, or alternatively, receiving the nucleotide-sample slide comprises receiving the nucleotide-sample slide comprising a sample library fragment including a calibration sequence between a binding adapter sequence and an indexing sequence, between the indexing sequence and a read priming sequence, or between the read priming sequence and a sample genomic sequence.

Relatedly, in some cases, the calibration sequence between the binding adapter sequence and the indexing sequence comprises the calibration sequence between a P7 binding adapter sequence and an i7 indexing sequence or between a P5 binding adapter sequence and an i5 indexing sequence; the calibration sequence between the indexing sequence and the read priming sequence comprises the calibration sequence between the i7 indexing sequence and a first read priming sequence or between the i5 indexing sequence and a second read priming sequence; and the calibration sequence between the read priming sequence and the sample genomic sequence comprises the calibration sequence between the first read priming sequence and the sample genomic sequence or between the second read priming sequence and the sample genomic sequence.

Additionally, or alternatively, receiving the nucleotide-sample slide comprises receiving the nucleotide-sample slide comprising a sample library fragment including a calibration sequence as part or all of a non-random unique molecular identifier (UMI) sequence, as part or all of a unique dual index (UDI) sequence, as part or all of an indexing sequence, or as part or all of a binding adapter sequence.

As further shown in FIG. 9, the acts 900 include an act 904 of performing calibration cycles using a sequencing device and the calibration sequences. In particular, in some embodiments, the act 904 includes performing one or more calibration cycles using a sequencing device to incorporate nucleobases into oligonucleotides corresponding to the calibration sequences. In some cases, performing the one or more calibration cycles comprises performing at least four calibration sequencing cycles.

As suggested above, in certain embodiments, performing the one or more calibration cycles comprises incorporating nucleobases of one nucleobase type or two nucleobase types into a calibration-sequence position of a set of growing oligonucleotides corresponding to a set of calibration sequences. Further, in some cases, performing the one or more calibration cycles comprises incorporating nucleobases having a proportional distribution of two nucleobase types, a proportional distribution of three nucleobase types, or a proportional distribution of four nucleobase types into a calibration-sequence position of a set of growing oligonucleotides corresponding to a set of calibration sequences.

As further suggested above, in one or more embodiments, performing the one or more calibration cycles comprises incorporating nucleobases into a set of growing oligonucleotides corresponding to a set of calibration sequences without determining nucleobase calls for the set of calibration sequences. Further, in certain implementations, performing the one or more calibration cycles comprises performing the one or more calibration cycles before or after performing genomic sequencing cycles using the sequencing device to determine nucleobase calls for sample genomic sequences within sample library fragments.

As further shown in FIG. 9, the acts 900 include an act 906 of determining a sequencing parameter corresponding to the sequencing device based on the calibration cycles. In particular, in certain implementations, the act 906 includes determining a sequencing parameter corresponding to the sequencing device based on the one or more calibration cycles and the calibration sequences. As suggested above, in some embodiments, determining the sequencing parameter corresponding to the sequencing device comprises detecting the sequencing parameter from the sequencing device during or after the one or more calibration cycles.

Further, in some cases, determining the initial sequencing parameters or the sequencing parameter comprises determining one or more of equalizer coefficients, convolutional kernel coefficients, nucleobase centroids for intensity values, intensity-value boundaries for particular nucleobase types, nucleobase-specific-background intensity values, intensity normalization coefficients, gaussian covariance matrices, gaussian mean parameters, gaussian seed parameters, fully functional nucleotide (fFN) specific nucleotide location parameters, non-linear optical distortion parameters, structured illumination microscopy (SIM) parameters, per-cluster normalization parameters, channel-specific normalization parameters, cluster-specific-signal normalization parameters, color-channel-to-color-channel crosstalk parameters, or polycolonality parameters.

In addition to the acts 902-906, in certain implementations, the acts 900 further include performing the one or more calibration cycles to determine initial sequencing parameters associated with the incorporated nucleobases of the oligonucleotides corresponding to the calibration sequences; and determining the sequencing parameter corresponding to the sequencing device by estimating the sequencing parameter based on the initial sequencing parameters.

As further suggested above, in certain cases, the acts 900 further include performing the one or more calibration cycles by performing a calibration cycle to determine an initial sequencing parameter corresponding to the sequencing device and a nucleobase call for given nucleobases incorporated into a set of growing oligonucleotides corresponding to one or more calibration sequences; and determining the sequencing parameter corresponding to the sequencing device by: determining a base-call difference from a comparison between the nucleobase call for the given nucleobases incorporated into the set of growing oligonucleotides and known complimentary nucleobases for the one or more calibration sequences; and adjusting the initial sequencing parameter corresponding to the sequencing device based on the base-call difference.

Beyond or in the alternative to the acts 900 described above, in some embodiments, the acts 900 include performing the one or more calibration cycles followed by: performing indexing cycles to determine nucleobase calls for indexing sequences before performing genomic sequencing cycles to determine nucleobase calls for sample genomic sequences; or performing the genomic sequencing cycles to determine nucleobase calls for the sample genomic sequences before performing the indexing cycles to determine nucleobase calls for the indexing sequences.

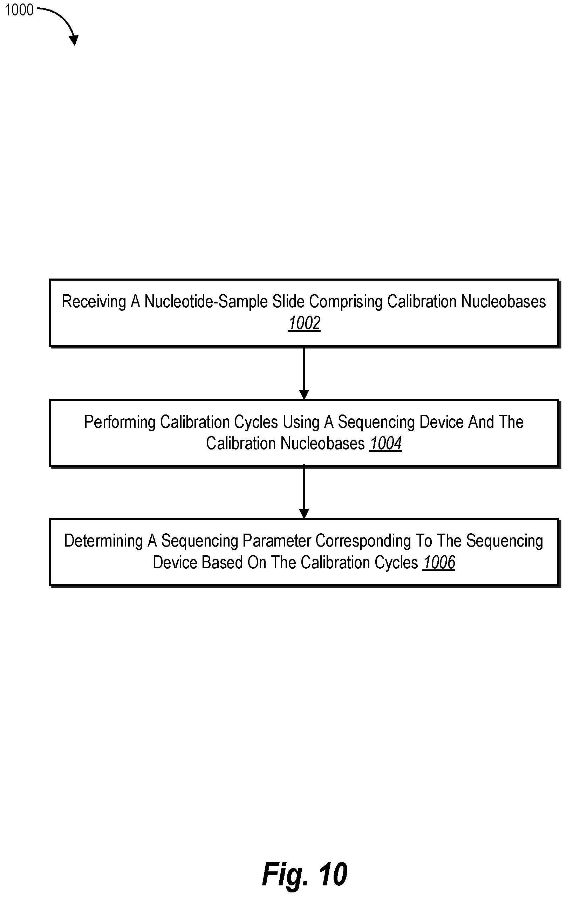

Turning now to FIG. 10, this figure illustrates a flowchart of a series of acts 1000 of receiving nucleotide-sample slide comprising calibration sequences and determining one or more sequencing parameters corresponding to a sequencing device based on the calibration sequences in accordance with one or more embodiments of the present disclosure. While FIG. 10 illustrates acts according to one embodiment, alternative embodiments may omit, add to, reorder, and/or modify any of the acts shown in FIG. 10. The acts of FIG. 10 can be performed as part of a method. Alternatively, a non-transitory computer readable storage medium can comprise instructions that, when executed by one or more processors, cause a computing device or a system to perform the acts depicted in FIG. 10. In still further embodiments, a system comprising at least one processor and a non-transitory computer readable medium comprising instructions that, when executed by one or more processors, cause the system to perform the acts of FIG. 10.

As shown in FIG. 10, the acts 1000 include an act 1002 of receiving a nucleotide-sample slide comprising calibration nucleobases. In particular, in some embodiments, the act 1002 includes receiving a nucleotide-sample slide comprising calibration nucleobases and sample library fragments associated with the calibration nucleobases. As noted above, in certain cases, a calibration nucleobase of the calibration nucleobases is part of the sample library fragments or part of surface-bound oligonucleotides that are attached to a surface of the nucleotide-sample slide.

In some cases, the calibration nucleobases comprise a proportional distribution of different nucleobase types. Relatedly, in certain embodiments, a first set of sample library fragments comprise a first calibration nucleobase of a first nucleobase type and a second set of sample library fragments comprise a second calibration nucleobase of a second nucleobase type.

As further shown in FIG. 10, the acts 1000 include an act 1004 of performing calibration cycles using a sequencing device and the calibration nucleobases. In particular, in some embodiments, the act 1004 includes performing one or more calibration cycles using a sequencing device to incorporate nucleobases into oligonucleotides corresponding to the calibration nucleobases and the associated sample library fragments. Additionally, or alternatively, performing the one or more calibration cycles does not comprise determining nucleobase calls for the incorporated nucleobases.

As suggested above, in certain embodiments, performing the one or more calibration cycles comprises performing the one or more calibration cycles before or after performing genomic sequencing cycles using the sequencing device to determine nucleobase calls for sample genomic sequences within sample library fragments.

As further shown in FIG. 10, the acts 1000 include an act 1006 of determining a sequencing parameter corresponding to the sequencing device based on the calibration cycles. In particular, in certain implementations, the act 1006 includes determining a sequencing parameter corresponding to the sequencing device based on the one or more calibration cycles and the calibration nucleobases.

Further, in some cases, determining the sequencing parameter corresponding to the sequencing device comprises determining one or more of equalizer coefficients, convolutional kernel coefficients, nucleobase centroids for intensity values, intensity-value boundaries for particular nucleobase types, nucleobase-specific-background intensity values, intensity normalization coefficients, gaussian covariance matrices, gaussian mean parameters, gaussian seed parameters, fully functional nucleotide (fFN) specific nucleotide location parameters, non-linear optical distortion parameters, structured illumination microscopy (SIM) parameters, per-cluster normalization parameters for a particular channel, channel-specific normalization parameters, cluster-specific-signal normalization parameters, color-channel-to-color-channel crosstalk parameters, or polycolonality parameters.

The methods described herein can be used in conjunction with a variety of nucleic acid sequencing techniques. Particularly applicable techniques are those wherein nucleic acids are attached at fixed locations in an array such that their relative positions do not change and wherein the array is repeatedly imaged. Embodiments in which images are obtained in different color channels, for example, coinciding with different labels used to distinguish one nucleotide base type from another are particularly applicable. In some embodiments, the process to determine the nucleotide sequence of a target nucleic acid (i.e., a nucleic-acid polymer) can be an automated process. Preferred embodiments include sequencing-by-synthesis (SBS) techniques.

SBS techniques generally involve the enzymatic extension of a nascent nucleic acid strand through the iterative addition of nucleotides against a template strand. In traditional methods of SBS, a single nucleotide monomer may be provided to a target nucleotide in the presence of a polymerase in each delivery. However, in the methods described herein, more than one type of nucleotide monomer can be provided to a target nucleic acid in the presence of a polymerase in a delivery.

SBS can utilize nucleotide monomers that have a terminator moiety or those that lack any terminator moieties. Methods utilizing nucleotide monomers lacking terminators include, for example, pyrosequencing and sequencing using γ-phosphate-labeled nucleotides, as set forth in further detail below. In methods using nucleotide monomers lacking terminators, the number of nucleotides added in each cycle is generally variable and dependent upon the template sequence and the mode of nucleotide delivery. For SBS techniques that utilize nucleotide monomers having a terminator moiety, the terminator can be effectively irreversible under the sequencing conditions used as is the case for traditional Sanger sequencing which utilizes dideoxynucleotides, or the terminator can be reversible as is the case for sequencing methods developed by Solexa (now Illumina, Inc.).

SBS techniques can utilize nucleotide monomers that have a label moiety or those that lack a label moiety. Accordingly, incorporation events can be detected based on a characteristic of the label, such as fluorescence of the label; a characteristic of the nucleotide monomer such as molecular weight or charge; a byproduct of incorporation of the nucleotide, such as release of pyrophosphate; or the like. In embodiments, where two or more different nucleotides are present in a sequencing reagent, the different nucleotides can be distinguishable from each other, or alternatively, the two or more different labels can be the indistinguishable under the detection techniques being used. For example, the different nucleotides present in a sequencing reagent can have different labels and they can be distinguished using appropriate optics as exemplified by the sequencing methods developed by Solexa (now Illumina, Inc.).

Preferred embodiments include pyrosequencing techniques. Pyrosequencing detects the release of inorganic pyrophosphate (PPi) as particular nucleotides are incorporated into the nascent strand (Ronaghi, M., Karamohamed, S., Pettersson, B., Uhlen, M. and Nyren, P. (1996) "Real-time DNA sequencing using detection of pyrophosphate release." Analytical Biochemistry 242(1), 84-9; Ronaghi, M. (2001) "Pyrosequencing sheds light on DNA sequencing." Genome Res. 11(1), 3-11; Ronaghi, M., Uhlen, M. and Nyren, P. (1998) "A sequencing method based on real-time pyrophosphate." Science 281(5375), 363; U.S. Pat. No. 6,210,891; U.S. Pat. No. 6,258,568 and U.S. Pat. No. 6,274,320, the disclosures of which are incorporated herein by reference in their entireties). In pyrosequencing, released PPi can be detected by being immediately converted to adenosine triphosphate (ATP) by ATP sulfurylase, and the level of ATP generated is detected via luciferase-produced photons. The nucleic acids to be sequenced can be attached to features in an array and the array can be imaged to capture the chemiluminescent signals that are produced due to incorporation of a nucleotides at the features of the array. An image can be obtained after the array is treated with a particular nucleotide type (e.g., A, T, C or G). Images obtained after addition of each nucleotide type will differ with regard to which features in the array are detected. These differences in the image reflect the different sequence content of the features on the array. However, the relative locations of each feature will remain unchanged in the images. The images can be stored, processed and analyzed using the methods set forth herein. For example, images obtained after treatment of the array with each different nucleotide type can be handled in the same way as exemplified herein for images obtained from different detection channels for reversible terminator-based sequencing methods.

In another exemplary type of SBS, cycle sequencing is accomplished by stepwise addition of reversible terminator nucleotides containing, for example, a cleavable or photobleachable dye label as described, for example, in WO 04/018497 and U.S. Pat. No. 7,057,026, the disclosures of which are incorporated herein by reference. This approach is being commercialized by Solexa (now Illumina Inc.), and is also described in WO 91/06678 and WO 07/123,744, each of which is incorporated herein by reference. The availability of fluorescently-labeled terminators in which both the termination can be reversed and the fluorescent label cleaved facilitates efficient cyclic reversible termination (CRT) sequencing. Polymerases can also be co-engineered to efficiently incorporate and extend from these modified nucleotides.

Preferably in reversible terminator-based sequencing embodiments, the labels do not substantially inhibit extension under SBS reaction conditions. However, the detection labels can be removable, for example, by cleavage or degradation. Images can be captured following incorporation of labels into arrayed nucleic acid features. In particular embodiments, each cycle involves simultaneous delivery of four different nucleotide types to the array and each nucleotide type has a spectrally distinct label. Four images can then be obtained, each using a detection channel that is selective for one of the four different labels. Alternatively, different nucleotide types can be added sequentially and an image of the array can be obtained between each addition step. In such embodiments, each image will show nucleic acid features that have incorporated nucleotides of a particular type. Different features are present or absent in the different images due the different sequence content of each feature. However, the relative position of the features will remain unchanged in the images. Images obtained from such reversible terminator-SBS methods can be stored, processed and analyzed as set forth herein. Following the image capture step, labels can be removed and reversible terminator moieties can be removed for subsequent cycles of nucleotide addition and detection. Removal of the labels after they have been detected in a particular cycle and prior to a subsequent cycle can provide the advantage of reducing background signal and crosstalk between cycles. Examples of useful labels and removal methods are set forth below.

In particular embodiments some or all of the nucleotide monomers can include reversible terminators. In such embodiments, reversible terminators/cleavable fluors can include fluor linked to the ribose moiety via a 3' ester linkage (Metzker, Genome Res. 15:1767-1776 (2005), which is incorporated herein by reference). Other approaches have separated the terminator chemistry from the cleavage of the fluorescence label (Ruparel et al., Proc Natl Acad Sci USA 102: 5932-7 (2005), which is incorporated herein by reference in its entirety). Ruparel et al described the development of reversible terminators that used a small 3' allyl group to block extension, but could easily be deblocked by a short treatment with a palladium catalyst. The fluorophore was attached to the base via a photocleavable linker that could easily be cleaved by a 30 second exposure to long wavelength UV light. Thus, either disulfide reduction or photocleavage can be used as a cleavable linker. Another approach to reversible termination is the use of natural termination that ensues after placement of a bulky dye on a dNTP. The presence of a charged bulky dye on the dNTP can act as an effective terminator through steric and/or electrostatic hindrance. The presence of one incorporation event prevents further incorporations unless the dye is removed. Cleavage of the dye removes the fluor and effectively reverses the termination. Examples of modified nucleotides are also described in U.S. Pat. No. 7,427,673, and U.S. Pat. No. 7,057,026, the disclosures of which are incorporated herein by reference in their entireties.

Additional exemplary SBS systems and methods which can be utilized with the methods and systems described herein are described in U.S. Patent Application Publication No. 2007/0166705, U.S. Patent Application Publication No. 2006/0188901, U.S. Pat. No. 7,057,026, U.S. Patent Application Publication No. 2006/0240439, U.S. Patent Application Publication No. 2006/0281109, PCT Publication No. WO 05/065814, U.S. Patent Application Publication No. 2005/0100900, PCT Publication No. WO 06/064199, PCT Publication No. WO 07/010,251, U.S. Patent Application Publication No. 2012/0270305 and U.S. Patent Application Publication No. 2013/0260372, the disclosures of which are incorporated herein by reference in their entireties.

Some embodiments can utilize detection of four different nucleotides using fewer than four different labels. For example, SBS can be performed utilizing methods and systems described in the incorporated materials of U.S. Patent Application Publication No. 2013/0079232. As a first example, a pair of nucleotide types can be detected at the same wavelength, but distinguished based on a difference in intensity for one member of the pair compared to the other, or based on a change to one member of the pair (e.g. via chemical modification, photochemical modification or physical modification) that causes apparent signal to appear or disappear compared to the signal detected for the other member of the pair. As a second example, three of four different nucleotide types can be detected under particular conditions while a fourth nucleotide type lacks a label that is detectable under those conditions, or is minimally detected under those conditions (e.g., minimal detection due to background fluorescence, etc.). Incorporation of the first three nucleotide types into a nucleic acid can be determined based on presence of their respective signals and incorporation of the fourth nucleotide type into the nucleic acid can be determined based on absence or minimal detection of any signal. As a third example, one nucleotide type can include label(s) that are detected in two different channels, whereas other nucleotide types are detected in no more than one of the channels. The aforementioned three exemplary configurations are not considered mutually exclusive and can be used in various combinations. An exemplary embodiment that combines all three examples, is a fluorescent-based SBS method that uses a first nucleotide type that is detected in a first channel (e.g. dATP having a label that is detected in the first channel when excited by a first excitation wavelength), a second nucleotide type that is detected in a second channel (e.g. dCTP having a label that is detected in the second channel when excited by a second excitation wavelength), a third nucleotide type that is detected in both the first and the second channel (e.g. dTTP having at least one label that is detected in both channels when excited by the first and/or second excitation wavelength) and a fourth nucleotide type that lacks a label that is not, or minimally, detected in either channel (e.g. dGTP having no label).

Further, as described in the incorporated materials of U.S. Patent Application Publication No. 2013/0079232, sequencing data can be obtained using a single channel. In such so-called one-dye sequencing approaches, the first nucleotide type is labeled but the label is removed after the first image is generated, and the second nucleotide type is labeled only after a first image is generated. The third nucleotide type retains its label in both the first and second images, and the fourth nucleotide type remains unlabeled in both images.

Some embodiments can utilize sequencing by ligation techniques. Such techniques utilize DNA ligase to incorporate oligonucleotides and identify the incorporation of such oligonucleotides. The oligonucleotides typically have different labels that are correlated with the identity of a particular nucleotide in a sequence to which the oligonucleotides hybridize. As with other SBS methods, images can be obtained following treatment of an array of nucleic acid features with the labeled sequencing reagents. Each image will show nucleic acid features that have incorporated labels of a particular type. Different features are present or absent in the different images due the different sequence content of each feature, but the relative position of the features will remain unchanged in the images. Images obtained from ligation-based sequencing methods can be stored, processed and analyzed as set forth herein. Exemplary SBS systems and methods which can be utilized with the methods and systems described herein are described in U.S. Pat. No. 6,969,488, U.S. Pat. No. 6,172,218, and U.S. Pat. No. 6,306,597, the disclosures of which are incorporated herein by reference in their entireties.

Some embodiments can utilize nanopore sequencing (Deamer, D. W. & Akeson, M. "Nanopores and nucleic acids: prospects for ultrarapid sequencing." Trends Biotechnol. 18, 147-151 (2000); Deamer, D. and D. Branton, "Characterization of nucleic acids by nanopore analysis". Acc. Chem. Res. 35:817-825 (2002); Li, J., M. Gershow, D. Stein, E. Brandin, and J. A. Golovchenko, "DNA molecules and configurations in a solid-state nanopore microscope" Nat. Mater. 2:611-615 (2003), the disclosures of which are incorporated herein by reference in their entireties). In such embodiments, the target nucleic acid passes through a nanopore. The nanopore can be a synthetic pore or biological membrane protein, such as α-hemolysin. As the target nucleic acid passes through the nanopore, each base-pair can be identified by measuring fluctuations in the electrical conductance of the pore. (U.S. Pat. No. 7,001,792; Soni, G. V. & Meller, "A. Progress toward ultrafast DNA sequencing using solid-state nanopores." Clin. Chem. 53, 1996-2001 (2007); Healy, K. "Nanopore-based single-molecule DNA analysis." Nanomed. 2, 459-481 (2007); Cockroft, S. L., Chu, J., Amorin, M. & Ghadiri, M. R. "A single-molecule nanopore device detects DNA polymerase activity with single-nucleotide resolution." J. Am. Chem. Soc. 130, 818-820 (2008), the disclosures of which are incorporated herein by reference in their entireties). Data obtained from nanopore sequencing can be stored, processed and analyzed as set forth herein. In particular, the data can be treated as an image in accordance with the exemplary treatment of optical images and other images that is set forth herein.

Some embodiments can utilize methods involving the real-time monitoring of DNA polymerase activity. Nucleotide incorporations can be detected through fluorescence resonance energy transfer (FRET) interactions between a fluorophore-bearing polymerase and γ-phosphate-labeled nucleotides as described, for example, in U.S. Pat. No. 7,329,492 and U.S. Pat. No. 7,211,414 (each of which is incorporated herein by reference) or nucleotide incorporations can be detected with zero-mode waveguides as described, for example, in U.S. Pat. No. 7,315,019 (which is incorporated herein by reference) and using fluorescent nucleotide analogs and engineered polymerases as described, for example, in U.S. Pat. No. 7,405,281 and U.S. Patent Application Publication No. 2008/0108082 (each of which is incorporated herein by reference). The illumination can be restricted to a zeptoliter-scale volume around a surface-tethered polymerase such that incorporation of fluorescently labeled nucleotides can be observed with low background (Levene, M. J. et al. "Zero-mode waveguides for single-molecule analysis at high concentrations." Science 299, 682-686 (2003); Lundquist, P. M. et al. "Parallel confocal detection of single molecules in real time." Opt. Lett. 33, 1026-1028 (2008); Korlach, J. et al. "Selective aluminum passivation for targeted immobilization of single DNA polymerase molecules in zero-mode waveguide nano structures." Proc. Natl. Acad. Sci. USA 105, 1176-1181 (2008), the disclosures of which are incorporated herein by reference in their entireties). Images obtained from such methods can be stored, processed and analyzed as set forth herein.

Some SBS embodiments include detection of a proton released upon incorporation of a nucleotide into an extension product. For example, sequencing based on detection of released protons can use an electrical detector and associated techniques that are commercially available from Ion Torrent (Guilford, CT, a Life Technologies subsidiary) or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

The above SBS methods can be advantageously carried out in multiplex formats such that multiple different target nucleic acids are manipulated simultaneously. In particular embodiments, different target nucleic acids can be treated in a common reaction vessel or on a surface of a particular substrate. This allows convenient delivery of sequencing reagents, removal of unreacted reagents and detection of incorporation events in a multiplex manner. In embodiments using surface-bound target nucleic acids, the target nucleic acids can be in an array format. In an array format, the target nucleic acids can be typically bound to a surface in a spatially distinguishable manner. The target nucleic acids can be bound by direct covalent attachment, attachment to a bead or other particle or binding to a polymerase or other molecule that is attached to the surface. The array can include a single copy of a target nucleic acid at each site (also referred to as a feature) or multiple copies having the same sequence can be present at each site or feature. Multiple copies can be produced by amplification methods such as, bridge amplification or emulsion PCR as described in further detail below.

The methods set forth herein can use arrays having features at any of a variety of densities including, for example, at least about 10 features/cm2, 100 features/cm2, 500 features/cm2, 1,000 features/cm2, 5,000 features/cm2, 10,000 features/cm2, 50,000 features/cm2, 100,000 features/cm2, 1,000,000 features/cm2, 5,000,000 features/cm2, or higher.

An advantage of the methods set forth herein is that they provide for rapid and efficient detection of a plurality of target nucleic acid in parallel. Accordingly the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666, each of which is incorporated herein by reference. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternatively, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeqTM platform (Illumina, Inc., San Diego, CA) and devices described in US Ser. No. 13/273,666, which is incorporated herein by reference.

The sequencing system described above sequences nucleic-acid polymers present in samples received by a sequencing device. As defined herein, "sample" and its derivatives, is used in its broadest sense and includes any specimen, culture and the like that is suspected of including a target. In some embodiments, the sample comprises DNA, RNA, PNA, LNA, chimeric or hybrid forms of nucleic acids. The sample can include any biological, clinical, surgical, agricultural, atmospheric or aquatic-based specimen containing one or more nucleic acids. The term also includes any isolated nucleic acid sample such a genomic DNA, fresh-frozen or formalin-fixed paraffin-embedded nucleic acid specimen. It is also envisioned that the sample can be from a single individual, a collection of nucleic acid samples from genetically related members, nucleic acid samples from genetically unrelated members, nucleic acid samples (matched) from a single individual such as a tumor sample and normal tissue sample, or sample from a single source that contains two distinct forms of genetic material such as maternal and fetal DNA obtained from a maternal subject, or the presence of contaminating bacterial DNA in a sample that contains plant or animal DNA. In some embodiments, the source of nucleic acid material can include nucleic acids obtained from a newborn, for example as typically used for newborn screening.

The nucleic acid sample can include high molecular weight material such as genomic DNA (gDNA). The sample can include low molecular weight material such as nucleic acid molecules obtained from FFPE or archived DNA samples. In another embodiment, low molecular weight material includes enzymatically or mechanically fragmented DNA. The sample can include cell-free circulating DNA. In some embodiments, the sample can include nucleic acid molecules obtained from biopsies, tumors, scrapings, swabs, blood, mucus, urine, plasma, semen, hair, laser capture micro-dissections, surgical resections, and other clinical or laboratory obtained samples. In some embodiments, the sample can be an epidemiological, agricultural, forensic or pathogenic sample. In some embodiments, the sample can include nucleic acid molecules obtained from an animal such as a human or mammalian source. In another embodiment, the sample can include nucleic acid molecules obtained from a non-mammalian source such as a plant, bacteria, virus or fungus. In some embodiments, the source of the nucleic acid molecules may be an archived or extinct sample or species.

Further, the methods and compositions disclosed herein may be useful to amplify a nucleic acid sample having low-quality nucleic acid molecules, such as degraded and/or fragmented genomic DNA from a forensic sample. In one embodiment, forensic samples can include nucleic acids obtained from a crime scene, nucleic acids obtained from a missing persons DNA database, nucleic acids obtained from a laboratory associated with a forensic investigation or include forensic samples obtained by law enforcement agencies, one or more military services or any such personnel. The nucleic acid sample may be a purified sample or a crude DNA containing lysate, for example derived from a buccal swab, paper, fabric or other substrate that may be impregnated with saliva, blood, or other bodily fluids. As such, in some embodiments, the nucleic acid sample may comprise low amounts of, or fragmented portions of DNA, such as genomic DNA. In some embodiments, target sequences can be present in one or more bodily fluids including but not limited to, blood, sputum, plasma, semen, urine and serum. In some embodiments, target sequences can be obtained from hair, skin, tissue samples, autopsy or remains of a victim. In some embodiments, nucleic acids including one or more target sequences can be obtained from a deceased animal or human. In some embodiments, target sequences can include nucleic acids obtained from non-human DNA such a microbial, plant or entomological DNA. In some embodiments, target sequences or amplified target sequences are directed to purposes of human identification. In some embodiments, the disclosure relates generally to methods for identifying characteristics of a forensic sample. In some embodiments, the disclosure relates generally to human identification methods using one or more target specific primers disclosed herein or one or more target specific primers designed using the primer design criteria outlined herein. In one embodiment, a forensic or human identification sample containing at least one target sequence can be amplified using any one or more of the target-specific primers disclosed herein or using the primer criteria outlined herein.

The components of the calibration-sequencing system 106 can include software, hardware, or both. For example, the components of the calibration-sequencing system 106 can include one or more instructions stored on a computer-readable storage medium and executable by processors of one or more computing devices (e.g., the user client device 108). When executed by the one or more processors, the computer-executable instructions of the calibration-sequencing system 106 can cause the computing devices to perform the bubble detection methods described herein. Alternatively, the components of the calibration-sequencing system 106 can comprise hardware, such as special purpose processing devices to perform a certain function or group of functions. Additionally, or alternatively, the components of the calibration-sequencing system 106 can include a combination of computer-executable instructions and hardware.

Furthermore, the components of the calibration-sequencing system 106 performing the functions described herein with respect to the calibration-sequencing system 106 may, for example, be implemented as part of a stand-alone application, as a module of an application, as a plug-in for applications, as a library function or functions that may be called by other applications, and/or as a cloud-computing model. Thus, components of the calibration-sequencing system 106 may be implemented as part of a stand-alone application on a personal computing device or a mobile device. Additionally, or alternatively, the components of the calibration-sequencing system 106 may be implemented in any application that provides sequencing services including, but not limited to Illumina BaseSpace, Illumina DRAGEN, or Illumina TruSight software. "Illumina," "BaseSpace," "DRAGEN," and "TruSight," are either registered trademarks or trademarks of Illumina, Inc. in the United States and/or other countries.

Embodiments of the present disclosure may comprise or utilize a special purpose or general-purpose computer including computer hardware, such as, for example, one or more processors and system memory, as discussed in greater detail below. Embodiments within the scope of the present disclosure also include physical and other computer-readable media for carrying or storing computer-executable instructions and/or data structures. In particular, one or more of the processes described herein may be implemented at least in part as instructions embodied in a non-transitory computer-readable medium and executable by one or more computing devices (e.g., any of the media content access devices described herein). In general, a processor (e.g., a microprocessor) receives instructions, from a non-transitory computer-readable medium, (e.g., a memory, etc.), and executes those instructions, thereby performing one or more processes, including one or more of the processes described herein.

Computer-readable media can be any available media that can be accessed by a general purpose or special purpose computer system. Computer-readable media that store computer-executable instructions are non-transitory computer-readable storage media (devices). Computer-readable media that carry computer-executable instructions are transmission media. Thus, by way of example, and not limitation, embodiments of the disclosure can comprise at least two distinctly different kinds of computer-readable media: non-transitory computer-readable storage media (devices) and transmission media.

Non-transitory computer-readable storage media (devices) includes RAM, ROM, EEPROM, CD-ROM, solid state drives (SSDs) (e.g., based on RAM), Flash memory, phase-change memory (PCM), other types of memory, other optical disk storage, magnetic disk storage or other magnetic storage devices, or any other medium which can be used to store desired program code means in the form of computer-executable instructions or data structures and which can be accessed by a general purpose or special purpose computer.

A "network" is defined as one or more data links that enable the transport of electronic data between computer systems and/or modules and/or other electronic devices. When information is transferred or provided over a network or another communications connection (either hardwired, wireless, or a combination of hardwired or wireless) to a computer, the computer properly views the connection as a transmission medium. Transmissions media can include a network and/or data links which can be used to carry desired program code means in the form of computer-executable instructions or data structures and which can be accessed by a general purpose or special purpose computer. Combinations of the above should also be included within the scope of computer-readable media.

Further, upon reaching various computer system components, program code means in the form of computer-executable instructions or data structures can be transferred automatically from transmission media to non-transitory computer-readable storage media (devices) (or vice versa). For example, computer-executable instructions or data structures received over a network or data link can be buffered in RAM within a network interface module (e.g., a NIC), and then eventually transferred to computer system RAM and/or to less volatile computer storage media (devices) at a computer system. Thus, it should be understood that non-transitory computer-readable storage media (devices) can be included in computer system components that also (or even primarily) utilize transmission media.

Computer-executable instructions comprise, for example, instructions and data which, when executed at a processor, cause a general-purpose computer, special purpose computer, or special purpose processing device to perform a certain function or group of functions. In some embodiments, computer-executable instructions are executed on a general-purpose computer to turn the general-purpose computer into a special purpose computer implementing elements of the disclosure. The computer executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the disclosure may be practiced in network computing environments with many types of computer system configurations, including, personal computers, desktop computers, laptop computers, message processors, hand-held devices, multiprocessor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. In a distributed system environment, program modules may be located in both local and remote memory storage devices.

Embodiments of the present disclosure can also be implemented in cloud computing environments. In this description, "cloud computing" is defined as a model for enabling on-demand network access to a shared pool of configurable computing resources. For example, cloud computing can be employed in the marketplace to offer ubiquitous and convenient on-demand access to the shared pool of configurable computing resources. The shared pool of configurable computing resources can be rapidly provisioned via virtualization and released with low management effort or service provider interaction, and then scaled accordingly.

A cloud-computing model can be composed of various characteristics such as, for example, on-demand self-service, broad network access, resource pooling, rapid elasticity, measured service, and so forth. A cloud-computing model can also expose various service models, such as, for example, Software as a Service (SaaS), Platform as a Service (PaaS), and Infrastructure as a Service (IaaS). A cloud-computing model can also be deployed using different deployment models such as private cloud, community cloud, public cloud, hybrid cloud, and so forth. In this description and in the claims, a "cloud-computing environment" is an environment in which cloud computing is employed.

FIG. 11 illustrates a block diagram of a computing device 1100 that may be configured to perform one or more of the processes described above. One will appreciate that one or more computing devices such as the computing device 1100 may implement the calibration-sequencing system 106 and the sequencing system 104. As shown by FIG. 11, the computing device 1100 can comprise a processor 1102, a memory 1104, a storage device 1106, an I/O interface 1108, and a communication interface 1110, which may be communicatively coupled by way of a communication infrastructure 1112. In certain embodiments, the computing device 1100 can include fewer or more components than those shown in FIG. 11. The following paragraphs describe components of the computing device 1100 shown in FIG. 11 in additional detail.

In one or more embodiments, the processor 1102 includes hardware for executing instructions, such as those making up a computer program. As an example, and not by way of limitation, to execute instructions for dynamically modifying workflows, the processor 1102 may retrieve (or fetch) the instructions from an internal register, an internal cache, the memory 1104, or the storage device 1106 and decode and execute them. The memory 1104 may be a volatile or nonvolatile memory used for storing data, metadata, and programs for execution by the processor(s). The storage device 1106 includes storage, such as a hard disk, flash disk drive, or other digital storage device, for storing data or instructions for performing the methods described herein.

The I/O interface 1108 allows a user to provide input to, receive output from, and otherwise transfer data to and receive data from computing device 1100. The I/O interface 1108 may include a mouse, a keypad or a keyboard, a touch screen, a camera, an optical scanner, network interface, modem, other known I/O devices or a combination of such I/O interfaces. The I/O interface 1108 may include one or more devices for presenting output to a user, including, but not limited to, a graphics engine, a display (e.g., a display screen), one or more output drivers (e.g., display drivers), one or more audio speakers, and one or more audio drivers. In certain embodiments, the I/O interface 1108 is configured to provide graphical data to a display for presentation to a user. The graphical data may be representative of one or more graphical user interfaces and/or any other graphical content as may serve a particular implementation.

The communication interface 1110 can include hardware, software, or both. In any event, the communication interface 1110 can provide one or more interfaces for communication (such as, for example, packet-based communication) between the computing device 1100 and one or more other computing devices or networks. As an example, and not by way of limitation, the communication interface 1110 may include a network interface controller (NIC) or network adapter for communicating with an Ethernet or other wire-based network or a wireless NIC (WNIC) or wireless adapter for communicating with a wireless network, such as a WI-FI.

Additionally, the communication interface 1110 may facilitate communications with various types of wired or wireless networks. The communication interface 1110 may also facilitate communications using various communication protocols. The communication infrastructure 1112 may also include hardware, software, or both that couples components of the computing device 1100 to each other. For example, the communication interface 1110 may use one or more networks and/or protocols to enable a plurality of computing devices connected by a particular infrastructure to communicate with each other to perform one or more aspects of the processes described herein. To illustrate, the sequencing process can allow a plurality of devices (e.g., a client device, sequencing device, and server device(s)) to exchange information such as sequencing data and error notifications.

In the foregoing specification, the present disclosure has been described with reference to specific exemplary embodiments thereof. Various embodiments and aspects of the present disclosure(s) are described with reference to details discussed herein, and the accompanying drawings illustrate the various embodiments. The description above and drawings are illustrative of the disclosure and are not to be construed as limiting the disclosure. Numerous specific details are described to provide a thorough understanding of various embodiments of the present disclosure.

The present disclosure may be embodied in other specific forms without departing from its spirit or essential characteristics. The described embodiments are to be considered in all respects only as illustrative and not restrictive. For example, the methods described herein may be performed with less or more steps/acts or the steps/acts may be performed in differing orders. Additionally, the steps/acts described herein may be repeated or performed in parallel with one another or in parallel with different instances of the same or similar steps/acts. The scope of the present application is, therefore, indicated by the appended claims rather than by the foregoing description. All changes that come within the meaning and range of equivalency of the claims are to be embraced within their scope.

Aspects can be understood from the following numbered paragraphs .
1. A system comprising:
   at least one processor; and
   a non-transitory computer readable medium comprising instructions that, when executed by the at least one processor, cause the system to:
      receive a nucleotide-sample slide comprising calibration sequences of nucleobases;
      perform one or more calibration cycles using a sequencing device to incorporate nucleobases into oligonucleotides corresponding to the calibration sequences; and
      determine a sequencing parameter corresponding to the sequencing device based on the one or more calibration cycles and the calibration sequences.
2. The system of paragraph 1, wherein the calibration sequences are part of sample library fragments deposited on the nucleotide-sample slide or part of surface-bound oligonucleotides that are attached to a surface of the nucleotide-sample slide.
3. The system of paragraph 1, further comprising instructions that, when executed by the at least one processor, cause the system to determine the sequencing parameter corresponding to the sequencing device by detecting the sequencing parameter from the sequencing device during or after the one or more calibration cycles.
4. The system of paragraph 1, further comprising instructions that, when executed by the at least one processor, cause the system to:
   perform the one or more calibration cycles to determine initial sequencing parameters associated with the incorporated nucleobases of the oligonucleotides corresponding to the calibration sequences; and
   determine the sequencing parameter corresponding to the sequencing device by estimating the sequencing parameter based on the initial sequencing parameters.
5. The system of paragraph 4, further comprising instructions that, when executed by the at least one processor, cause the system to determine the initial sequencing parameters or the sequencing parameter by determining one or more of equalizer coefficients, convolutional kernel coefficients, nucleobase centroids for intensity values, intensity-value boundaries for particular nucleobase types, nucleobase-specific-background intensity values, intensity normalization coefficients, gaussian covariance matrices, gaussian mean parameters, gaussian seed parameters, fully functional nucleotide (fFN) specific nucleotide location parameters, non-linear optical distortion parameters, structured illumination microscopy (SIM) parameters, per-cluster normalization parameters for a particular channel, channel-specific normalization parameters, cluster-specific-signal normalization parameters, color-channel-to-color-channel crosstalk parameters, or polycolonality parameters.
6. The system of paragraph 1, further comprising instructions that, when executed by the at least one processor, cause the system to:
   perform the one or more calibration cycles by performing a calibration cycle to determine an initial sequencing parameter corresponding to the sequencing device and a nucleobase call for given nucleobases incorporated into a set of growing oligonucleotides corresponding to one or more calibration sequences; and
   determine the sequencing parameter corresponding to the sequencing device by:
      determining a base-call difference from a comparison between the nucleobase call for the given nucleobases incorporated into the set of growing oligonucleotides and known complimentary nucleobases for the one or more calibration sequences; and
      adjusting the initial sequencing parameter corresponding to the sequencing device based on the base-call difference.
7. The system of paragraph 1, further comprising instructions that, when executed by the at least one processor, cause the system to perform the one or more calibration cycles by incorporating nucleobases of one nucleobase type or two nucleobase types into a calibration-sequence position of a set of growing oligonucleotides corresponding to a set of calibration sequences.
8. The system of paragraph 1, further comprising instructions that, when executed by the at least one processor, cause the system to perform the one or more calibration cycles by incorporating nucleobases having a proportional distribution of two nucleobase types, a proportional distribution of three nucleobase types, or a proportional distribution of four nucleobase types into a calibration-sequence position of a set of growing oligonucleotides corresponding to a set of calibration sequences.
9. The system of paragraph 1, further comprising instructions that, when executed by the at least one processor, cause the system to receive the nucleotide-sample slide comprising:
   the calibration sequences in each well of the nucleotide-sample slide for seeding each cluster of oligonucleotides within each well;
   the calibration sequences in a subset of wells of the nucleotide-sample slide for seeding each cluster of oligonucleotides within the subset of wells; or
   the calibration sequences in the subset of wells of the nucleotide-sample slide for seeding a subset of clusters of oligonucleotides within the subset of wells.
10. The system of paragraph 1, further comprising instructions that, when executed by the at least one processor, cause the system to receive the nucleotide-sample slide comprising:
   a first set of sample library fragments including a first set of calibration sequences for calibrating a first sequencing parameter; and
   a second set of sample library fragments including a second set of calibration sequences for calibrating a second sequencing parameter.
11. The system of paragraph 1, further comprising instructions that, when executed by the at least one processor, cause the system to perform the one or more calibration cycles by incorporating nucleobases into a set of growing oligonucleotides corresponding to a set of calibration sequences without determining nucleobase calls for the set of calibration sequences.
12. The system of paragraph 1, further comprising instructions that, when executed by the at least one processor, cause the system to perform the one or more calibration cycles before or after performing genomic sequencing cycles using the sequencing device to determine nucleobase calls for sample genomic sequences within sample library fragments.
13. A computer-implemented method comprising:
   receiving a nucleotide-sample slide comprising calibration nucleobases and sample library fragments associated with the calibration nucleobases;
   performing one or more calibration cycles using a sequencing device to incorporate nucleobases into oligonucleotides corresponding to the calibration nucleobases and the associated sample library fragments; and
   determining a sequencing parameter corresponding to the sequencing device based on the one or more calibration cycles and the calibration nucleobases.
14. The computer-implemented method of paragraph 13, wherein the calibration nucleobases comprise a proportional distribution of different nucleobase types.
15. The computer-implemented method of paragraph 13, wherein a first set of sample library fragments comprise a first calibration nucleobase of a first nucleobase type and a second set of sample library fragments comprise a second calibration nucleobase of a second nucleobase type.
16. The computer-implemented method of paragraph 13, wherein a calibration nucleobase of the calibration nucleobases is part of the sample library fragments or part of surface-bound oligonucleotides that are attached to a surface of the nucleotide-sample slide.
17. The computer-implemented method of paragraph 13, wherein performing the one or more calibration cycles does not comprise determining nucleobase calls for the incorporated nucleobases.
18. The computer-implemented method of paragraph 13, wherein performing the one or more calibration cycles comprises performing the one or more calibration cycles before or after performing genomic sequencing cycles using the sequencing device to determine nucleobase calls for sample genomic sequences within sample library fragments.
19. The computer-implemented method of paragraph 13, wherein determining the sequencing parameter corresponding to the sequencing device comprises determining one or more of equalizer coefficients, convolutional kernel coefficients, nucleobase centroids for intensity values, intensity-value boundaries for particular nucleobase types, nucleobase-specific-background intensity values, intensity normalization coefficients, gaussian covariance matrices, gaussian mean parameters, gaussian seed parameters, fully functional nucleotide (fFN) specific nucleotide location parameters, non-linear optical distortion parameters, structured illumination microscopy (SIM) parameters, per-cluster normalization parameters, channel-specific normalization parameters, cluster-specific-signal normalization parameters, color-channel-to-color-channel crosstalk parameters, or polycolonality parameters.
20. A non-transitory computer readable medium comprising instructions that, when executed by at least one processor, cause a system to perform a method of any of the preceding paragraphs.

## Claims

1. A computer-implemented method comprising:
receiving a nucleotide-sample slide comprising, for a genomic sample, sample library fragments including (i) calibration sequences of nucleobases that are ligated into or integrated as part of sample library fragments and (ii) sample genomic sequences of the genomic sample;
performing one or more calibration cycles to determine a sequencing parameter corresponding to a sequencing device by incorporating nucleobases into nucleotides complementing the calibration sequences; and
performing, after the one or more calibration cycles, genomic sequencing cycles for the sample genomic sequences using the sequencing device to determine nucleobase calls for the sample genomic sequences within the sample library fragments.

2. The computer-implemented method of claim 1, wherein a sample library fragment of the sample library fragments includes a calibration sequence that is integrated as part or all of a read priming sequence.

3. The computer-implemented method of claim 1 or 2, wherein a sample library fragment of the sample library fragments includes a calibration sequence that is integrated as part or all of a binding adapter sequence.

4. The computer-implemented method of any one of claims 1-3, wherein a sample library fragment of the sample library fragments includes a calibration sequence that is integrated as part or all of an indexing sequence, as part or all of a unique molecular identifier (UMI) sequence, or as part or all of a unique dual index (UDI) sequence.

5. The computer-implemented method of any one of claims 1-4, wherein a sample library fragment of the sample library fragments includes a calibration sequence between a binding adapter sequence and an indexing sequence, between the indexing sequence and a read priming sequence, or between the read priming sequence and a sample genomic sequence.

6. The computer-implemented method of any one of claims 1-5, wherein performing the one or more calibration cycles comprises performing the one or more calibration cycles before performing indexing cycles to determine nucleobase calls for indexing sequences.

7. The computer-implemented method of any one of claims 1-6, wherein performing the one or more calibration cycles comprises performing a calibration cycle for a first nucleic-acid reaction site while performing an indexing cycle or a genomic sequencing cycle for a second nucleic-acid reaction site.

8. The computer-implemented method of any one of claims 1-7, further comprising determining the sequencing parameter corresponding to the sequencing device based on the one or more calibration cycles and the calibration sequences.

9. The computer-implemented method of claim 8, wherein determining the sequencing parameter corresponding to the sequencing device comprises determining one or more of equalizer coefficients, convolutional kernel coefficients, nucleobase centroids for intensity values, intensity-value boundaries for particular nucleobase types, nucleobase-specific-background intensity values, intensity normalization coefficients, gaussian covariance matrices, gaussian mean parameters, gaussian seed parameters, fully functional nucleotide (fFN) specific nucleotide location parameters, non-linear optical distortion parameters, structured illumination microscopy (SIM) parameters, per-cluster normalization parameters, channel-specific normalization parameters, cluster-specific-signal normalization parameters, color-channel-to-color-channel crosstalk parameters, or polycolonality parameters.

10. The computer-implemented method of any one of claims 1-9, wherein receiving the nucleotide-sample slide comprises receiving the nucleotide-sample slide comprising the sample library fragments including:
the calibration sequences with known nucleobases; and
the sample genomic sequences from an unknown genomic sample comprising unknown nucleobases.

11. The computer-implemented method of any one of claims 1-10, wherein performing the one or more calibration cycles comprises incorporating nucleobases into a set of growing nucleotides corresponding to a set of calibration sequences without determining nucleobase calls for the set of calibration sequences.

12. The computer-implemented method of any one of claims 1-11, wherein the calibration sequences comprise different sequences of nucleobases having a proportional distribution of four nucleobase types.

13. The computer-implemented method of any one of claims 1-12, wherein the calibration sequences each comprise a single sequence of nucleobases following a same order of nucleobases and having four nucleobase types within the single sequence of nucleobases.

14. A system comprising at least one processor and a non-transitory computer readable medium comprising instructions that, when executed by the at least one processor, cause the system to perform a method according to any one of claims 1-13.

15. A non-transitory computer-readable medium storing instructions that, when executed by at least one processor, cause a system to perform a method according to any one of claims 1-13.
